# EUROPEAN PATENT APPLICATION

(11) **EP 4 223 751 A1**
(43) Date of publication of application: **09.08.2023**
(21) Application number: 21874584.2
(22) Date of filing: 30.09.2021
(51) Int. Cl.: C07D 417/12, C07D 417/14, A61K 31/427, A61K 31/506, A61K 31/501, A61P 25/28, A61P 13/08, A61P 25/04, A61P 13/00, A61P 29/00

(54) **BENZAMIDE COMPOUND AND USE THEREOF**

(30) Priority: 30.09.2020 CN 202011059868; 19.03.2021 CN 202110296983
(71) Applicant: Humanwell Healthcare (Group) Co., Ltd., Wuhan, Hubei 430075 (CN); Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); LI, Xueqiang, Wuhan, Hubei 430075 (CN); YANG, Chengbing, Wuhan, Hubei 430075 (CN); WANG, Yonggang, Wuhan, Hubei 430075 (CN); ZHANG, Bo, Wuhan, Hubei 430075 (CN); LI, Yang, Wuhan, Hubei 430075 (CN); LIU, Lifei, Wuhan, Hubei 430075 (CN); YANG, Jun, Wuhan, Hubei 430075 (CN); LI, Lie, Wuhan, Hubei 430075 (CN)
(74) Representative: Wilson, Justin Scott
(86) International application number: PCT/CN2021/122232
(87) International publication number: WO 2022/068930

(57) **Abstract**

Provided are a novel compound that effectively antagonizes P2X3 receptor, a preparation method thereof, and use thereof in the preparation of drugs. The compound is a compound represented by Formula I, or a tautomer, a stereoisomer, a hydrate, a solvate, a pharmaceutically acceptable salt, or a prodrug thereof.

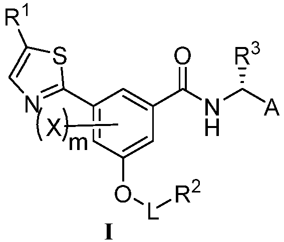

## Description

### PRIORITY INFORMATION

The present application claims priority to and the benefits of Chinese Patent Application No. 202011059868.0, filed with the China National Intellectual Property Administration on September 30, 2020 and Chinese Patent Application No. 202110296983.8, filed with the China National Intellectual Property Administration on March 19, 2021, the disclosures of which are hereby incorporated by reference in their entireties.

### FIELD

The present disclosure relates to the field of medicinal chemistry. Specifically, the present disclosure relates to a benzamide compound, and more particularly, the present disclosure relates to a benzamide compound, a preparation method thereof, and use thereof in the preparation of drugs.

### BACKGROUND

P2X receptor is a non-selective ATP-gated ion channel receptor, i.e., a purinergic receptor, capable of binding to extracellular ATP mainly derived from damaged or inflamed tissues. This receptor is widely expressed in the nervous, immune, cardiovascular, skeletal, gastrointestinal, respiratory, endocrine, and other systems, and is involved in regulation of cardiac rhythm and contractility, regulation of vascular tone, regulation of nociception (especially chronic pain), contraction of vas deferens during ejaculation, bladder contraction during voiding, aggregation of platelets, activation of macrophages, apoptosis, neuron-glial interactions, and other physiological processes. The above P2X receptor includes: seven homologous receptors, i.e., P2X1, P2X2, P2X3, P2X4, P2X5, P2X6 and P2X7; and three heterologous receptors, i.e., P2X2/3, P2X4/6, P2X1/5.

P2X3 is a subtype of the P2X receptor family and is selectively expressed in dorsal root ganglia, spinal cord, and brain neurons of nerve endings, i.e., in primary sensory neurons of medium and small diameters.

Numerous studies indicate that activation of P2X3 and P2X2/3 expressed in the primary sensory neurons plays an important role in acute injury, hyperalgesia, and hypersensitivity in rodents. Many studies reveal that an up-regulation of P2X3 receptor expression may lead to the formation of hyperalgesia, which is involved in pain signaling. P2X3-knockout mice exhibited reduced pain responses, and P2X3 receptor antagonists exhibited an effect of reducing nociception in models of pain and inflammatory pain.

P2X3 is distributed in primary afferent nerves around the airway and is capable of regulating cough. Studies have indicated that ATP released from damaged or inflamed tissues in the airway acts on P2X3 receptors in primary neurons, triggering depolarization and action potentials, the transfer of the potentials causes the impulse to cough and thus induces coughing. P2X3 receptors play an important role in cough reflex hypersensitivity. By antagonizing the binding to P2X3 receptors, the hypersensitivity of the cough reflex can be inhibited, thereby suppressing excessive coughing in patients with chronic cough. In addition, studies have also indicated that P2X3 antagonists can treat chronic obstructive pulmonary diseases, pulmonary fibrosis, pulmonary hypertension, or asthma. Therefore, P2X3 antagonists are also promising to become new drugs for the treatment of the above-mentioned diseases.

It has been reported that P2X3 is involved in the afferent pathway that controls the bladder volume reflex, and P2X3 knockout mice have significantly reduced urination frequency and significantly increased bladder capacity. Thus, the binding of P2X3 receptor-inhibiting antagonists to P2X3 receptors has effects in the treatment of urinary storage disorders and voiding disorders, such as overactive bladder. Therefore, P2X3 antagonists may be potential drugs for the treatment of overactive bladder and other related diseases.

P2X3 antagonists exhibit a wide application prospect. Currently, the commonly used clinical cough drugs such as Gabapentin, Morphine, and Amitriptyline, or treatment with speech pathology, can reduce cough for many patients, but they are not suitable for all patients. The central nervous system agents such as Gabapentin may cause adverse side effects and thus are unsuitable for long-term medication. It is urgent o develop chronic refractory cough drugs suitable for long-term medication, to provide doctors with more medication options. Therefore, the development of P2X3 antagonists is of great clinical significance.

### SUMMARY

The present disclosure aims to provide a P2X3 receptor antagonist, which can be used to prepare drugs for treating cough, pains, respiratory diseases, and urogenital diseases.

In a first aspect of the present disclosure, the present disclosure provides a compound, which, according to an embodiment of the present disclosure, is a compound represented by Formula I, or a tautomer, a stereoisomer, a hydrate, a solvate, a pharmaceutically acceptable salt, or a prodrug thereof: 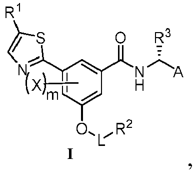 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted five-to ten-membered heteroaryl, wherein the R^{e}-substituted five-to ten-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different.

In a preferred embodiment of the present disclosure, the compound represented by Formula I, or the tautomer, the stereoisomer, the hydrate, the solvate, the pharmaceutically acceptable salt, or the prodrug thereof is: 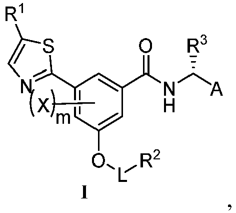 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents each independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted five-to ten-membered heteroaryl, wherein the R^{e}-substituted five-to ten-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents include a plurality of R^{e} substituents, the plurality of R^{e} substituents is identical or different.

In a preferred embodiment of the present disclosure, when R¹ is halogen, the halogen is F, Cl, Br, or I, and preferably Cl.

In a preferred embodiment of the present disclosure, when R¹ is the unsubstituted C₁-C₄ alkyl, the C₁-C₄ alkyl is methyl, ethyl, n-propyl, or isopropyl, and preferably methyl or ethyl.

In a preferred embodiment of the present disclosure, when R¹ is the C₁-C₄ alkyl substituted with 1 to 5 identical or different halogen atoms, C₁-C₄ alkyl is methyl, ethyl, n-propyl, or isopropyl, and preferably methyl or ethyl.

In a preferred embodiment of the present disclosure, when R¹ is the C₁-C₄ alkyl substituted with 1 to 5 identical or different halogen atoms, the halogen atoms are F, Cl, Br, or I, and preferably Cl or F.

In a preferred embodiment of the present disclosure, when X is halogen, the halogen is F or Cl.

In a preferred embodiment of the present disclosure, m is an integer selected from 1, 2 or 3, and preferably 1.

In a preferred embodiment of the present disclosure, when L is -(CH₂)ₙ-, n is an integer of 0, 1, or 2, and preferably n is 0 or 1.

In a preferred embodiment of the present disclosure, when R² is the unsubstituted or R^{a}-substituted C₁-C₆ alkyl, the C₁-C₆ alkyl is C₁-C₄ alkyl, and preferably sec-butyl.

In a preferred embodiment of the present disclosure, when R² is the unsubstituted or R^{a}-substituted C₁-C₆ alkyl, the C₁-C₆ alkyl is preferably methyl, ethyl, n-propyl, isopropyl, n-butyl, isobutyl, sec-butyl, or tert-butyl.

In a preferred embodiment of the present disclosure, when R² is the unsubstituted or R^{a}-substituted C₃-C₇ cycloalkyl, the C₃-C₇ alkyl is cyclopropyl, cyclobutyl, cyclopentyl, or cyclohexyl, and preferably cyclopropyl.

In a preferred embodiment of the present disclosure, when R² is the unsubstituted or R^{a}-substituted four-to seven-membered heterocycloalkyl, the four-to seven-membered heterocycloalkyl is four-membered, five-membered, or six-membered heterocycloalkyl.

In a preferred embodiment of the present disclosure, when R² is the unsubstituted or R^{a}-substituted four-to seven-membered heterocycloalkyl, the four-to seven-membered heterocycloalkyl has one or more heteroatoms selected from N, S, O, or P, and preferably selected from N or O.

In a preferred embodiment of the present disclosure, when R² is the unsubstituted or R^{a}-substituted four-to seven-membered heterocycloalkyl, the four-to seven-membered heterocycloalkyl has one to three heteroatoms, and preferably one or two heteroatoms.

In a preferred embodiment of the present disclosure, when R² is the unsubstituted or R^{a}-substituted four-to seven-membered heterocycloalkyl, R² has one to three R^{a} substituents, and preferably one R^{a} substituent.

In a preferred embodiment of the present disclosure, R^{a} is hydroxyl.

In a preferred embodiment of the present disclosure, when R^{a} is halogen, the halogen is F, Cl, Br, or I, and preferably F or Cl.

In a preferred embodiment of the present disclosure, when R^{a} is C₁-C₆ alkyl, the C₁-C₆ alkyl is C₁-C₃ alkyl, and preferably methyl.

In a preferred embodiment of the present disclosure, when R^{a} is the deuterated C₁-C₆ alkyl, the C₁-C₆ alkyl is deuterated C₁-C₃ alkyl, and preferably 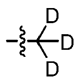 .

In a preferred embodiment of the present disclosure, when R^{a} is -(C₁-C₆ alkylene)-OH, the C₁-C₆ alkylene is C₁-C₄ alkylene, preferably methylene, ethylene, n-propylene, or isopropylidene, and more preferably methylene.

In a preferred embodiment of the present disclosure, R³ is hydrogen.

In a preferred embodiment of the present disclosure, when R³ is the unsubstituted C₁-C₄ alkyl, the C₁-C₄ alkyl is methyl, ethyl, n-propyl, or isopropyl, and preferably methyl.

In a preferred embodiment of the present disclosure, when A is the R^{e}-substituted five-to ten-membered heteroaryl, the five-to ten-membered heteroaryl is six-membered heteroaryl, and preferably pyrimidinyl or pyridazinyl.

In a preferred embodiment of the present disclosure, when A is the R^{e}-substituted five-to ten-membered heteroaryl, A has one R^{e} substituent.

In a preferred embodiment of the present disclosure, when A is the R^{e}-substituted five-to ten-membered heteroaryl, the R^{e} substituent is C₁-C₃ alkyl substituted with 1 to 5 identical or difference halogen atoms, and preferably trifluoromethyl.

In a preferred embodiment of the present disclosure, when A is the R^{e}-substituted five-to ten-membered heteroaryl, the R^{e} substituent is unsubstituted C₁-C₃ alkyl, and preferably methyl.

In a preferred embodiment of the present disclosure, -L-R² is selected from:

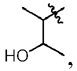

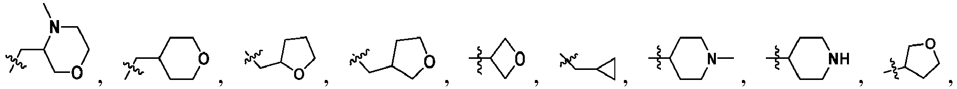

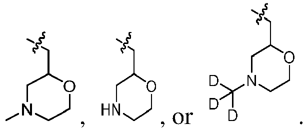

In a preferred embodiment of the present disclosure, -L-R² is

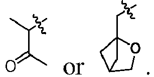

In a preferred embodiment of the present disclosure, -L-R² is selected from: 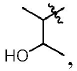 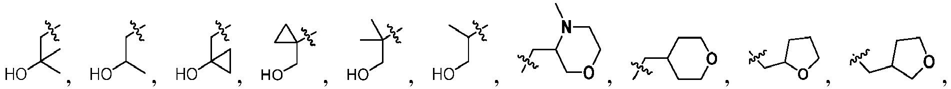 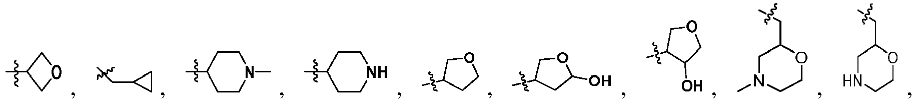 or

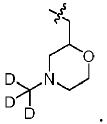

In a preferred embodiment of the present disclosure, R¹ is methyl, ethyl, or Cl.

In a preferred embodiment of the present disclosure, -L-R² is selected from:

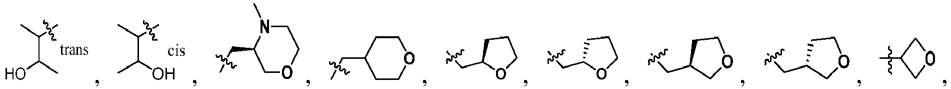

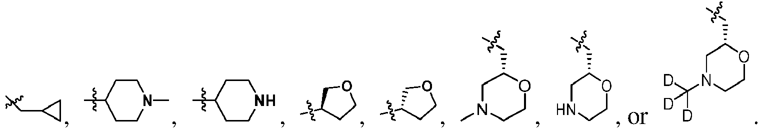

In a preferred embodiment of the present disclosure, -L-R² is selected from: 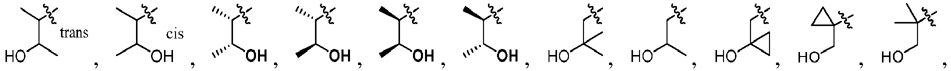 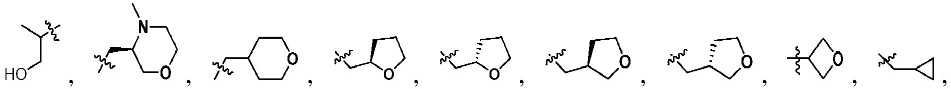 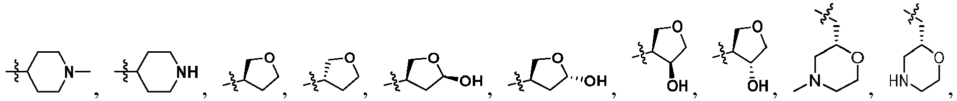 or

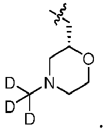

In a preferred embodiment of the present disclosure, R³ is methyl or hydrogen.

In a preferred embodiment of the present disclosure, A is 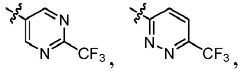 or

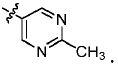

In a preferred embodiment of the present disclosure, the compound represented by Formula I, or the tautomer, the stereoisomer, the hydrate, the solvate, the pharmaceutically acceptable salt, or the prodrug thereof is: 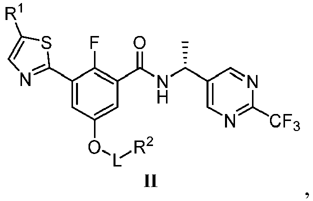 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, or the compound represented by Formula I, the tautomer, the stereoisomer, the hydrate, the solvate, the pharmaceutically acceptable salt, or the prodrug thereof is: 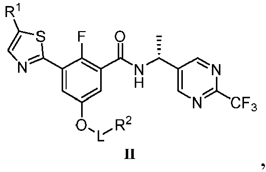 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 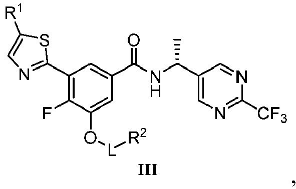 wherein
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 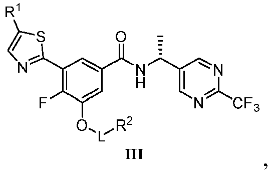 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 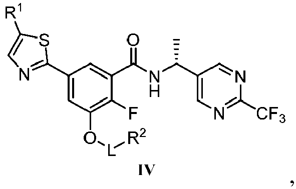 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 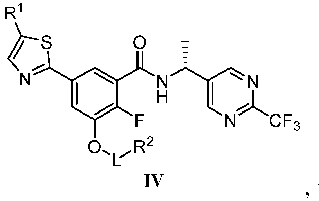 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 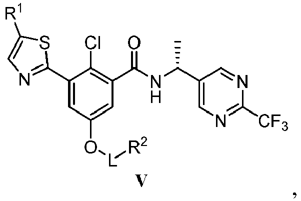 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 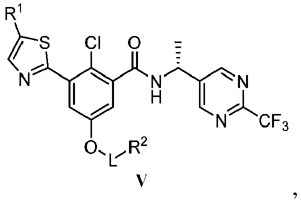 wherein:
R¹ is selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 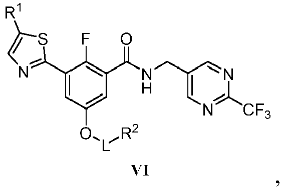 wherein
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 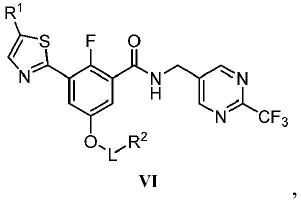 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 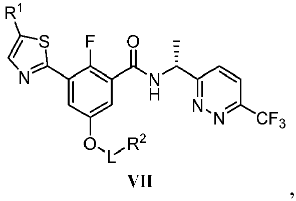 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 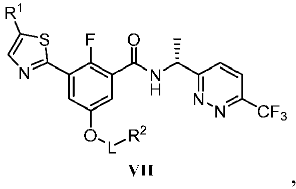 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 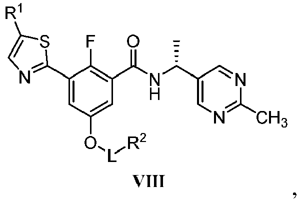 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is: 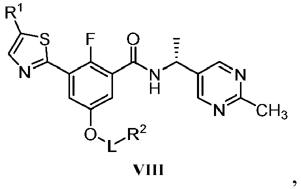 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=0), -C(O)O-(C₁-C₄ alkyl), -C(O)-(C₁-C₄ alkyl), or deuterated C₁-C₆ alkyl; when the one or more R^{a} substituents include a plurality of R^{a} substituents, the plurality of R^{a} substituents is identical or different; and wherein:
   the unsubstituted or R^{a}-substituted five-to ten-membered heteroaryl has 1 to 3 heteroatoms each selected from N, S, O, or P;
   the unsubstituted or R^{a}-substituted four-to seven-membered heterocycloalkyl has 1 to 3 heteroatoms each selected from N, S, O, or P; and
   the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

In a preferred embodiment of the present disclosure, the compound represented by Formula I, or the tautomer, the stereoisomer, the hydrate, the solvate, the pharmaceutically acceptable salt, or the prodrug thereof is selected from:

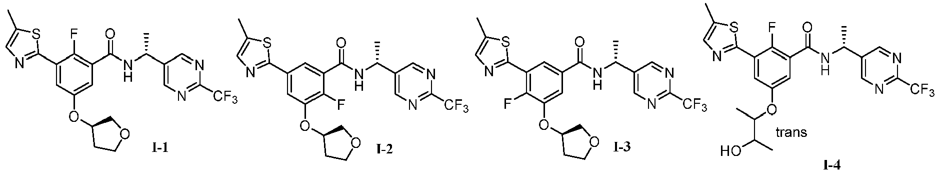

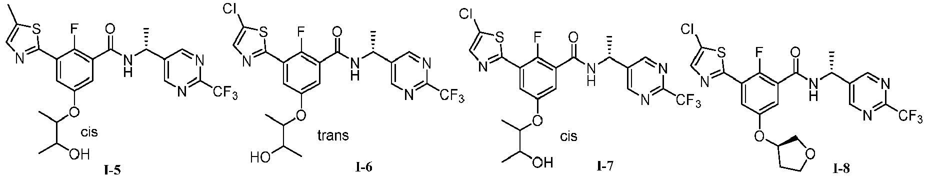

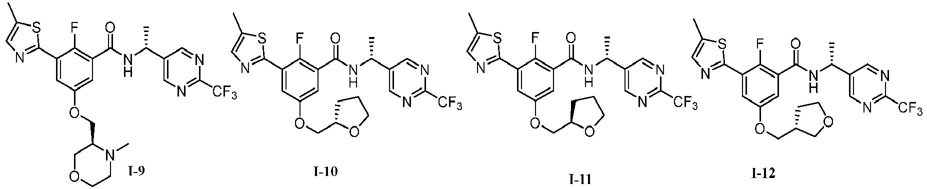

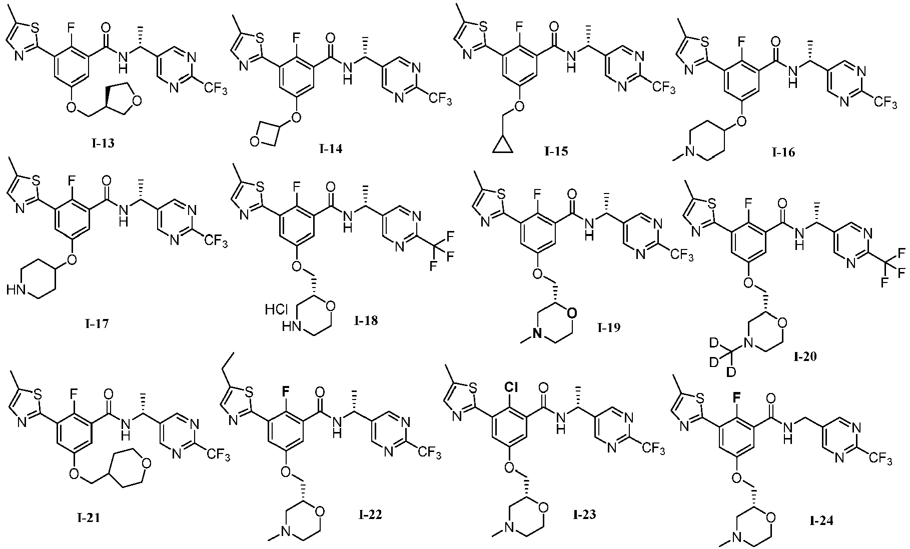

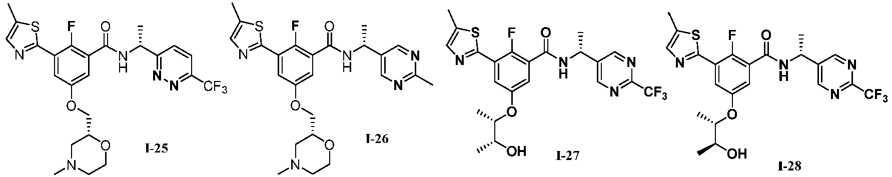

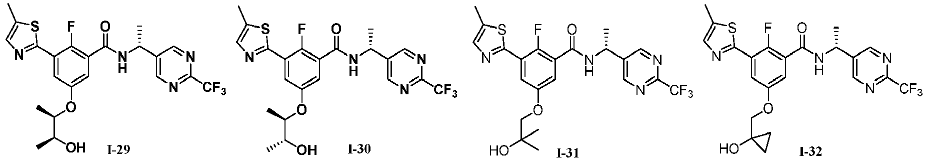

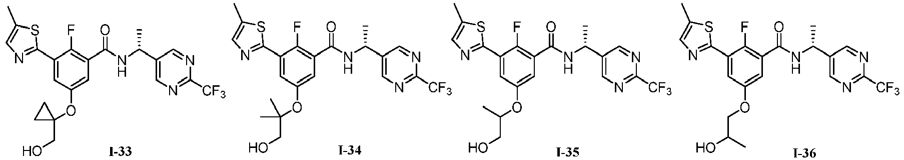

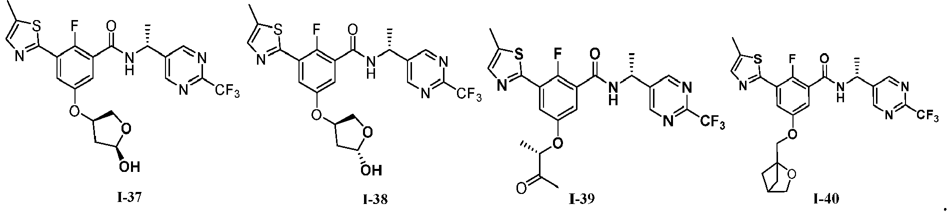

In a second aspect of the present disclosure, the present disclosure provides a pharmaceutical composition. The pharmaceutical composition includes: a therapeutically effective amount of the compound, or the tautomer, the stereoisomer, the hydrate, the solvate, the pharmaceutically acceptable salt, or the prodrug thereof as described above; and a pharmaceutically acceptable carrier, diluent, or excipient.

According to specific embodiments of the present disclosure, the therapeutically effective amount of the compound, or the tautomer, the stereoisomer, the hydrate, the solvate, the pharmaceutically acceptable salt, or the prodrug thereof and the pharmaceutically acceptable carrier, diluent, or excipient, which are included in the pharmaceutical composition of the present disclosure, can be mixed to prepare a pharmaceutical preparation suitable for oral or parenteral administration. Methods of administration include, but are not limited to, intradermal, intramuscular, intraperitoneal, intravenous, subcutaneous, intranasal, and oral routes. The preparation may be administered via any route, such as via infusion or bolus injection, or via administration route of absorption through the epithelium or mucocutaneous (e.g., oral mucosa or rectum, etc.). Administration can be systemic or local. Examples of preparations for oral administration include solid or liquid dosage forms, specifically, including tablets, pills, granules, powders, capsules, syrups, emulsions, suspensions and the like. The preparations can be prepared by methods known in the related art and include carriers, diluents or excipients conventionally used in the field of pharmaceutical formulations.

In a third aspect of the present disclosure, the present disclosure provides use of the compound, or the tautomer, the stereoisomer, the hydrate, the solvate, the pharmaceutically acceptable salt, or the prodrug thereof, or the pharmaceutical composition in the manufacture of a medicament for treating a P2X3-related disease.

According to specific embodiments of the present disclosure, for the use of the compound, or the tautomer, the stereoisomer, the hydrate, the solvate, the pharmaceutically acceptable salt, or the prodrug thereof, or the pharmaceutical composition in the manufacture of the medicament, the medicament can be used for treating or preventing pains. The pains include, for example, chronic pain, acute pain, endometriosis pain, neuropathic pain, back pain, cancer pain, inflammatory pain, surgical pain, migraine, and visceral pain.

According to specific embodiments of the present disclosure, for the use of the compound, or the tautomer, the stereoisomer, the hydrate, the solvate, the pharmaceutically acceptable salt, or the prodrug thereof, or the pharmaceutical composition in the manufacture of the medicament, the medicament can be used for treating or preventing urogenital diseases. These diseases include, for example, decreased bladder capacity, frequent urination, urge incontinence, stress incontinence, hyperresponsive bladder, benign prostatic hypertrophy, prostatitis, detrusor hyperreflexia, frequent micturition, nocturia, urgent urination, overactive bladder syndrome, pelvic hypersensitivity, urethritis, pelvic pain syndrome, prostate pain, and cystitis.

According to specific embodiments of the present disclosure, for the use of the compound, or the tautomer, the stereoisomer, the hydrate, the solvate, the pharmaceutically acceptable salt, or the prodrug thereof, or the pharmaceutical composition in the manufacture of the medicament, the medicament can be used for treating or preventing respiratory diseases. These diseases include, for example, chronic obstructive pulmonary disease, pulmonary hypertension, pulmonary fibrosis, asthma and obstructive apnea, chronic cough, refractory chronic cough, and acute cough.

In a fourth aspect of the present disclosure, the present disclosure provides a method for treating a P2X3-related disease. The method includes: administering, to a subject, the compound represented by Formula I, or the tautomer, the stereoisomer, the hydrate, the solvate, the pharmaceutically acceptable prodrug, or the salt thereof as described above, and/or the above-mentioned pharmaceutical composition.

According to specific embodiments of the present disclosure, for the use of the compound, or the tautomer, the stereoisomer, the hydrate, the solvate, the pharmaceutically acceptable salt, or the prodrug thereof, or the pharmaceutical composition in the manufacture of the medicament, the medicament can be used for treating or preventing pains. The pains include, for example, chronic pain, acute pain, endometriosis pain, neuropathic pain, back pain, cancer pain, inflammatory pain, surgical pain, migraine, and visceral pain.

According to specific embodiments of the present disclosure, for the use of the compound, the tautomer, the stereoisomer, the hydrate, the solvate, the pharmaceutically acceptable salt, or the prodrug thereof, or the pharmaceutical composition in the manufacture of the medicament, the medicament can be used for treating or preventing urogenital diseases. These diseases include, for example, decreased bladder capacity, frequent urination, urge incontinence, stress incontinence, hyperresponsive bladder, benign prostatic hypertrophy, prostatitis, detrusor hyperreflexia, frequent micturition, nocturia, urgent urination, overactive bladder syndrome, pelvic hypersensitivity, urethritis, pelvic pain syndrome, prostate pain, and cystitis.

According to specific embodiments of the present disclosure, for the use of the compound, or the tautomer, the stereoisomer, the hydrate, the solvate, the pharmaceutically acceptable salt, or the prodrug thereof, or the pharmaceutical composition in the manufacture of the medicament, the medicament can be used for treating or preventing respiratory diseases. These diseases include, for example, chronic obstructive pulmonary disease, pulmonary hypertension, pulmonary fibrosis, asthma and obstructive apnea, chronic cough, refractory chronic cough, and acute cough.

### Terms and Definitions

Unless otherwise indicated, terms used in the present disclosure, including the specification and claims, are defined as follows.

Those skilled in the art can understand that, according to the usual practice in the related art, 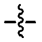 in the structural formula of the present disclosure is used to delineate a chemical bond, which is the point at which a moiety or substituent is connected to the core structure or the backbone structure.

The term "pharmaceutically acceptable" is referred for the compounds, materials, compositions and/or dosage forms, and means that they are suitable for use in contact with human and animal tissues without excess toxicity, irritation, allergic reactions or other problems or complications within the scope of reliable medical judgment, and are commensurate with a reasonable benefit/risk ratio.

The term "pharmaceutically acceptable salt" refers to salts of pharmaceutically acceptable non-toxic acids or bases, including salts of inorganic acids and bases and organic acids and bases.

The term "pharmaceutical composition" refers to a mixture of one or more of the compounds described herein or physiologically/pharmaceutically acceptable salts or prodrugs thereof and other chemical components. The other chemical components can be, for example, physiologically/pharmaceutically acceptable carriers and excipients. The pharmaceutical composition aims to facilitate the administration of the compound to an organism.

The term "excipient" refers to a pharmaceutically acceptable inert ingredient. Examples of the "excipient" include, but are not limited to, binders, disintegrants, lubricants, glidants, stabilizers, fillers, diluents, and the like. The term "solvate" refers to the compound of the present disclosure or a salt thereof bonded with a stoichiometric or non-stoichiometric solvent through an intermolecular non-covalent force. When the solvent is water, the solvate is a hydrate.

The term "prodrug" refers to substances that can be converted, under physiological conditions or through solvolysis, into the compound of the present disclosure having biological activity. The prodrug of the present disclosure is prepared by modifying the functional groups in the compound, and the modification can be removed by conventional operations or removed *in vivo,* to obtain the parent compound. The prodrug includes a compound which is formed by connecting a hydroxyl group or amino group in the compound of the present disclosure to any group. When the prodrug of the compound of the present disclosure is administered to a mammal individual, the prodrug is dissociated to form a free hydroxyl or amino group.

The term "stereoisomer" refers to an isomer produced by a different spatial arrangement of atoms in the molecule, including *cis* and *trans* isomers, enantiomers, diastereomers, and conformers.

In accordance with the selection of raw materials and methods, the compound of the present disclosure may exist in the form of one of the possible isomers or a mixture thereof, for example, as a pure optical isomer, or as a mixture of isomers such as a racemic mixture and a diastereoisomeric mixture, depending on the number of asymmetric carbon atoms. When describing a compound with an optical activity, the prefixes D and L, or R and S are symbols used to denote the absolute configuration of the molecule with respect to the chiral center (or multiple chiral centers) in the molecule. The prefixes D and L, or (+) and (-) are symbols used to specify a rotation of plane-polarized light caused by a compound, where (-) or L indicates that the compound is levorotatory, and the prefix (+) or D indicates that the compound is dextrorotatory. For a given chemical structure, these stereoisomers are identical except that these stereoisomers are mirror images of each other. Specific stereoisomers can be referred to as enantiomers, and a mixture of such isomers is called an enantiomeric mixture. A mixture of enantiomers in 50: 50 is called a racemic mixture or a racemate, which may occur when there is no stereoselectivity or stereospecificity in a chemical reaction or process. Numerous geometric isomers of olefins, C=N double bonds, etc. may also exist in the compounds described herein, and all such stable isomers shall be contemplated in the present disclosure. If the compounds described herein contain olefinic double bonds, such double bonds include both E-and Z-geometric isomers, unless otherwise specified. If the compound contains a disubstituted cycloalkyl, the substituent of the cycloalkyl may have a *cis-* or trans-conformation.

When the bond with a chiral carbon in the formula of the present disclosure is depicted in a straight line, it should be understood that the two configurations (*R*) and (*S*) of the chiral carbon and both the resulting enantiomerically pure compound and mixture are included in the scope defined by the general formula. The diagrammatic presentation of the racemate or enantiomerically pure compound herein is from Maehr, J. Chem. Ed. 1985, 62: 114-120. Unless otherwise specified, the wedge bond and the dashed bond are used to represent the absolute configuration of a stereocenter.

Optically active (R)-or (S)-isomers can be prepared using chiral synthons or chiral preparations, or resolved using conventional techniques. The compounds of the present disclosure containing asymmetrically substituted carbon atoms can be separated in an optically active form or in a racemic form. The resolution of a racemic mixture of a compound can be carried out with any of a variety of methods known in the art. Exemplary methods include fractional recrystallization using chiral resolving acids, which are optically active salt-forming organic acids. For example, the suitable resolving agents for fractional recrystallization are optically active acids, such as tartaric acid, diacetyl tartaric acid, dibenzoyl tartaric acid, mandelic acid, malic acid, lactic acid, or various optically active camphor sulfonic acids such as the *D* and *L* forms of β-camphor sulfonic acid. Other resolving agents suitable for fractional crystallization include α-methyl-benzylamine in a pure stereoisomeric form (for example, *S* and *R* forms or a diastereomerically pure form), 2-phenylglycinol, norephedrine, ephedrine, N-methylephedrine, cyclohexylethylamine, 1, 2-diaminocyclohexane, etc. The resolution of the racemic mixture can also be carried out by elution on a column filled with an optically active resolving agent (for example, dinitrobenzoylphenylglycine). High performance liquid chromatography (HPLC) or supercritical fluid chromatography (SFC) can also be employed. The specific method, elution conditions, and the chromatographic columns can be selected by those skilled in the art according to the structures of the compounds and the experimental results. Further, pure optically active starting materials or reagents with known configurations can also be used to obtain any enantiomers or diastereomers of the compounds described in the present disclosure through stereo-organic synthesis.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. The prototropic tautomer is resulted from a migration of covalently bonded hydrogen atoms between two atoms. The tautomer generally exists in an equilibrium form. When trying to separate a single tautomer, a mixture is usually produced, the physical and chemical properties of which are consistent with the mixture of compounds. The position of equilibrium depends on the intramolecularly chemical properties. For example, for many aliphatic aldehydes and ketones, such as acetaldehyde, the ketonic type is dominant; and for phenols, the enol type is dominant. All tautomeric forms of the compounds are included in the present disclosure.

The compound of the present disclosure may contain atomic isotopes in an unnatural ratio on one or more of the atoms constituting the compound. For example, the compound may be labeled with a radioisotope, such as deuterium (²H), tritium (³H), iodine-125 (¹²⁵I), or C-14 (¹⁴C). The transformation of all isotopic compositions of the compounds of the present disclosure, whether radioactive or not, are included within the scope of the present disclosure.

With respect to a drug or pharmacologically active agent, the term "effective amount" or "therapeutically effective amount" refers to a non-toxic but sufficient amount of the drug or agent to achieve the desired effects. For oral dosage forms in the present disclosure, an "effective amount" of one active substance in a composition refers to an amount required to achieve the desired effects when used in combination with another active substance in the composition. The determination of the effective amount varies from person to person, depending on the age and general condition of the subject, as well as the specific active substance. The appropriate effective amount in individual cases can be determined by those skilled in the art based on routine experiments.

The terms "active component", "therapeutic agent", "active substance", or "active agent" refer to a chemical entity that is effective in treating the target disorder, disease, or condition.

The term "substituted" means that any one or more hydrogen atoms on a specified atom are replaced by one or more substituents, including deuterium and hydrogen variants, as long as the valence of the specified atom is normal and the substituted compound is stable. When the substituent is keto (i.e., =O), it means that two hydrogen atoms are substituted. Ketone substitution does not occur on aromatic groups. The term "optionally substituted" means that it is substituted or unsubstituted. Unless otherwise specified, the type and number of substituents may be arbitrary on a chemically achievable basis.

The term "C₁-C₆ alkyl" refers to a linear or branched, saturated, monovalent hydrocarbon group having 1, 2, 3, 4, 5, or 6 carbon atoms. Said alkyl is, for example, methyl, ethyl, propyl, butyl, pentyl, hexyl, isopropyl, isobutyl, sec-butyl, tert-butyl, isoamyl, 2-methylbutyl, 1-methylbutyl, 1-ethylpropyl, 1, 2-dimethylpropyl, neopentyl, 1, 1-dimethylpropyl, 4-methylpentyl, 3-methylpentyl, 2-methylpentyl, 1-methylpentyl, 2-ethylbutyl, 1-ethylbutyl, 3, 3-dimethylbutyl, 2, 2-dimethylbutyl, 1, 1-dimethylbutyl, 2, 3-dimethylbutyl, 1, 3-dimethylbutyl, 1, 2-dimethylbutyl, etc., or an isomer thereof. Specifically, said group has 1, 2, 3, or 4 carbon atoms ("C₁-C₄ alkyl"), e.g., methyl, ethyl, n-propyl, isopropyl, n-butyl, isobutyl, sec-butyl, or tert-butyl.

The term "C₁-C₃ alkoxy" should be understood as -O-(C₁-C₃ alkyl), where "C₁-C₃ alkyl" is defined as above.

The term "deuterated C₁-C₆ alkyl" should be understood as C₁-C₆ alkyl in which one or more hydrogen atoms are substituted by deuterium, where "C₁-C₆ alkyl" is defined as above.

The term "C₃-C₇ cycloalkyl" should be understood as a saturated monovalent monocyclic or bicyclic hydrocarbon ring having 3 to 7 carbon atoms, including fused or bridged polycyclic ring systems. Similarly, "C₃-C₆ cycloalkyl" should be understood as a saturated monovalent monocyclic or bicyclic hydrocarbon ring as defined above having 3 to 6 carbon atoms. Examples include, but are not limited to, cyclopropyl, cyclobutyl, cyclopentyl, cyclohexyl.

The term "alkylene" should be understood as a saturated divalent hydrocarbyl group obtained by removing two hydrogen atoms from a saturated straight or branched hydrocarbon. Unless otherwise specified, alkylene contain 1 to 10 carbon atoms. In some embodiments, alkylene contains 1 to 6 carbon atoms; in some other embodiments, alkylene contains 1 to 4 carbon atoms; in still some other embodiments, alkylene contains 1 to 2 carbon atoms. Examples include, but are not limited to, methylene (-CH₂-), ethylene (-CH₂CH₂-), n-propylene (-CH₂CH₂CH₂-), isopropylidene (-CH (CH₃)CH₂-), etc.

The term "heterocycloalkyl" should be understood as a saturated monovalent monocyclic hydrocarbon ring having the indicated number of ring atoms, in which one, two or three ring atoms of the hydrocarbon ring are independently substituted by one, two, or three heteroatoms or heteroatom-containing groups, which are independently selected from O, S, S (=O), S (=O)₂, or N. The term "four-to seven-membered heterocycloalkyl" should be understood as a saturated monovalent monocyclic "heterocycloalkyl" ring as defined above containing 4, 5, 6, or 7 ring atoms. Similarly, the term "four-to six-membered heterocycloalkyl" should be understood as a saturated monovalent monocyclic "heterocycloalkyl" ring as defined above containing 4, 5, or 6 ring atoms.

The term "six-to twelve-membered heterobicycloalkyl" should be understood as a saturated monovalent bicyclic hydrocarbyl group containing 5, 6, 7, 8, 9, or 10 carbon atoms and one, two, or three heteroatoms or heteroatom-containing groups independently selected from O, S, S (=O), S (=O)₂, or N, in which two rings share one or two common ring atoms, provided that the total number of ring atoms is not greater than 12.

The term "five-to eight-membered aryl" should be understood as a monovalent aromatic or partially aromatic monocyclic, bicyclic, or tricyclic hydrocarbon ring having 5 to 8 carbon atoms, especially a ring having 6 carbon atoms ("C₆ aryl"), such as phenyl. The five-to eight-membered aryl may be mono-or poly-substituted, and the substitution site is not limited, for example, it may be ortho-, para-, or meta-substituted.

The term "five-to ten-membered heteroaryl" should be understood as a monovalent monocyclic, bicyclic, or tricyclic aromatic ring group having 5 to 10 ring atoms (especially, 5 or 6 carbon atoms) and 1 to 5 heteroatoms (preferably 1 to 3 heteroatoms) independently selected from N, O, or S. In addition, in each case, it may be benzo-fused group. Particularly, heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, etc.; or pyridyl, pyridazinyl, pyrimidinyl, pyrazinyl, triazinyl, etc.; cinnolinyl, phthalazinyl, quinazolinyl, quinoxalinyl, naphthyridinyl, pteridinyl, carbazolyl, acridinyl, phenazinyl, phenothiazinyl, phenoxazinyl, etc.

The term "halogen substituent" or "halogen" is fluorine, chlorine, bromine, or iodine.

In addition, it should be noted that, unless clearly stated otherwise, the description involving "...independently" in the present disclosure should be understood in a broad sense, meaning that the described individuals are independent of each other and may be independently the same or different specific groups. In more detail, the description involving "...independently" can either mean that among different groups, the specific options expressed by the same symbols do not affect each other, or it can mean that in the same group, the specific options expressed by the same symbols do not affect each other.

### Beneficial Effects

According to the embodiments of the present disclosure, the present disclosure at least has at least one of the following technical effects:
1) P2X3 antagonists with novel structures, excellent pharmacokinetic properties, and good efficacy or druggability are provided and can be used for effectively treating P2X3-related diseases and disorders;
2) According to the examples of the present disclosure, the compounds of the present disclosure (especially the compounds I-1, I-27, I-28, I-29, and I-30) have better pharmacokinetic properties than the positive reference group compounds, i.e., the pharmacokinetic properties being improved significantly;
3) According to the embodiments of the present disclosure, compared with the positive reference compound, the compounds of the present disclosure (especially the compounds I-1, I-27, and I-30) have less interference with the taste sense of rats, i.e., the interference with the taste sense of rats being significantly less than that of Reference compound 1;
4) According to the embodiments of the present disclosure, compared with the positive reference compound, the compounds of the present disclosure significantly reduce the cough times of animals in the citric acid/histamine-stimulated guinea pig cough model as well as in the citric acid/ATP-stimulated guinea pig cough model, prolong the cough latency, and have good antitussive effect.

Additional aspects and advantages of the present disclosure will be set forth, in part, from the following description, and in part will become apparent from the following description, or may be learned by practice of the present disclosure.

### BRIEF DESCRIPTION OF THE DRAWINGS

FIG. 1 shows consumptions of water and an aqueous quinine solution of rats in different administration groups after compound administration according to an embodiment of the present disclosure.
FIG. 2 shows cough times of histamine/citric acid-stimulated guinea pigs in different administration groups after compound administration according to an embodiment of the present disclosure.
FIG. 3 shows cough times of ATP/citric acid-stimulated guinea pigs in different administration groups after compound administration according to an embodiment of the present disclosure.

### DETAILED DESCRIPTION

Solutions of the present disclosure will be explained below in connection with the examples. It will be appreciated by those skilled in the art that the following examples are merely illustrative of the present disclosure and should not be construed as limiting the scope of the present disclosure. Techniques or conditions that are not specified in the examples shall be performed in accordance with the techniques or conditions described in the literatures in the art or in accordance with the product instruction. Reagents or instruments without indicating the manufacturer are all common products that are commercially available.

Structures of the compounds of the present application are identified by nuclear magnetic resonance (NMR) and/or mass spectrometry (MS), unless otherwise specified. The unit of NMR shift is 10⁻⁶ (ppm). Solvents for NMR were deuterated dimethyl sulfoxide, deuterated chloroform, deuterated methanol, etc., and internal standard was tetramethylsilane (TMS).

Abbreviations in the present disclosure are defined as follows:
M: molar concentration, for example, 1M hydrochloric acid represents 1 mol/L hydrochloric acid solution
N: equivalent concentration, for example, 2N hydrochloric acid represents 2 mol/L hydrochloric acid solution
T₃P: propyl phosphate tricyclic anhydride, i.e., 2, 4, 6-tripropyl-1, 3, 5, 2, 4, 6-trioxatriphosphine-2, 4, 6-trioxide or propylphosphonic acid anhydride
TsCl: p-toluenesulfonyl chloride
Et₃N: triethylamine
DMF: N, N-dimethylformamide
LC-MS: Liquid chromatography-mass spectrometry
DCM: dichloromethane
DMSO: dimethyl sulfoxide
DMAP: 4-dimethylaminopyridine
DIPEA: also written as DIEA, diisopropylethylamine, i.e., N, N-diisopropylethylamine
DIAD: diisopropyl azodicarboxylate
HEPES: 4-(2-hydroxyethyl)piperazine-1-ethanesulfonic acid
NIS: N-Iodosuccinimide
PPh₃: triphenylphosphine
T₃P: propyl phosphate tricyclic anhydride, i.e., 2, 4, 6-tripropyl-1, 3, 5, 2, 4, 6-trioxatriphosphine-2, 4, 6-trioxide or propylphosphonic acid anhydride
THF: tetrahydrofuran
TLC: Thin Layer Chromatography
IC₅₀: half inhibitory concentration, indicating a concentration at which half of the maximal inhibitory effect is achieved.

Unless otherwise indicated, the compounds exemplified herein are named and numbered using ChemBioDraw Ultra 14.0.

### Reference Example 1: Reference compound 1 and preparation thereof

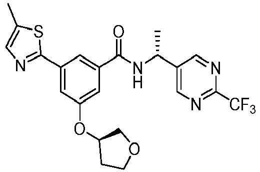

Reference compound 1 was synthesized with reference to patent application WO 2016/091776.

"Reference compound 1" referred hereinafter refers to the compound as described in Reference Example 1.

### Reference Example 2: Reference compound 2 and preparation thereof

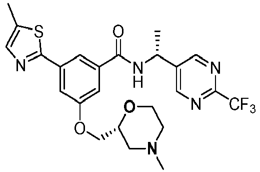

Reference compound 2 was synthesized with reference to patent application WO 2016/091776.

"Reference compound 2" referred hereinafter refers to the compound as described in **Reference** Example 2.

### Example 1: preparation of target compound 1-1

### 2-fluoro-3-(5-methylthiazol-2-yl)-5-(((R)-tetrahydrofuran-3-yl)oxy)-N-((R)-1-(2-(t rifluoromethyl)pyrimidin-5-yl)ethyl)benzamide (target compound I-1)

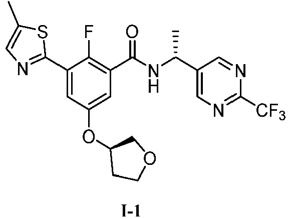

The synthesis scheme for target compound 1-1 is shown below:

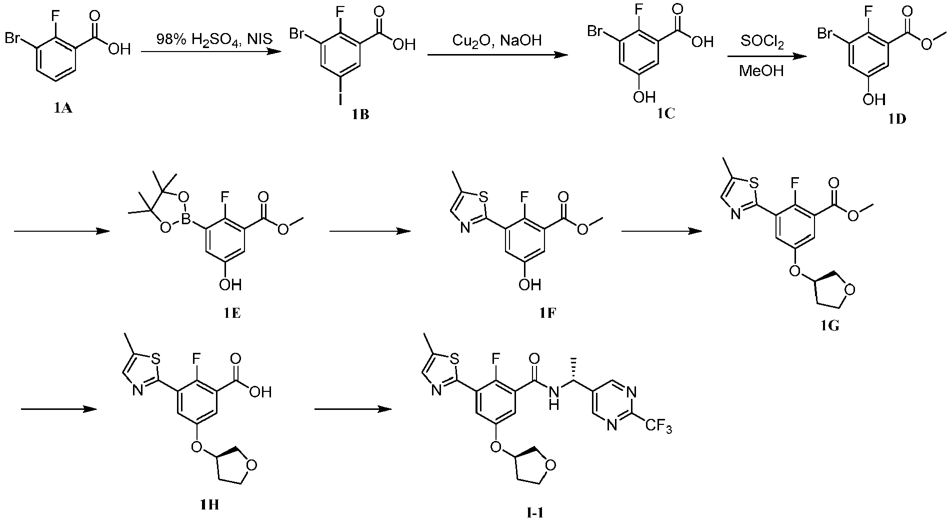

### First step: synthesis of 3-bromo-2-fluoro-5-iodobenzoic acid

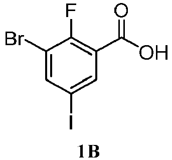

3-bromo-2-fluorobenzoic acid (10 g, 45.7 mmol) was dissolved in concentrated sulfuric acid (40 mL). NIS (10.27 g, 45.7 mmol) was added in batches at 0°C. The mixture was stirred at room temperature for three hours and then quenched with ice water (200 mL), followed by filtering. After the filter cake was washed five times with water (200 mL), vacuum drying was performed to obtain 3-bromo-2-fluoro-5-iodobenzoic acid, a white solid (10.9 g, yield 69.2 %).

### Second step: synthesis of 3-bromo-2-fluoro-5-hydroxybenzoic acid

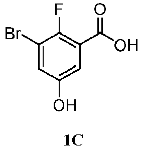

Cuprous oxide (0.656 g, 4.74 mmol) was added to a solution of 3-bromo-2-fluoro-5-iodobenzoic acid (10.9 g, 31.6 mmol) and sodium hydroxide (6.32 g, 158 mmol) in water (100 mL), and the mixture reacted overnight at 100 °C. The mixture, after being cooled to room temperature, was filtrated, and the filtrate was adjusted to pH=1 with a 2M hydrochloric acid solution, followed by extraction with ethyl acetate (60 mL×3). The organic phase was concentrated to dryness to obtain 3-bromo-2-fluoro-5-hydroxybenzoic acid, a yellow solid (7.2 g, yield 96.8 %).

### Third step: synthesis of methyl 3-bromo-2-fluoro-5-hydroxybenzoate

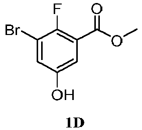

Thionyl chloride (10.9 g, 91.8 mmol) was added to a solution of 3-bromo-2-fluoro-5-hydroxybenzoic acid (7.2 g, 30.6 mmol) in methanol (120 mL), and stirred at 55 °C for 16 hours. The solvent was then removed under reduced pressure, and thus the mixture was concentrated to obtain a solid compound, methyl 3-bromo-2-fluoro-5-hydroxybenzoate (3.1 g, yield 40.8 %), which was used in the next step without further purification.

### Fourth step: synthesis of methyl 2-fluoro-5-hydroxy-3-(4, 4, 5, 5-tetramethyl-1, 3, 2-dioxaborolan-2-yl)benzoate

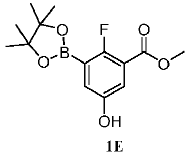

Methyl 3-bromo-2-fluoro-5-hydroxybenzoate (3.1 g, 12.45 mmol), bis (pinacolato)diboron (3.48 g, 13.69 mmol), and potassium acetate (3.67 g, 37.3 mmol) were dissolved in 1, 4-dioxane (50mL). The solution was degassed with a stream of nitrogen for 2 min. Pd(dppf)Cl₂ (0.455 g, 0.622 mmol) was added, and the obtained solution was degassed with a stream of nitrogen for another 2 min. Then, the reaction mixture was stirred at 100 °C for 16 hours. The reaction mixture was filtrated and concentrated in vacuum, and the residue was separated and purified by silica gel column to obtain methyl 2-fluoro-5-hydroxy-3-(4, 4, 5, 5-tetramethyl-1, 3, 2-dioxaborolan-2-yl )benzoate, a white solid (3.4 g, yield 92%).

### Fifth step: synthesis of methyl 2-fluoro-5-hydroxy-3-(5-methylthiazol-2-yl)benzoate

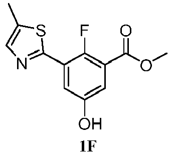

At room temperature, Pd(dppf)Cl₂ (1.260 g, 1.722 mmol) was added to a mixture solution of methyl 2-fluoro-5-hydroxy-3-(4, 4, 5, 5-tetramethyl-1, 3, 2-dioxaborolan-2-yl)benzoate (3.4 g, 11.48 mmol), 2-bromo-5-methylthiazole (2.453 g, 13.78 mmol), and potassium carbonate (3.81 g, 27.6 mmol) in THF (30 mL) and water (10 mL). After three times of vacuum nitrogen replacement, the mixture reacted at 90 °C for 16 h. The mixture was diluted with water (30 mL), followed by extraction with ethyl acetate (40 mL×3). The organic phase was concentrated to dryness. The residue was separated and purified by silica gel column to obtain methyl 2-fluoro-5-hydroxy-3-(5-methylthiazol-2-yl)benzoate, a yellow solid (1.41 g, yield 45.9 %).
LC-MS, M/Z: 268.2 [M+H]⁺

### Sixth step: synthesis of methyl (R)-2-fluoro-3-(5-methylthiazol-2-yl)-5-((tetrahydrofuran-3-yl)oxy)benzoate

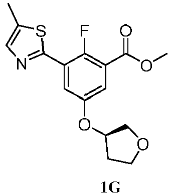

Under nitrogen protection, cesium carbonate (2.58 g, 7.91 mmol) was added to a solution of methyl 2-fluoro-5-hydroxy-3-(5-methylthiazol-2-yl)benzoate (1.41 g, 5.28 mmol) and (S)-3-tosyltetrahydrofuran (1.53 g, 6.33 mmol) in DMF (15 mL). The mixture reacted at 90°C for 18 h, and then diluted with water (20 mL), followed by extraction with ethyl acetate (20 mL×3). The organic phase was concentrated to dryness. The residue was separated and purified by silica gel column to obtain methyl (R)-2-fluoro-3-(5-methylthiazol-2-yl)-5-((tetrahydrofuran-3-yl)oxy)benzoate, a yellow solid (0.4 g, yield 22.5%).
LC-MS, M/Z: 338.4 [M+H]⁺

### Seventh step: synthesis of (R)-2-fluoro-3-(5-methylthiazol-2-yl)-5-((tetrahydrofuran-3-yl)oxy)benzoic acid

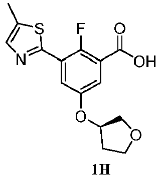

At room temperature, lithium hydroxide monohydrate (100 mg, 2.371 mmol) was added to a mixture solution of methyl (R)-2-fluoro-3-(5-methylthiazol-2-yl)-5-((tetrahydrofuran-3-yl)oxy)benzoate (400 mg, 1.186 mmol) in THF (6 mL), water (2 mL), and MeOH (2 mL). The mixture was stirred at room temperature for 3 hours. Then, methanol and tetrahydrofuran were removed by rotary evaporation under reduced pressure. The aqueous phase was adjusted to a pH of about 4 with 2M hydrochloric acid, followed by extraction with dichloromethane (10 mL×2). The organic phase was dried with anhydrous sodium sulfate, filtrated, and concentrated to obtain (R)-2-fluoro-3-(5-methylthiazol-2-yl)-5-((tetrahydrofuran-3-yl)oxy)benzoic acid, a white solid (300 mg, 78 % yield).
LC-MS, M/Z: 324.3 [M+H]⁺

### Eighth step: synthesis of 2-fluoro-3-(5-methylthiazol-2-yl)-5-(((R)-tetrahydrofuran-3-yl)oxy)-N-((R)-1-(2-(trifluoromet hyl)pyrimidin-5-yl) ethyl)benzamide (target compound I-1)

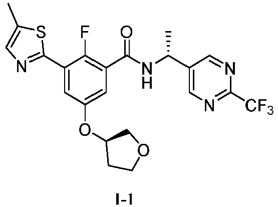

In ice bath, propylphosphonic acid anhydride (0.59 g, 0.928 mmol, 50% N, N-dimethylformamide solution) was added dropwise to a solution of (R)-2-fluoro-3-(5-methylthiazol-2-yl)-5-((tetrahydrofuran-3-yl)oxy)benzoic acid (120 mg, 0.371 mmol), (R)-1-(2-(trifluoromethyl)pyrimidin-5-yl)ethyl-1-amine hydrochloride (77 mg, 0.445 mmol), and N, N-diisopropylethylamine (240 mg, 1.856 mmol) in N, N-dimethylformamide (6 mL). The mixture was stirred and reacted overnight at room temperature, and diluted by adding 20 mL of water, followed by extraction with ethyl acetate (10 mL×2). The organic phase was concentrated to dryness. The residue was separated and purified by silica gel column to obtain 2-fluoro-3-(5-methylthiazol-2-yl)-5-(((R)-tetrahydrofuran-3-yl)oxy)-N-((R)-1-(2-(trifluoromet hyl)pyrimidin-5-yl) ethyl)benzamide, a white solid (**I-1**) (40 mg, yield 21.7%).

¹H NMR (400 MHz, CDCl₃) δ 8.93 (d, 2H), 7.84 (dd, 1H), 7.63-7.60 (d, 1H), 7.49 (dd, 1H), 7.09 (dd, 1H), 5.45-5.32 (m, 1H), 5.05-4.95 (m, 1H), 4.09-3.85 (m, 4H), 2.57 (d, 3H), 2.30-2.06 (m, 2H), 1.73 (d, 3H).

LC-MS, M/Z: 497.2 [M+H]⁺.

### Example 2: preparation of target compound I-2

### 2-fluoro-5-(5-methylthiazol-2-yl)-3-(((R)-tetrahydrofuran-3-yl)oxy)-N-((R)-1-(2-(t rifluoromethyl)pyrimidin-5-yl)ethyl)benzamide (target compound I-2)

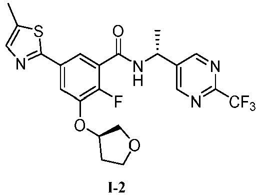

The synthesis scheme for target compound I-2 is shown below:

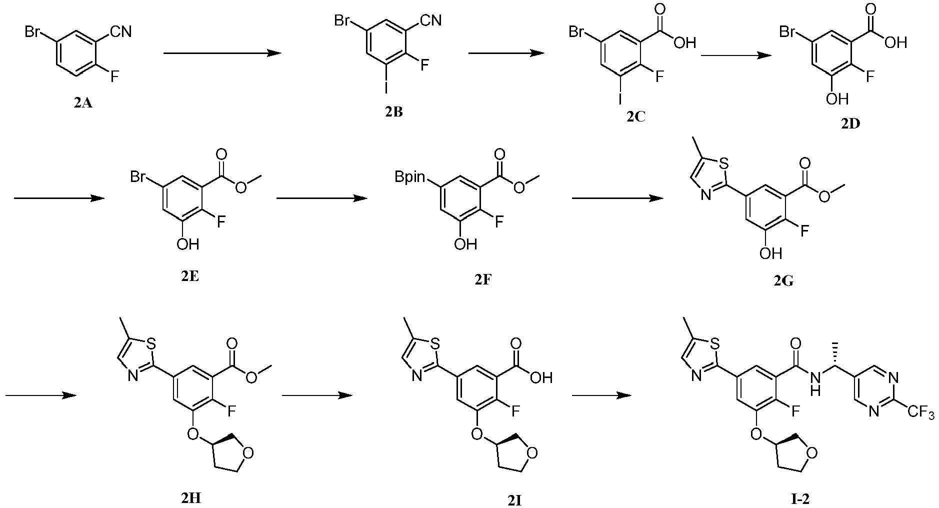

### First step: synthesis of 5-bromo-2-fluoro-3-iodobenzonitrile

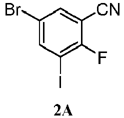

At room temperature, 2, 2, 6, 6-tetramethylpiperidine (8.86 mL, 52.5 mmol) was dissolved in THF (100 mL). At -20°C, n-butyllithium (21.00 mL, 52.5 mmol, 2.5 M n-hexane solution) was slowly added dropwise within more than 30 min, and the mixture was stirred and reacted at -10°C for 1 h. Then, the mixture was cooled to -70° C and added with diethylzinc (58.0 mL, 58.0 mmol, 1 M n-hexane solution). The mixture was slowly warmed up to 0°C and stirred at 0°C for 2 h. Then, the mixture was cooled to -70°C and added with a solution of 5-bromo-2-fluorobenzonitrile (10 g, 50.0 mmol) in THF (50 mL), and reacted at -70°C for 0.5 h. Then, the mixture was heated to -30°C and stirred for 5 h. The reaction system was cooled to -70°C again, added with a solution of iodine (44.4 g, 175 mmol) in THF (200 mL), and the reaction mixture was warmed to room temperature and stirred overnight for reaction. The reaction was quenched by adding saturated sodium bisulfite solution (50 mL), followed by filtering. The filtrate was extracted with ethyl acetate (200 mL×2), and the organic phases were combined and washed respectively with saturated sodium bisulfite solution (100 mL) and saturated salt water (100 mL). The organic phase was dried with anhydrous sodium sulfate, and concentrated under reduced pressure. The obtained solid was recrystallized with an ethyl acetate/petroleum ether system to obtain 5-bromo-2-fluoro-3-iodobenzonitrile, which was directly used in the next step.

### Second step: synthesis of 5-bromo-2-fluoro-3-iodobenzoic acid

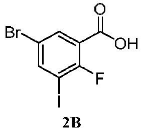

The 5-bromo-2-fluoro-3-iodobenzonitrile obtained in the previous step, was dissolved in concentrated sulfuric acid (30 mL), and the mixture reacted overnight at 120°C. Then, the mixture was diluted with iced water (100 mL) and extracted with ethyl acetate (50 mL×3). The organic phases were combined and concentrated under reduced pressure to obtain a solid of 5-bromo-2-fluoro-3-iodobenzoic acid (11.2 g, yield in two steps 64.9 %).

For the subsequent synthesis thereof, refer to Example 1.

¹H NMR (400 MHz, CDCl₃) δ 8.94 (s, 2H), 7.91 (dd, 1H), 7.78 (dd, 1H), 7.48 (d, 1H), 7.05 (dd, 1H), 5.48-5.27 (m, 1H), 5.15-5.09 (m, 1H), 4.09-3.92 (m, 4H), 2.51 (d, 3H), 2.33-2.18 (m, 2H), 1.70 (d, 3H).

LC-MS, M/Z: 497.2 [M+H]⁺

### Example 3: preparation of target compound I-3

### 4-fluoro-3-(5-methylthiazol-2-yl)-5-(((R)-tetrahydrofuran-3-yl)oxy)-N-((R)-1-(2-(t rifluoromethyl)pyrimidin-5-yl)ethyl)benzamide (target compound I-3)

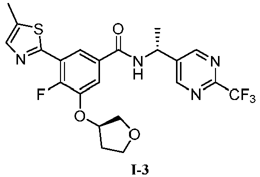

The synthesis scheme for target compound **I-3** is shown below:

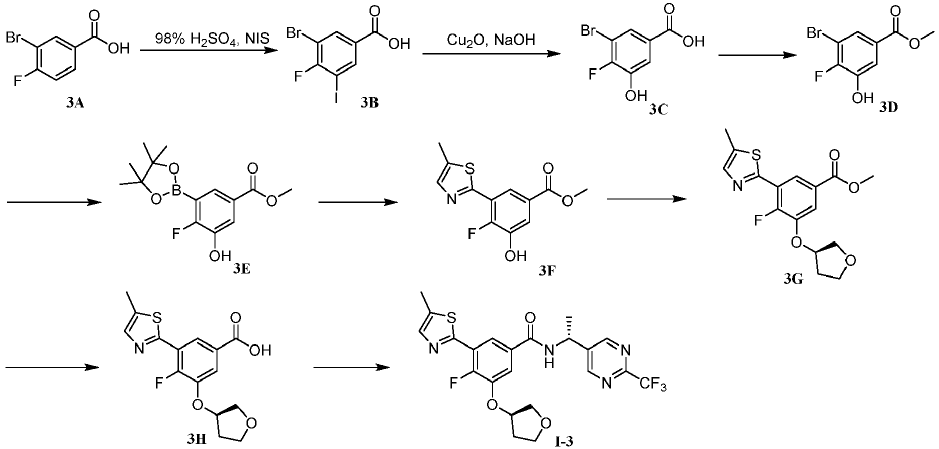

For the synthesis thereof, refer to Example 1.

¹H NMR (400 MHz, CDCl₃) δ 8.91 (d, 2H), 8.15 (dd, 1H), 7.62-7.59 (m, 1H), 7.55-7.46 (m, 1H), 6.88 (d, 1H), 5.39-5.29 (m, 1H), 5.09-5.04 (m, 1H), 4.08-4.00 (m, 3H), 3.93 (td, 1H), 2.57 (d, 3H), 2.32-2.16 (m, 2H), 1.72 (d, 3H).

LC-MS, M/Z: 497.2 [M+H]⁺.

### Example 4: preparation of target compound I-4

### 2-fluoro-5-((trans-3-hydroxybutan-2-yl)oxy)-3-(5-methylthiazol-2-yl)-N-((R)-1-(2 -(trifluoromethyl)pyrimidin-5-yl)ethyl)benzamide (target compound I-4)

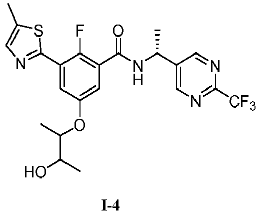

The synthesis scheme for target compound I-4 is shown below:

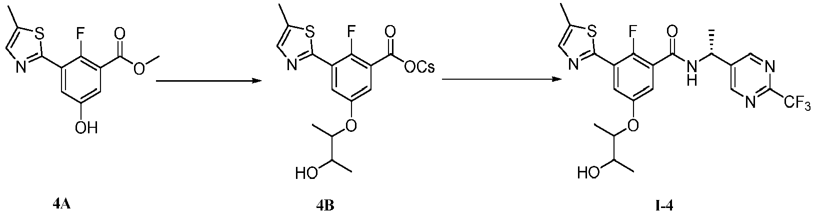

### First step: synthesis of cesium 2-fluoro-5-((trans-3-hydroxybutan-2-yl)oxy)-3-(5-methylthiazol-2-yl)benzoate (4B)

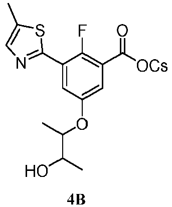

Methyl 2-fluoro-5-hydroxy-3-(5-methylthiazol-2-yl)benzoate (**4A**) (0.20 g, 0.749 mmol) (for synthesis thereof, refer to Example 1) was added to DMSO (2 mL), and cis-2, 3-dimethyloxirane (170.1 mg, 2.38 mmol) and cesium carbonate (732.5 mg, 2.38 mmol) were added. The mixture was heated to 100 °C, and stirred overnight for reaction. The reaction mixture was diluted by adding water (50 mL) and freeze-dried to obtain a compound cesium 2-fluoro-5-((trans-3-hydroxybutan-2-yl)oxy)-3-(5-methylthiazol-2-yl)benzoic acid (**4B**), an off-white solid crude product (1.3 g, yield 100%).

LC-MS, M/Z: 325.4 [M+H]⁺.

### Second step: synthesis of 2-fluoro-5-((trans-3-hydroxybutan-2-yl)oxy)-3-(5-methylthiazol-2-yl)-N-((R)-1-(2-(trifluoro methyl)pyrimidin-5-yl)ethyl)benzamide (target compound I-4)

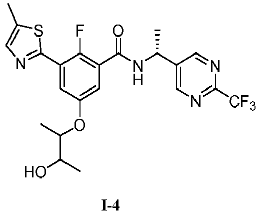

At room temperature, a crude product of cesium 2-fluoro-5-((trans-3-hydroxybutan-2-yl)oxy)-3-(5-methylthiazol-2-yl)benzoate (**4B**) (1.3 g, 0.749 mmol) was added to 3 mL of N, N-dimethylformamide, and (R)-1-(2-(trifluoromethyl)pyrimidin-5-yl)ethyl-1-amine hydrochloride (85.1 mg, 0.375 mmol) and diisopropylethylamine (96.6 mg, 0.749 mmol) were further added, followed by dropwise adding propylphosphonic acid anhydride (238.2 mg, 0.375 mmol, 50% N, N-dimethylformamide solution). After the dropwise addition, the mixture was stirred and reacted overnight at room temperature under nitrogen protection, with TLC indicating that the reaction of the raw materials was complete. The reaction mixture was diluted by adding 5 mL of water, followed by extraction with ethyl acetate (10 mL×2). The organic phase was concentrated to dryness, and the residue was separated and purified by silica gel column (petroleum ether: ethyl acetate (V/V) =2: 1) to obtain 2-fluoro-5-((trans-3-hydroxybutan-2-yl)oxy)-3-(5-methylthiazol-2-yl)-N-((R)-1-(2-(trifluoro methyl)pyrimidin-5-yl)ethyl)benzamide (**I-4**), an off-white solid (21 mg, yield 11.3%).

¹H NMR (400 MHz, CDCl₃) δ 8.96 (s, 2H), 7.90-7.88 (m, 1H), 7.62 (s, 1H), 7.56-7.53 (m, 1H), 7.10-7.06 (m, 1H), 5.41-5.38 (m, 1H), 4.43-4.40 (m, 1H), 4.03-4.02 (m, 1H), 2.58 (d, 3H), 1.95-1.94 (m, 1H), 1.74 (d, 2H), 1.29-1.24 (m, 6H).

LC-MS, M/Z: 499.5[M+H]⁺.

### Example 5: preparation of target compound I-5

### 2-fluoro-5-((cis-3-hydroxybutan-2-yl)oxy)-3-(5-methylthiazol-2-yl)-N-((R)-1-(2-(t rifluoromethyl)pyrimidin-5-yl)ethyl)benzamide (target compound I-5)

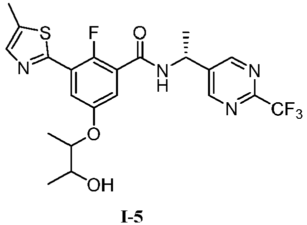

The synthesis scheme for target compound I-5 is shown below:

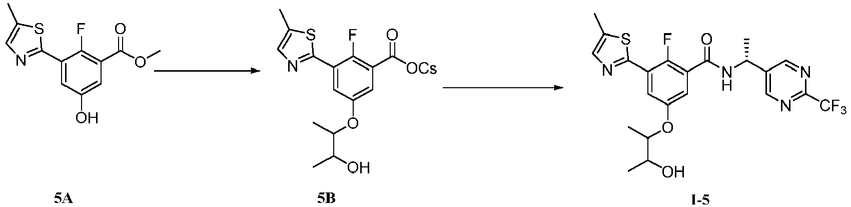

For the synthesis thereof, refer to Example 4.

¹H NMR (400 MHz, CDCl₃) δ 8.96 (s, 2H), 7.90-7.88 (m, 1H), 7.62 (s, 1H), 7.56-7.53 (m, 1H), 7.10-7.06 (m, 1H), 5.41-5.38 (m, 1H), 4.43-4.40 (m, 1H), 4.03-4.02 (m, 1H), 2.58 (d, 3H), 1.95-1.94 (m, 1H), 1.74 (d, 2H), 1.29-1.24 (m, 6H).

LC-MS, M/Z: 499.5 [M+H]⁺.

### Example 6: preparation of target compound I-6

### 3-(5-chlorothiazol-2-yl)-2-fluoro-5-((trans-3-hydroxybutan-2-yl)oxy)-N-((R)-1-(2-(trifluoromethyl)pyrimidin-5-yl)ethyl)benzamide (target compound I-6)

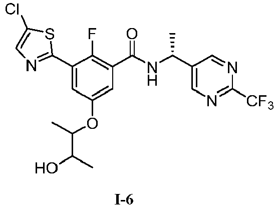

The synthesis scheme for target compound I-6 is shown below:

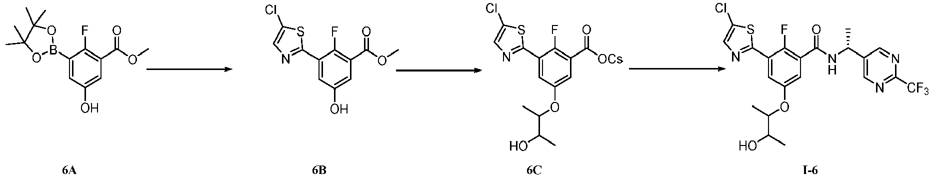

### First step: synthesis of methyl 3-(5-chlorothiazol-2-yl)-2-fluoro-5-hydroxybenzoate (6B)

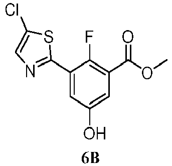

At room temperature, Pd(dppf)Cl₂ (0.544 g, 0.743 mmol) was added to a mixture solution of methyl 2-fluoro-5-hydroxy-3-(4, 4, 5, 5-tetramethyl-1, 3, 2-dioxaborolan-2-yl)benzoate (2.2 g, 7.43 mmol) (the for synthesis thereof, refer to Example 1), 2-bromo-5-chlorothiazole (1.475 g, 7.43 mmol), and sodium carbonate (1.575 g, 14.86 mmol) in 1, 4-dioxane (40 mL) and water (10 mL). After three times of vacuum nitrogen replacement, the mixture reacted at 90°C for 18 h. Then, the mixture was diluted by adding water (80 mL), and extracted with ethyl acetate (100 mL×3). The organic phase was concentrated to dryness, and the residue was separated and purified by silica gel column to obtain methyl 3-(5-chlorothiazol-2-yl)-2-fluoro-5-hydroxybenzoate (6B), a yellow solid (0.77 g, yield 36.0 %).
LC-MS, M/Z: 288.1 [M+H]⁺

### First step: synthesis of cesium 3-(5-chlorothiazol-2-yl)-2-fluoro-5-((trans-3-hydroxybutan-2-yl)oxy)benzoate (6C)

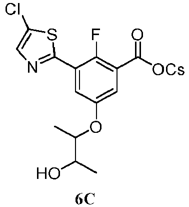

methyl 3-(5-chlorothiazol-2-yl)-2-fluoro-5-hydroxybenzoate (6B) (0.22 g, 0.749 mmol) was added to DMSO (2 mL), and cis-2, 3-dimethyloxirane (170.1 mg, 2.38 mmol) and cesium carbonate (732.5 mg, 2.38 mmol) were added. The mixture was heated to 100°C, and stirred overnight for reaction. The mixture was diluted by adding water (50 mL) and freeze-dried to obtain a compound cesium 3-(5-chlorothiazol-2-yl)-2-fluoro-5-((trans-3-hydroxybutan-2-yl)oxy)benzoate (**6C**), an off-white solid crude product (1.4 g, crude product).

LC-MS, M/Z: 345.8 [M+H-Cs]⁺.

### Second step: synthesis of 3-(5-chlorothiazol-2-yl)-2-fluoro-5-((trans-3-hydroxybutan-2-yl)oxy)-N-((R)-1-(2-(trifluorom ethyl)pyrimidin-5-yl)ethyl)benzamide (target compound I-6)

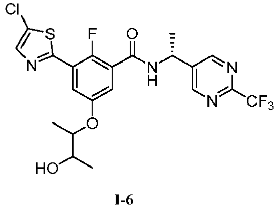

At room temperature, cesium 3-(5-chlorothiazol-2-yl)-2-fluoro-5-((trans-3-hydroxybutan-2-yl)oxy)benzoate (**6C**) (1.4 g, 0.749 mmol) was added to 3 mL of N, N-dimethylformamide, and then added with (R)-1-(2-(trifluoromethyl)pyrimidin-5-yl)ethyl-1-amine hydrochloride (85.1 mg, 0.375 mmol) and diisopropylethylamine (96.6 mg, 0.749 mmol). Propylphosphonic acid anhydride (238.2 mg, 0.375 mmol, 50% N, N-dimethylformamide solution) was added dropwise. After the dropwise addition, the mixture was stirred and reacted overnight at room temperature under nitrogen protection, with TLC (PE: EA=2: 1) indicating that the reaction of the raw materials was complete. The mixture was diluted by adding 5 mL of water, and extracted with ethyl acetate (10 mL×2). The organic phase was concentrated to dryness the residue was separated and purified by silica gel column (petroleum ether: ethyl acetate (V/V) =2: 1) to obtain 3-(5-chlorothiazol-2-yl)-2-fluoro-5-((trans-3-hydroxybutan-2-yl)oxy)-N-((R)-1-(2-(trifluorom ethyl)pyrimidin-5-yl)ethyl)benzamide (**I-6**), an off-white solid (33 mg, yield 8.5%).

¹H NMR (400 MHz, MeOD) δ 9.04 (s, 2H), 7.92-7.89 (m, 1H), 7.87-7.86 (m, 1H), 7.30-7.28 (m, 1H), 5.37-5.34 (m, 1H), 4.35-4.33 (m, 1H), 3.87-3.84 (m, 1H), 1.68 (d, 3H), 1.30-1.26 (m, 6H).
LC-MS, M/Z: 519.9[M+H]⁺

### Example 7: preparation of target compound I-7

### 3-(5-chlorothiazol-2-yl)-2-fluoro-5-((cis-3-hydroxybutan-2-yl)oxy)-N-((R)-1-(2-(tr ifluoromethyl)pyrimidin-5-yl)ethyl)benzamide (target compound I-7)

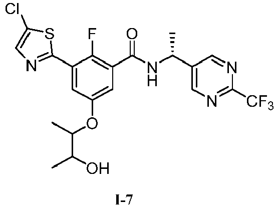

The synthesis scheme for target compound 1-7 is shown below:

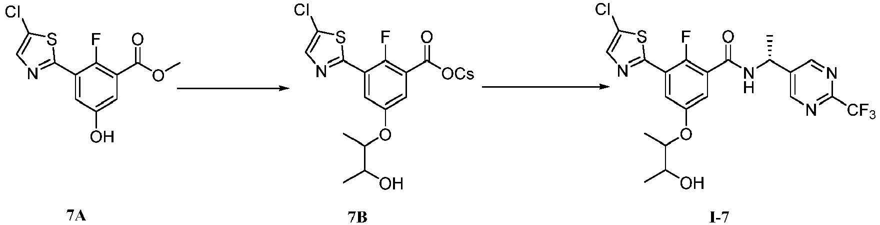

For the synthesis thereof, refer to Example 6
¹H NMR (400 MHz, MeOD) δ 9.04 (s, 2H), 7.92-7.89 (m, 1H), 7.87-7.86 (m, 1H), 7.30-7.28 (m, 1H), 5.37-5.34 (m, 1H), 4.35-4.33 (m, 1H), 3.87-3.84 (m, 1H), 1.68 (d, 3H), 1.30-1.26 (m, 6H).
LC-MS, M/Z: 519.9[M+H]⁺.

### Example 8: preparation of target compound I-8

### 3-(5-chlorothiazol-2-yl)-2-fluoro-5-(((R)-tetrahydrofuran-3-yl)oxy)-N-((R)-1-(2-(t rifluoromethyl)pyrimidin-5-yl)ethyl)benzamide (I-8)

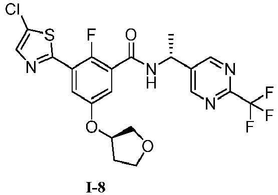

The synthesis scheme for compound I-8 is shown below:

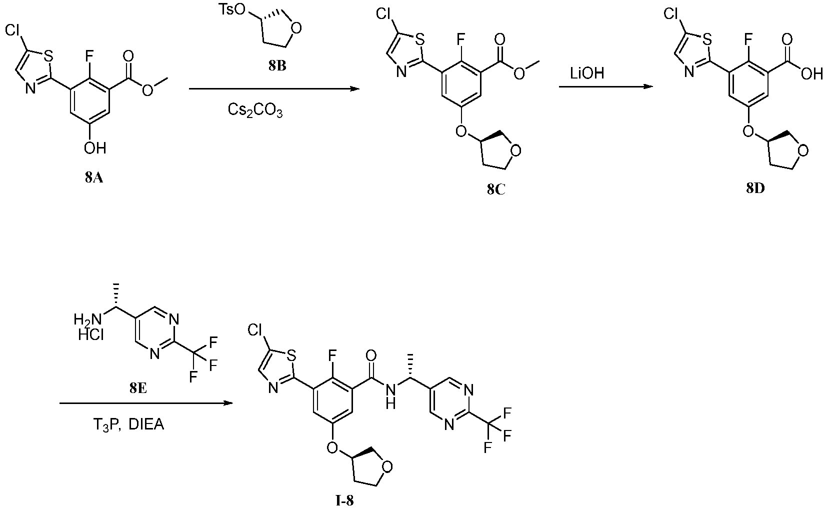

### First step: synthesis of methyl (R)-3-(5-chlorothiazol-2-yl)-2-fluoro-5-((tetrahydrofuran-3-yl)oxy)benzoate (8C)

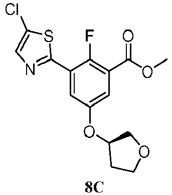

Cesium carbonate (340 mg, 1.043 mmol) was added into a solution of methyl 3-(5-chlorothiazol-2-yl)-2-fluoro-5-hydroxybenzoate (**8A**) (200 mg, 0.695 mmol) (for synthesis thereof, refer to Example 6) in N, N-dimethylformamide (4 mL), and the mixture was stirred at room temperature for 10 min. (S)-tetrahydrofuran-3-yl 4-methylbenzenesulfonate (**8B**) (202 mg, 0.834 mmol) was added. Under nitrogen protection, the mixture was heated to 90 °C and reacted for 3 hours, and then cooled to room temperature. The reaction was quenched by adding water (50 mL), followed by extraction with ethyl acetate (30 mL×3). The organic phases were combined, dried over anhydrous sodium sulfate, and then concentrated. The residue was purified with silica gel column (petroleum ether: ethyl acetate (V/V) =2: 1) to obtain methyl (R)-3-(5-chlorothiazol-2-yl)-2-fluoro-5-((tetrahydrofuran-3-yl)oxy)benzoate (**8C**), a white solid (140 mg, yield 56.3%).

LC-MS, M/Z: 358.1 [M+H]⁺.

### Second step: synthesis of (R)-3-(5-chlorothiazol-2-yl)-2-fluoro-5-((tetrahydrofuran-3-yl)oxy)benzoic acid (8D)

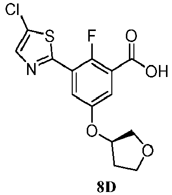

Methyl (R)-3-(5-chlorothiazol-2-yl)-2-fluoro-5-((tetrahydrofuran-3-yl)oxy)benzoate (**8C**) (140 mg, 0.391 mmol) was dissolved in methanol (2 mL) and tetrahydrofuran (2 mL), and then added with lithium hydroxide monohydrate (65.7 mg, 1.565 mmol) and water (2 mL). The mixture was stirred and reacted at room temperature for 18 h. The reaction solution was adjusted to pH<2 with 2N diluted hydrochloric acid, and extracted with ethyl acetate (20mL × 3). The organic phases were combined, dried over anhydrous sodium sulfate, and concentrated to obtain a crude product of (R)-3-(5-chlorothiazol-2-yl)-2-fluoro-5-((tetrahydrofuran-3-yl)oxy)benzoic acid (**8D**) (135 mg, yield 100%), which was directly put into the next step of reaction.

LC-MS, M/Z: 344.1 [M+H]⁺.

### Third step: synthesis of 3-(5-chlorothiazol-2-yl)-2-fluoro-5-(((R)-tetrahydrofuran-3-yl)oxy)-N-((R)-1-(2-(trifluoromet hyl)pyrimidin-5-yl)ethyl)benzamide (I-8)

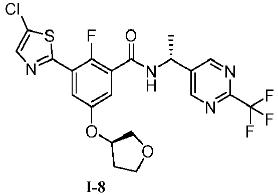

(R)-3-(5-chlorothiazol-2-yl)-2-fluoro-5-((tetrahydrofuran-3-yl)oxy)benzoic acid (**8D**) (135 mg, 0.393 mmol), (R)-1-(2-(trifluoromethyl)pyrimidin-5-yl)ethyl-1-amine hydrochloride (**8E**) (89 mg, 0.393 mmol), N, N-diisopropylethylamine (254 mg, 1.964 mmol), and N, N-dimethylformamide (4 mL) were successively added into a reaction flask. The mixture was cooled to about 0 °C, and added with propylphosphonic acid anhydride (50% of N, N-dimethylformamide solution, 625 mg, 0.982 mmol) dropwise. After the dropwise addition, the mixture was heated to room temperature and reacted for 3h. The reaction was quenched by adding water (30 mL), followed by extraction with ethyl acetate (30 mL × 3). The organic phases were combined, dried over anhydrous sodium sulfate, and then concentrated. The residue was separated and purified with a silica gel plate (petroleum ether: ethyl acetate (V/V) =1: 1) to obtain a white solid of 3-(5-chlorothiazol-2-yl)-2-fluoro-5-(((R)-tetrahydrofuran-3-yl)oxy)-N-((R)-1-(2-(trifluoromet hyl)pyrimidin-5-yl)ethyl)benzamide **(I-8)** (6.8 mg, yield 3.4%).

¹H NMR (400 MHz, DMSO-d₆) δ 9.25-9.24 (d, 1H), 9.12 (s, 2H), 8.12-8.11 (d, 1H), 7.73-7.71 (m, 1H), 7.30-7.28 (m, 1H), 5.29-5.26 (m, 1H), 5.19-5.16 (m, 1H), 3.90-3.77 (m, 4H), 2.26-2.21 (m, 1H), 2.02-1.97 (m, 1H), 1.58-1.56 (d, 3H).

LC-MS, M/Z: 517.1 [M+H]⁺.

**Example 9:** preparation of target compound I-9

### 2-fluoro-5-(((S)-4-methylmorpholin-3-yl)methoxy)-3-(5-methylthiazol-2-yl)-N-(( R)-1-(2-(trifluoromethyl)pyrimidin-5-yl)ethyl)benzamide (target compound I-9)

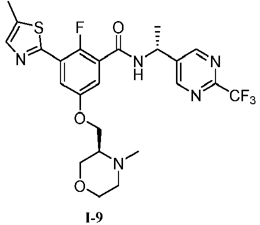

The synthesis scheme for target compound I-9 is shown below:

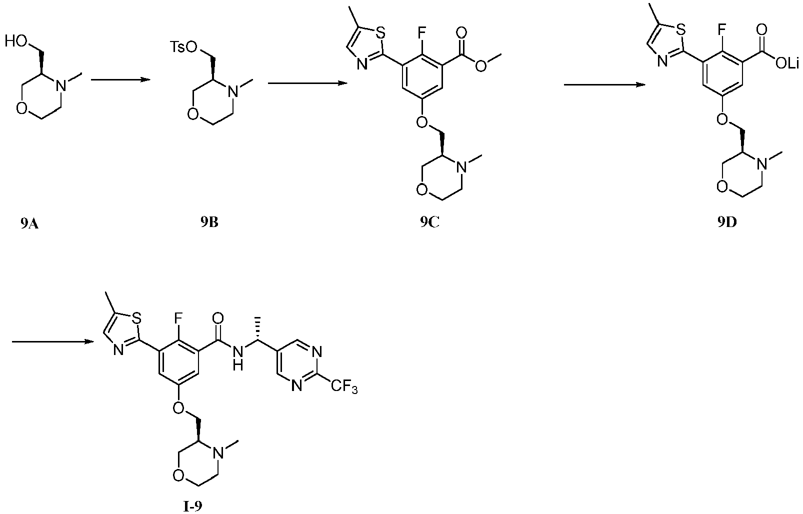

### First step: synthesis of methyl ((S)-4-methylmorpholin-3-yl)methyl 4-methylbenzenesulfonate (9B)

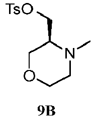

(R)-(4-methylmorpholin-3-yl)methanol (**9A**) (1.31 g, 10.0 mmol) was added to dichloromethane (20 mL). Then, triethylamine (2.01 g, 20 mmol) and 4-dimethylaminopyridine (244 mg, 2 mmol) were added, and the mixture was cooled to 0°C. After p-toluenesulfonyl chloride (2.10 g, 11.0 mmol) was added, the mixture was stirred overnight at room temperature for reaction. The mixture was diluted by adding dichloromethane (50 mL), followed by concentration with silica gel (50 mL). The residue was separated and purified by silica gel column (ethyl acetate: methanol: ammonia (V/V) =10: 1: 0.03) to obtain oily compound of ((S)-4-methylmorpholin-3-yl)methyl 4-methylbenzenesulfonate (**9B**) (0.6 g, yield 21.1%).

### Second step: synthesis of methyl (S)-2-fluoro-5-((4-methylmorpholin-3-yl)methoxy)-3-(5-methylthiazol-2-yl)benzoate (9C)

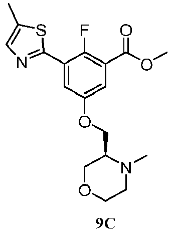

Compound methyl 2-fluoro-5-hydroxy-3-(5-methylthiazol-2-yl)benzoate (0.20 g, 0.749 mmol) (for synthesis thereof, refer to Example 1) was added to DMF (2 mL). After cesium carbonate (0.37 g, 1.124 mmol) was added, the mixture was heated to 50 °C and stirred for 0.5h. Then, the crude product of ((S)-4-methylmorpholin-3-yl)methyl 4-methylbenzenesulfonate (**9B**) (0.33 g, 1.124 mmol) was added, and the mixture was heated to 90 °C and stirred overnight for reaction. The mixture was then cooled to room temperature and diluted by adding distilled water (10 mL), followed by extraction with ethyl acetate (10 mL×3). The organic phases were combined and washed with saturated salt water (10 mL×2). After liquid separation, the organic phase was dried over anhydrous sodium sulfate, filtrated, and concentrated to obtain an oily compound methyl (S)-2-fluoro-5-((4-methylmorpholin-3-yl)methoxy)-3-(5-methylthiazol-2-yl)benzoate (**9C**) as a crude product (90mg, yield 21.0%).

LC-MS, M/Z: 381.4 [M+H]⁺.

### Third step: synthesis of lithium (S)-2-fluoro-5-((4-methylmorpholin-3-yl)methoxy)-3-(5-methylthiazol-2-yl)benzonate (9D)

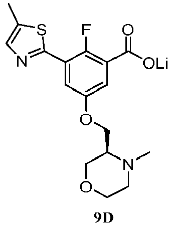

At room temperature, the crude product of methyl (S)-2-fluoro-5-((4-methylmorpholin-3-yl)methoxy)-3-(5-methylthiazol-2-yl)benzoate (**9C**) (90 mg, 0.237 mmol) was added to 0.5 mL of tetrahydrofuran solution. Then, 0.2 mL of water and lithium hydroxide (56.8 mg, 2.37 mmol) were added, and the mixture reacted overnight at room temperature for reaction, and concentrated to dryness. The residue was added with water (10 mL) for free-drying to obtain lithium (S)-2-fluoro-5-((4-methylmorpholin-3-yl)methoxy)-3-(5-methylthiazol-2-yl)benzonate (**9D**) (150 mg, yield 100%).

LC-MS, M/Z: 367.4[M+2H-Li]⁺.

### Fourth step: synthesis of 2-fluoro-5-(((S)-4-methylmorpholin-3-yl)methoxy)-3-(5-methylthiazol-2-yl)-N-((R)-1-(2-(trif luoromethyl)pyrimidin-5-yl)ethyl)benzamide (target compound I-9)

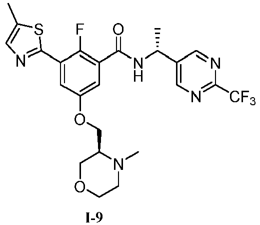

At room temperature, lithium (S)-2-fluoro-5-((4-methylmorpholin-3-yl)methoxy)-3-(5-methylthiazol-2-yl)benzonate (**9D**) (150 mg, 0.237 mmol) was added to 3 mL of N, N-dimethylformamide. Then, (R)-1-(2-(trifluoromethyl)pyrimidin-5-yl)ethyl-1-amine hydrochloride (53.9 mg, 0.237 mmol) and diisopropylethylamine (106 mg, 0.811 mmol) were added, and propylphosphonic acid anhydride (104.6 mg, 0.411 mmol, 50% N, N-dimethylformamide solution) was added dropwise. After the dropwise addition, the mixture was stirred and reacted overnight at room temperature under nitrogen protection. Then, the mixture was diluted by adding 5 mL of water and extracted with ethyl acetate (10 mL×2). The organic phase was concentrated to dryness. The residue was separated with a silica gel plate (ethyl acetate: methanol: ammonia (V/V) =10: 1: 0.02) to obtain a grey-like solid of 2-fluoro-5-(((S)-4-methylmorpholin-3-yl)methoxy)-3-(5-methylthiazol-2-yl)-N-((R)-1-(2-(trif luoromethyl)pyrimidin-5-yl)ethyl)benzamide (**I-9**) (3.6 mg, yield 2.8%).

¹H NMR (400 MHz, DMSO-d₆) δ 9.13 (s, 2H), 8.34 (s, 2H), 7.76-7.74 (m, 1H), 7.25-7.23 (m, 1H), 5.29-5.26 (m, 1H), 4.21-4.17 (m, 1H), 4.01-3.97 (m, 1H), 3.86-3.83 (m, 2H), 3.63-3.56 (m, 2H), 2.69-2.66 (m, 2H), 2.63-2.59 (s, 3H), 2.44 (s, 3H), 1.87 (s, 1H), 1.57 (d, 3H).

LC-MS, M/Z: 540.6 [M+H]⁺.

### Example 10: preparation of target compound I-10

### 2-fluoro-3-(5-methylthiazol-2-yl)-5-(((S)-tetrahydrofuran-2-yl)methoxy)-N-((R)-1 -(2-(trifluoromethyl)pyrimidin-5-yl)ethyl)benzamide (target compound I-10)

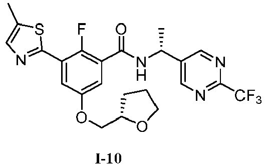

The synthesis scheme for target compound I-10 is shown below:

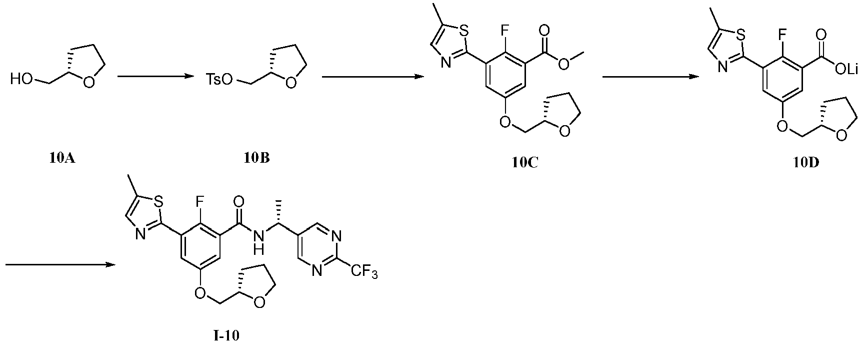

### First step: synthesis of ((S)-tetrahydrofuran-2-yl)methyl 4-methylbenzenesulfonate (10B)

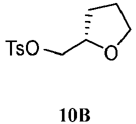

((S)-tetrahydrofuran-2-yl)methanol (**10A**) (1.02 g, 10.0 mmol) was added to dichloromethane (20 mL), and then triethylamine (2.01 g, 20 mmol) and 4-dimethylaminopyridine (244 mg, 2 mmol) were added. The mixture was cooled to 0 °C, and after p-toluenesulfonyl chloride (2.10 g, 11.0 mmol) was added, the mixture was stirred and reacted overnight at room temperature. Then, the mixture was diluted with dichloromethane (50 mL), followed by concentration with silica gel (50 mL). The residue was separated and purified by silica gel column (petroleum ether: ethyl acetate (V/V) =3: 1) to obtain a compound ((S)-tetrahydrofuran-2-yl)methyl 4-methylbenzenesulfonate (**10B**) (1.31 g, yield 50.8%).

### Second step: synthesis of methyl (S)-2-fluoro-3-(5-methylthiazol-2-yl)-5-((tetrahydrofuran-2-yl)methoxy)benzoate (10C)

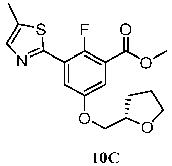

Compound methyl 2-fluoro-5-hydroxy-3-(5-methylthiazol-2-yl)benzoate (0.20 g, 0.749 mmol) (for synthesis thereof, refer to Example 1) was added to DMF (2 mL). After cesium carbonate (0.37 g, 1.124 mmol) was added, the mixture was heated to 50 °C, stirred for 0.5h, and then added with ((S)-tetrahydrofuran-2-yl)methyl 4-methylbenzenesulfonate (**10B**) (0.29 g, 1.124 mmol). The mixture was heated to 90 °C and stirred overnight for reaction. Then, the mixture was cooled to room temperature and diluted by adding distilled water (10 mL), followed by extraction with ethyl acetate (10 mL×3). The organic phases were combined and washed with saturated salt water (10 mL×2). After liquid separation, the organic phase was dried over anhydrous sodium sulfate, filtrated, and concentrated. The residue was purified with silica gel column (petroleum ether: ethyl acetate (V/V) =2: 1) to obtain an oily compound methyl (S)-2-fluoro-3-(5-methylthiazol-2-yl)-5-((tetrahydrofuran-2-yl)methoxy)benzoate (**10C**) (70 mg, yield 26.6%).

LC-MS, M/Z: 352.4 [M+H]⁺.

### Third step: synthesis of lithium (S)-2-fluoro-3-(5-methylthiazol-2-yl)-5-((tetrahydrofuran-2-yl)methoxy)benzonate (10D)

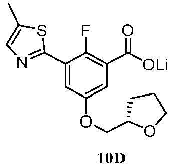

At room temperature, methyl (S)-2-fluoro-3-(5-methylthiazol-2-yl)-5-((tetrahydrofuran-2-yl)methoxy)benzoate (**10C**) (70 mg, 0.199 mmol) was added to 0.5 mL of tetrahydrofuran solution, and then 0.2 mL of water and lithium hydroxide (47.8 mg, 1.99 mmol) were added. The mixture reacted overnight at room temperature for reaction, and concentrated to dryness. The residue was added with water (10 mL) for free-drying to obtain lithium (S)-2-fluoro-3-(5-methylthiazol-2-yl)-5-((tetrahydrofuran-2-yl)methoxy)benzonate (**10D**) (110.1 mg, yield 100%).

LC-MS, M/Z: 337.3[M+H-Li]⁺.

### Fourth step: 2-fluoro-3-(5-methylthiazol-2-yl)-5-(((S)-tetrahydrofuran-2-yl)methoxy)-N-((R)-1-(2-(trifluor omethyl)pyrimidin-5-yl)ethyl)benzamide (target compound I-10)

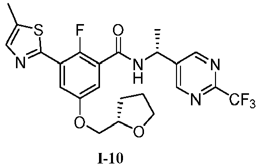

At room temperature, lithium (S)-2-fluoro-3-(5-methylthiazol-2-yl)-5-((tetrahydrofuran-2-yl)methoxy)benzonate (**10D**) (110.1 mg, 0.199 mmol) was added to 3 mL of N, N-dimethylformamide. Then, (R)-1-(2-(trifluoromethyl)pyrimidin-5-yl)ethyl-1-amine hydrochloride (45.3 mg, 0.199 mmol) and diisopropylethylamine (77 mg, 0.597mmol) were added, and propylphosphonic acid anhydride (190 mg, 0.299 mmol, 50% N, N-dimethylformamide solution) was added dropwise. After the dropwise addition the mixture was stirred and reacted overnight at room temperature under nitrogen protection, with TLC (PE: EA=2: 1) indicating reaction of the raw materials was complete. The mixture was diluted by adding 5 mL of water and extracted with ethyl acetate (10 mL×2). The organic phase was concentrated to dryness and the residue was separated and purified by silica gel column (petroleum ether: ethyl acetate (V/V) =2: 1) to obtain an off-white solid of 2-fluoro-3-(5-methylthiazol-2-yl)-5-(((S)-tetrahydrofuran-2-yl)methoxy)-N-((R)-1-(2-(trifluor omethyl)pyrimidin-5-yl)ethyl)benzamide (**I-10**) (16 mg, yield 15.8%).

¹H NMR (400 MHz, DMSO-d₆) δ 9.19 (d, 1H), 9.10 (s, 2H), 7.74-7.72 (m, 2H), 7.22-7.20 (m, 1H), 5.27-5.24 (m, 1H), 4.16-3.96 (m, 3H), 3.77-3.74 (m, 1H), 3.69-3.63 (m, 1H), 2.51-2.48 (m, 3H), 2.00-1.97 (m, 1H), 1.96-1.88 (m, 2H), 1.86-1.72 (m, 1H), 1.55 (d, 3H).

LC-MS, M/Z: 511.5[M+H]⁺.

### Example 11: preparation of target compound I-11

### 2-fluoro-3-(5-methylthiazol-2-yl)-5-(((R)-tetrahydrofuran-2-yl)methoxy)-N-((R)-1 -(2-(trifluoromethyl)pyrimidin-5-yl)ethyl)benzamide (target compound I-11)

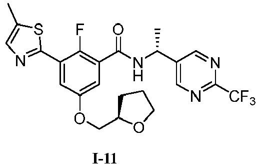

The synthesis scheme for target compound I-11 is shown below:

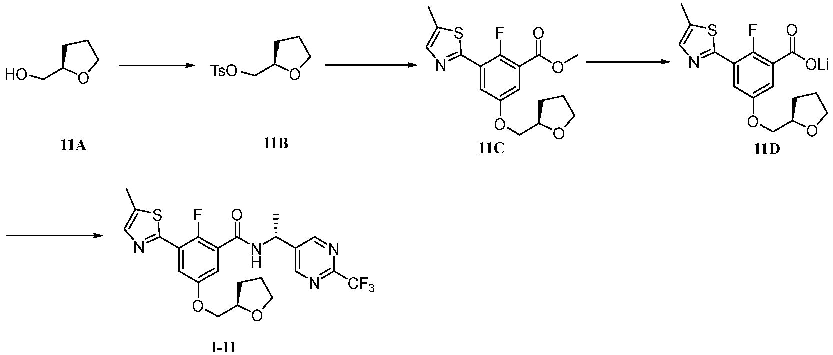

For the synthesis thereof, refer to Example 10.

¹H NMR (400 MHz, DMSO-d₆) δ 9.19 (d, 1H), 9.10 (s, 2H), 7.74-7.72 (m, 2H), 7.22-7.20 (m, 1H), 5.27-5.24 (m, 1H), 4.16-3.96 (m, 3H), 3.77-3.74 (m, 1H), 3.69-3.63 (m, 1H), 2.51-2.48 (m, 3H), 2.00-1.97 (m, 1H), 1.96-1.88 (m, 2H), 1.86-1.72 (m, 1H), 1.55 (d, 3H).

LC-MS, M/Z: 511.5[M+H]⁺.

### Example 12: preparation of target compound I-12

### 2-fluoro-3-(5-methylthiazol-2-yl)-5-(((R)-tetrahydrofuran-3-yl)methoxy)-N-((R)-1 -(2-(trifluoromethyl)pyrimidin-5-yl)ethyl)benzamide (target compound 1-12)

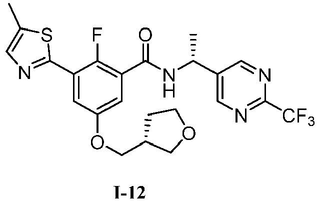

The synthesis scheme for target compound 1-12 is shown below:

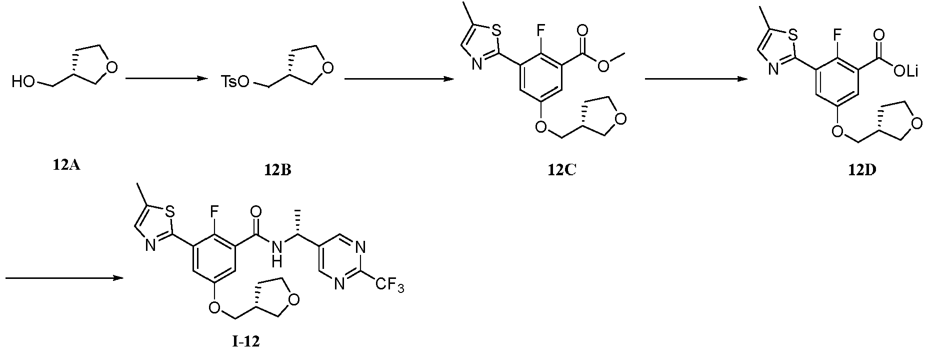

For the synthesis thereof, refer to Example 10.

¹H NMR (400 MHz, DMSO-d₆) δ 9.20-9.18 (m, 1H), 9.10 (s, 2H), 7.74-7.72 (m, 2H), 7.22-7.20 (m, 1H), 5.29-5.22 (m, 1H), 4.02-3.95 (m, 2H), 3.78-3.74 (m, 2H), 3.65-3.63 (m, 1H), 3.55-3.52 (m, 1H), 2.64-2.52 (m, 1H), 2.51-2.48 (m, 3H), 2.01-1.98 (m, 1H), 1.71-1.66 (m, 1H), 1.59 (d, 3H).

LC-MS, M/Z: 511.5 [M+H]⁺.

### Example 13: preparation of target compound 1-13

### 2-fluoro-3-(5-methylthiazol-2-yl)-5-(((S)-tetrahydrofuran-3-yl)methoxy)-N-((R)-1 -(2-(trifluoromethyl)pyrimidin-5-yl)ethyl)benzamide (target compound I-13)

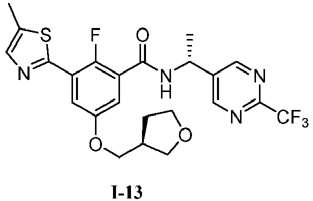

The synthesis scheme for target compound 1-13 is shown below:

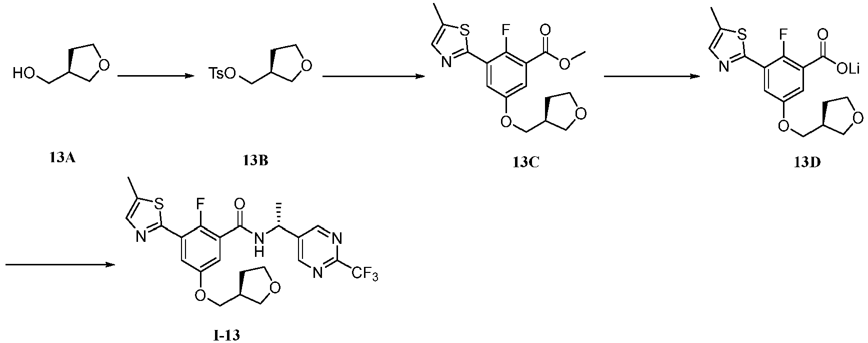

For the synthesis thereof, refer to Example 10.

¹H NMR (400 MHz, DMSO-d₆) δ 9.20-9.18 (m, 1H), 9.10 (s, 2H), 7.74-7.72 (m, 2H), 7.22-7.20 (m, 1H), 5.29-5.22 (m, 1H), 4.02-3.95 (m, 2H), 3.78-3.74 (m, 2H), 3.65-3.63 (m, 1H), 3.55-3.52 (m, 1H), 2.64-2.52 (m, 1H), 2.51-2.48 (m, 3H), 2.01-1.98 (m, 1H), 1.71-1.66 (m, 1H), 1.59 (d, 3H).

LC-MS, M/Z: 511.5[M+H]⁺.

### Example 14: preparation of target compound 1-14

### (R)-2-fluoro-3-(5-methylthiazol-2-yl)-5-(oxetane-3-yl oxy)-N-(1-(2-(trifluoromethyl)pyrimidin-5-yl)ethyl)benzamide (target compound I-14)

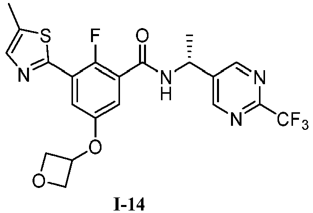

The synthesis scheme for target compound 1-14 is shown below:

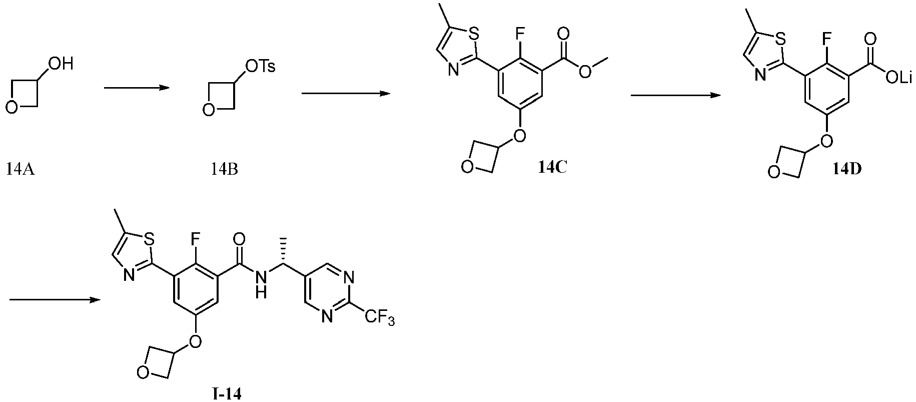

### First step: synthesis of oxetan-3-yl 4-methylbenzenesulfonate (14B)

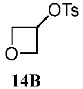

Oxetan-3-ol (**14A**) (740 mg, 10.0 mmol) was added to dichloromethane (20 mL), and triethylamine (2.01 g, 20 mmol) and 4-dimethylaminopyridine (244 mg, 2 mmol) were added. The mixture was cooled to 0°C, and after p-toluenesulfonyl chloride (2.10 g, 11.0 mmol) was added, the mixture was stirred overnight for reaction. Then, the mixture was diluted by adding dichloromethane (50 mL), followed by concentration with silica gel (50 mL). The residue was separated and purified by silica gel column (petroleum ether: ethyl acetate (V/V) =2: 1) to obtain compound oxetan-3-yl 4-methylbenzenesulfonate (**14B**)**,** an off-white solid (1.40 g, yield 61%).

### Second step: synthesis of methyl 2-fluoro-3-(5-methylthiazol-2-yl)-5-(oxetan-3-yloxy)benzoate (14C)

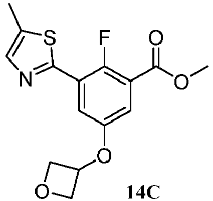

Compound methyl 2-fluoro-5-hydroxy-3-(5-methylthiazol-2-yl)benzoate (0.20 g, 0.749 mmol) (for synthesis thereof, refer to Example 1) was added to DMF (2 mL). After cesium carbonate (0.37 g, 1.124 mmol) was added, the mixture was heated to 50 °C and stirred for 0.5h. Then, oxetan-3-yl 4-methylbenzenesulfonate (**14B**) (0.26 g, 1.124 mmol) was added, and the mixture was heated to 90 °C and stirred overnight for reaction. Then, the mixture was cooled to room temperature and diluted by adding distilled water (10 mL), followed by extraction with ethyl acetate (10 mL×3). The organic phases were combined and washed with saturated salt water (10 mL×2). After liquid separation, the organic phase was dried over anhydrous sodium sulfate, followed by filtration and concentration. The residue was purified with silica gel column (petroleum ether: ethyl acetate (V/V) = 2: 1) to obtain an oily compound methyl 2-fluoro-3-(5-methylthiazol-2-yl)-5-(oxetan-3-yloxy)benzoate (**14C**) (80 mg, yield 33.1%).

LC-MS, M/Z: 324.3 [M+H]⁺.

### Third step: synthesis of lithium 2-fluoro-3-(5-methylthiazol-2-yl)-5-(oxetan-3-yloxy)benzonate (14D)

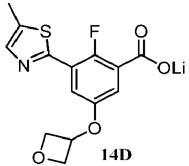

At room temperature, methyl 2-fluoro-3-(5-methylthiazol-2-yl)-5-(oxetan-3-yloxy)benzoate (**14C**) (80 mg, 0.248 mmol) was added to 0.5 mL of tetrahydrofuran solution, and then 0.2 mL of water and lithium hydroxide monohydrate (59.4 mg, 2.48 mmol) were added. The mixture reacted overnight at room temperature, and then concentrated to dryness. The residue was added with water (10 mL) for free-drying to obtain a crude product of lithium 2-fluoro-3-(5-methylthiazol-2-yl)-5-(oxetan-3-yloxy)benzonate (**14D**) (138.1 mg, yield 100%).

LC-MS, M/Z: 309.3 [M+H-Li]⁺.

### Fourth step: synthesis of (R)-2-fluoro-3-(5-methylthiazol-2-yl)-5-(oxetane-3-yl oxy)-N-(1-(2-(trifluoromethyl)pyrimidin-5-yl)ethyl)benzamide (target compound I-14)

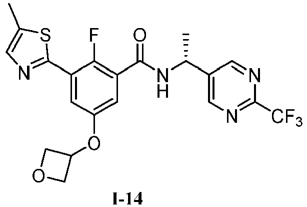

At room temperature, the crude product of lithium 2-fluoro-3-(5-methylthiazol-2-yl)-5-(oxetan-3-yloxy)benzonate (**14D**) (138.1 mg, 0.248 mmol) was added to 3 mL of N, N-dimethylformamide, and then (R)-1-(2-(trifluoromethyl)pyrimidin-5-yl)ethyl-1-amine hydrochloride (59.2 mg, 0.248 mmol) and diisopropylethylamine (96 mg, 0.744 mmol) were added. Propylphosphonic acid anhydride (237 mg, 0.372 mmol, 50% N, N-dimethylformamide solution) was then added dropwise. After the dropwise addition the mixture was stirred and reacted overnight at room temperature under nitrogen protection, with TLC (PE: EA=1: 1) indicating that the reaction of the raw materials was complete. The mixture was diluted by adding 5 mL of water and extracted with ethyl acetate (10 mL×2). The organic phase was concentrated to dryness the residue was separated and purified by silica gel column (petroleum ether: ethyl acetate (V/V) =2: 1) to obtain an off-white solid of (R)-2-fluoro-3-(5-methylthiazol-2-yl)-5-(oxetan-3-yloxy)-N-(1-(2-(trifluoromethyl)pyrimidin-5-yl)ethyl)benzamide (**I-14**) (27 mg, yield 22.5%).

¹H NMR (400 MHz, DMSO-d₆) δ 9.23 (d, 1H), 9.12 (s, 2H), 7.44 (d, 1H), 7.56 (q, 1H), 7.11 (q, 1H), 5.44-5.39 (m, 1H), 5.29-5.26 (m, 1H), 4.95-4.92 (m, 2H), 4.58-4.55 (m, 2H), 2.53-2.49 (s, 3H), 1.56 (s, 3H).

LC-MS, M/Z: 483.5[M+H]⁺.

### Example 15: preparation of target compound 1-15

### (R)-5-(cyclopropylmethoxy)-2-fluoro-3-(5-methylthiazol-2-yl)-N-(1-(2-(trifluoromethyl)pyri midin-5-yl)ethyl)benzamide (target compound I-15)

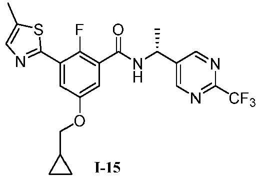

The synthesis scheme for target compound 1-15 is shown below:

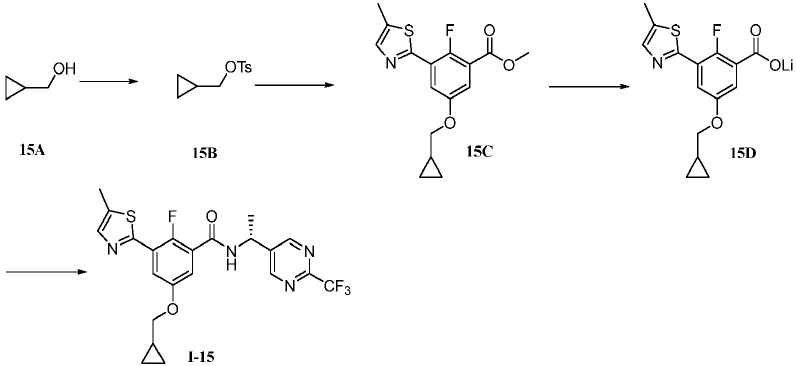

### First step: synthesis of cyclopropylmethyl 4-methylbenzenesulfonate (15B)

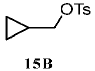

Cyclopropylmethanol (15**A**) (720 mg, 10.0 mmol) was added to dichloromethane (20 mL), and then triethylamine (2.01 g, 20 mmol) and 4-dimethylaminopyridine (244 mg, 2 mmol) were added. The mixture was cooled to 0°C, and after p-toluenesulfonyl chloride (2.10 g, 11.0 mmol) was added, the mixture was stirred overnight at room temperature for reaction. Then, the mixture was diluted by adding dichloromethane (50 mL), followed by concentration with silica gel (50 mL). The residue was separated and purified by silica gel column (petroleum ether: ethyl acetate (V/V) =2: 1) to obtain a compound of cyclopropylmethyl 4-methylbenzenesulfonate (**15B**), an off-white solid (1.65 g, yield 73%).

### Second step: synthesis of methyl 5-(cyclopropylmethoxy)-2-fluoro-3-(5-methylthiazol-2-yl)benzoate (15C)

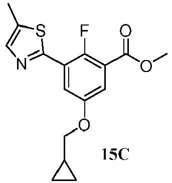

The compound of methyl 2-fluoro-5-hydroxy-3-(5-methylthiazol-2-yl)benzoate (0.20 g, 0.749 mmol) (for synthesis thereof, refer to Example 1) was added to DMF (2 mL), and after cesium carbonate (0.37 g, 1. 1 24 mmol) was added, the mixture was heated to 50 °C and stirred for 0.5h. Cyclopropylmethyl 4-methylbenzenesulfonate (**15B**) (0.25 g, 1.124 mmol) was added, and the mixture was heated to 90 °C, and stirred overnight for reaction. Thereafter, the mixture was cooled to room temperature and diluted by adding distilled water (10 mL), followed by extraction with ethyl acetate (10 mL×3). The organic phases were combined and washed with saturated salt water (10 mL×2). After liquid separation, the organic phase was dried over anhydrous sodium sulfate, followed by filtration and concentration. The residue was purified with silica gel column (petroleum ether: ethyl acetate (V/V) =2: 1) to obtain an oily compound methyl 5-(cyclopropylmethoxy)-2-fluoro-3-(5-methylthiazol-2-yl)benzoate (**15C**) (65 mg, yield 27.0%).

LC-MS, M/Z: 322.4 [M+H]⁺.

### Third step: synthesis of lithium 5-(cyclopropylmethoxy)-2-fluoro-3-(5-methylthiazol-2-yl)benzonate (15D)

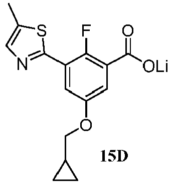

At room temperature, methyl 5-(cyclopropylmethoxy)-2-fluoro-3-(5-methylthiazol-2-yl)benzoate (**15C**) (65 mg, 0.202 mmol) was added to 0.5 mL of tetrahydrofuran solution, and then 0.2 mL of water and lithium hydroxide (48.5 mg, 2.02 mmol) were added. The mixture reacted overnight at room temperature and concentrated to dryness. The residue was added with water (10 mL) for free-drying to obtain lithium 5-(cyclopropylmethoxy)-2-fluoro-3-(5-methylthiazol-2-yl)benzonate (**15D**) (103.3 mg, yield 100%).

LC-MS, M/Z: 307.3 [M+H-Li]⁺.

### Fourth step: synthesis of (R)-5-(cyclopropylmethoxy)-2-fluoro-3-(5-methylthiazol-2-yl)-N-(1-(2-(trifluoromethyl)pyri midin-5-yl)ethyl)benzamide (target compound I-15)

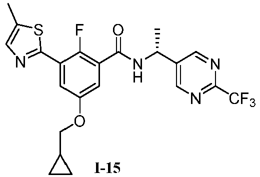

At room temperature, the lithium 5-(cyclopropylmethoxy)-2-fluoro-3-(5-methylthiazol-2-yl)benzonate crude product (**15D**) (103.3 mg, 0.202 mmol) was added to 3 mL of N, N-dimethylformamide. Then, (R)-1-(2-(trifluoromethyl)pyrimidin-5-yl)ethyl-1-amine hydrochloride (58.8 mg, 0.202 mmol) and diisopropylethylamine (78 mg, 0.606 mmol) were added, and propylphosphonic acid anhydride (192 mg, 0.303 mmol, 50% 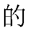 N, N-dimethylformamide solution) was added dropwise. After the dropwise addition, the mixture was stirred and reacted overnight at room temperature under nitrogen protection, with TLC (PE: EA=1: 1) indicating that the reaction of the raw materials was complete. Thereafter, the mixture was diluted by adding 5 mL of water, and extracted with ethyl acetate (10 mL×2). The organic phase was concentrated to dryness the residue was separated and purified by silica gel column (petroleum ether: ethyl acetate (V/V) =2: 1) to obtain an off-white solid of (R)-5-(cyclopropylmethoxy)-2-fluoro-3-(5-methylthiazol-2-yl)-N-(1-(2-(trifluoromethyl)pyri midin-5-yl)ethyl)benzamide (**I-15**) (19 mg, yield 19.6%).

¹H NMR (400 MHz, CDCl₃) δ 8.88 (s, 2H), 7.87 (d, 1H), 7.54 (s, 1H), 7.45 (d, 1H), 7.02-6.93 (m, 1H), 5.33-5.30 (m, 1H), 3.80 (d, 2H), 2.50 (s, 3H), 1.65 (d, 3H), 1.12-1.06 (m, 1H), 0.60-0.55 (m, 2H), 0.29-026 (m, 2H).

LC-MS, M/Z: 481.5 [M+H]⁺.

### Example 16: preparation of target compound 1-16

### (R)-2-fluoro-5-((1-methylpiperidin-4-yl)oxy)-3-(5-methylthiazol-2-yl)-N-(1-(2-(trifluorometh yl)pyrimidin-5-yl)ethyl)benzamide (target compound I-16)

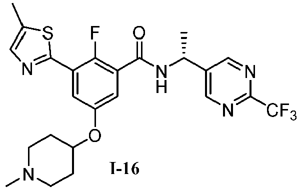

The synthesis scheme for target compound 1-16 is shown below:

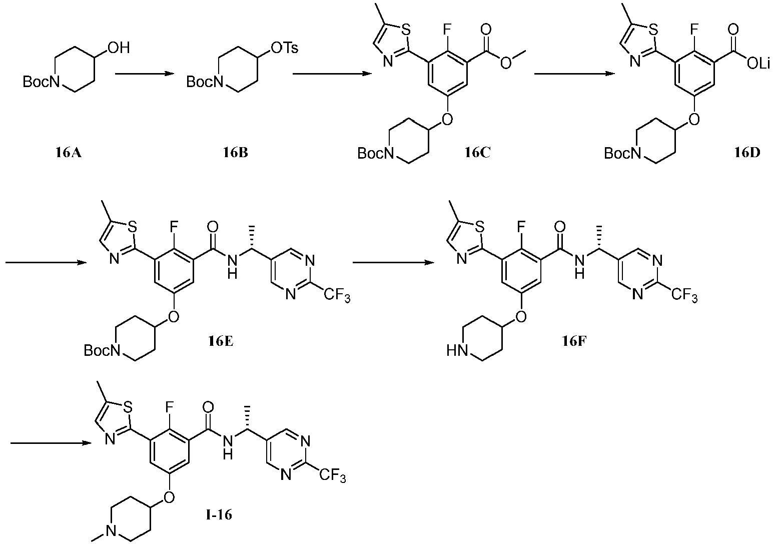

### First step: synthesis of tert-butyl 4-(4-methylphenyl)sulfonylpiperidine-1-carboxylate (16B)

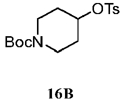

N-Boc-4-hydroxypiperidine (**16A**) (2.0 g, 9.94 mmol) was added to dichloromethane (20 mL), and then triethylamine (2.01 g, 19.87 mmol) and 4-dimethylaminopyridine (12 mg, 0.1 mmol) were added. The mixture was cooled to 0°C, and after p-toluenesulfonyl chloride (2.84 g, 14.91 mmol) was added, the mixture was stirred overnight at room temperature for reaction. Thereafter, the mixture was diluted by adding ethyl acetate (50 mL) and concentrated. The residue was separated and purified by silica gel column (petroleum ether: ethyl acetate (V/V) =5: 1) to obtain a white solid of tert-butyl 4-(4-methylphenyl)sulfonylpiperidine-1-carboxylate (**16B**) (2.3 g, yield 65.1%).

### Second step: synthesis of methyl 2-fluoro-5-((1-(tert-butoxycarbonyl)piperidin-4-yl)oxy)-3-(5-methylthiazol-2-yl)benzoate (16C)

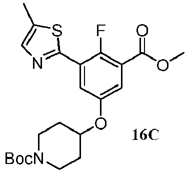

Compound methyl 2-fluoro-5-hydroxy-3-(5-methylthiazol-2-yl)benzoate (0.50 g, 1.871 mmol) (for synthesis thereof, refer to Example 1) was added to DMF (25 mL). Then, cesium carbonate (1.83 g, 5.61 mmol) and tert-butyl 4-(4-methylphenyl)sulfonylpiperidine-1-carboxylate (**16B**) (0.67 g, 1.871 mmol) were added. The mixture was heated to 80 °C, and stirred overnight for reaction, then cooled to room temperature, diluted by adding distilled water (50 mL), and extracted with ethyl acetate (20 mL×3). The organic phases were combined and washed with saturated salt water (10 mL×2). After liquid separation, the organic phase was dried over anhydrous sodium sulfate, filtrated, and concentrated. The residue was separated and purified by silica gel column (petroleum ether: ethyl acetate (V/V) =1: 1) to obtain a white solid of methyl 2-fluoro-5-((1-(tert-butoxycarbonyl)piperidin-4-yl)oxy)-3-(5-methylthiazol-2-yl)benzoate (**16C**) (0.4 g, yield 39.6 %).

LC-MS, M/Z: 451.5 [M+H]⁺.

### Third step: synthesis of lithium 2-fluoro-5-((1-(tert-butoxycarbonyl)piperidin-4-yl)oxy)-3-(5-methylthiazol-2-yl)benzonate (16D)

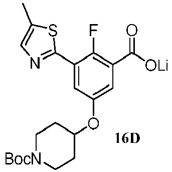

At room temperature, methyl 2-fluoro-5-((1-(tert-butoxycarbonyl)piperidin-4-yl)oxy)-3-(5-methylthiazol-2-yl)benzoate (**16C**) (0.4g, 0.888 mmol) was added to 10 mL of tetrahydrofuran solution. Then, 2.5 mL of water, 2.5 mL of methanol, and lithium hydroxide (0.11 g, 4.44 mmol) were added, and the mixture reacted overnight at room temperature and then concentrated to dryness. The residue was added with water (10 mL) for free-drying to obtain lithium 2-fluoro-5-((1-(tert-butoxycarbonyl)piperidin-4-yl)oxy)-3-(5-methylthiazol-2-yl)benzonate (**16D**) (0.38 g, yield 98%).

LC-MS, M/Z: 436.5[M+H-Li]⁺.

### Fourth step: synthesis of (R)-2-fluoro-5-((1-(tert-butoxycarbonyl)piperidin-4-yl)oxy)-3-(5-methylthiazol-2-yl)-N-(1-(2-(trifluoromethyl)pyrimidin-5-yl)ethyl)benzamide (16E)

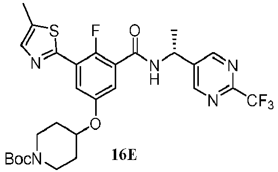

At room temperature, lithium 2-fluoro-5-((1-(tert-butoxycarbonyl)piperidin-4-yl)oxy)-3-(5-methylthiazol-2-yl)benzonate (**16D**) (0.38 g, 0.871 mmol) was added to 10 mL of N, N-dimethylformamide. Then, (R)-1-(2-(trifluoromethyl)pyrimidin-5-yl)ethyl-1-amine hydrochloride (0.20 g, 1.045 mmol) and diisopropylethylamine (0.34 g, 2.61 mmol) were added, and propylphosphonic acid anhydride (0.83 g, 2.61 mmol, 50% N, N-dimethylformamide solution) was added dropwise. After the dropwise addition, the mixture was stirred and reacted overnight at room temperature under nitrogen protection. Thereafter, the mixture was diluted by adding 10 mL of water and extracted with ethyl acetate (10 mL×2). The organic phase was concentrated to dryness. The residue was separated by a silica gel column (dichloromethane: methanol (V/V) =10: 1) to obtain a white solid of (R)-2-fluoro-5-((1-(tert-butoxycarbonyl)piperidin-4-yl)oxy)-3-(5-methylthiazol-2-yl)-N-(1-(2-(trifluoromethyl)pyrimidin-5-yl)ethyl)benzamide (**16E**) (160 mg, yield 30.1%).

LC-MS, M/Z: 610.6 [M+H]⁺.

### Fifth step: synthesis of (R)-2-fluoro-5-((1-piperidin-4-yl)oxy)-3-(5-methylthiazol-2-yl)-N-(1-(2-(trifluoromethyl)pyri midin-5-yl)ethyl)benzamide (16F)

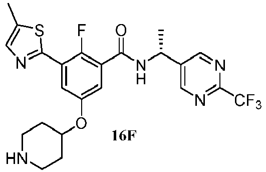

At room temperature, (R)-2-fluoro-5-((1-(tert-butoxycarbonyl)piperidin-4-yl)oxy)-3-(5-methylthiazol-2-yl)-N-(1-(2-(trifluoromethyl)pyrimidin-5-yl)ethyl)benzamide (**16E**) (160 mg, 0.262 mmol) was added to 2 mL of methanol, and a 4M dioxane hydrochloride solution (2.0 g, 8.0 mmol) was added. Thereafter, the mixture was stirred and reacted overnight at room temperature under nitrogen protection, followed by concentration and freeze-drying to obtain a white solid of (R)-2-fluoro-5-((1-piperidin-4-yl)oxy)-3-(5-methylthiazol-2-yl)-N-(1-(2-(trifluoromethyl)pyri midin-5-yl)ethyl)benzamide (**16F**) (50 mg, yield 37.4%).

LC-MS, M/Z: 510.5 [M+H]⁺.

### Sixth step: synthesis of (R)-2-fluoro-5-((1-methylpiperidin-4-yl)oxy)-3-(5-methylthiazol-2-yl)-N-(1-(2-(trifluorometh yl)pyrimidin-5-yl)ethyl)benzamide (target compound I-16)

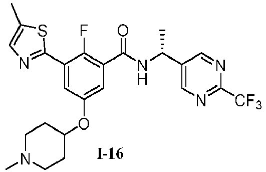

At room temperature, (R)-2-fluoro-5-((1-piperidin-4-yl)oxy)-3-(5-methylthiazol-2-yl)-N-(1-(2-(trifluoromethyl)pyri midin-5-yl)ethyl)benzamide (**16F**) (50 mg, 0.098 mmol) was added to 2 mL of methanol. Then, polyformaldehyde (15 mg, 0.491 mmol), sodium cyanoborohydride (31 mg, 0.491 mmol), and glacial acetic acid (0.59 mg, 0.01 mmol) were added. Thereafter, the mixture was stirred and reacted overnight at room temperature under nitrogen protection. The reaction solution was concentrated and then diluted with a saturated solution of sodium bicarbonate (10 mL), followed by extraction with dichloromethane (5 mL×3). The organic phase was concentrated to dryness. The residue was separated with a large silica gel plate (dichloromethane: methanol (V/V) =10: 1) to obtain a white solid of (R)-2-fluoro-5-((1-methylpiperidin-4-yl)oxy)-3-(5-methylthiazol-2-yl)-N-(1-(2-(trifluorometh yl)pyrimidin-5-yl)ethyl)benzamide (**I-16**) (30 mg, yield 58.0%).

¹H NMR (400 MHz, DMSO-d₆) δ 9.27 (d, 1H), 9.11 (s, 2H), 7.74 (m, 2H), 7.25 (s, 1H), 5.25 (s, 1H), 4.59 (s, 1H), 2.87 (s, 2H), 2.51 (s, 3H), 2.48 (s, 3H), 2.39 (s, 2H), 2.01 (s, 2H), 1.79 (s, 2H), 1.55 (d, 3H).

LC-MS, M/Z: 524.6 [M+H]⁺.

**Example 17:** preparation of target compound 1-17

### Synthesis of (R)-2-fluoro-5-((1-piperidin-4-yl)oxy)-3-(5-methylthiazol-2-yl)-N-(1-(2-(trifluoromethyl)pyri midin-5-yl)ethyl)benzamide (I-17)

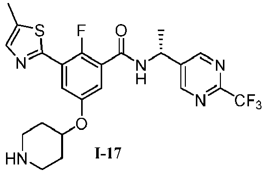

The synthesis scheme for target compound **I-17** is shown below:

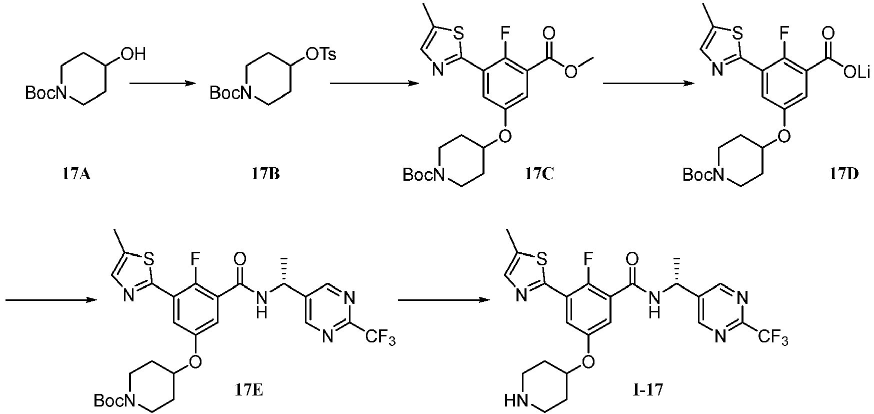

For the synthesis of target compound **I-17** (56 mg, yield 39.8 %), refer to the synthesis steps of compound 16F in Example 16.

¹H NMR (400 MHz, DMSO-d₆) δ 9.25 (s, 1H), 9.11 (s, 2H), 8.91 (s, 2H), 7.79 (s, 1H), 7.72 (s, 1H), 7.29 (s, 1H), 5.26 (s, 1H), 4.76 (s, 1H), 3.22 (s, 2H), 3.06 (s, 2H), 2.51 (s, 3H), 2.07 (s, 2H), 1.84 (s, 2H), 1.55 (d, 3H).

LC-MS, M/Z: 510.5 [M+H]⁺.

### Example 18: preparation of target compound I-18

### 2-fluoro-3-(5-methylthiazol-2-yl)-5-(((R)-morpholin-2-yl)methoxy)-N-((R)-1-(2-(t rifluoromethyl)pyrimidin-5-yl)ethyl)benzamide hydrochloride (I-18)

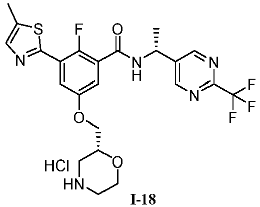

The synthesis scheme for target compound 1-18 is shown below:

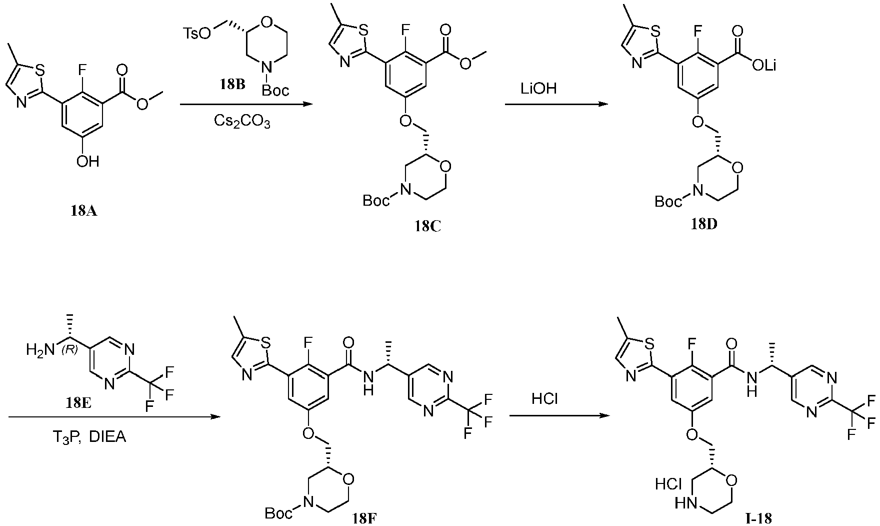

### First step: synthesis of tert-butyl (R)-2-((4-fluoro-3-(methoxycarbonyl)-5-(5-methylthiazol-2-yl)phenoxy)methyl)morpholine-4 -carboxylate (18C)

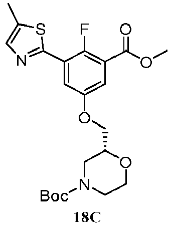

Methyl 2-fluoro-5-hydroxy-3-(5-methylthiazol-2-yl)benzoate (**18A**) (500 mg, 1.871 mmol) was dissolved in dried N, N-dimethylformamide (10 mL), and cesium carbonate (914 mg, 2.81 mmol) was added. The mixture was stirred and reacted at room temperature for 10 min. Then, tert-butyl (R)-2-((toluenesulfonyloxy)methyl)morpholine-4-carboxylate (1042 mg, 2.81 mmol) was added. After three times of nitrogen replacement, the mixture was heated to 90 °C and reacted under nitrogen protection for 4 h. Thereafter, the reaction solution was added with water (50 mL) and extracted with ethyl acetate (60 mL×3). The organic phases were combined and dried over anhydrous sodium sulfate, and concentrated to obtain a crude product of tert-butyl (R)-2-((4-fluoro-3-(methoxycarbonyl)-5-(5-methylthiazol-2-yl)phenoxy)methyl)morpholine-4 -carboxylate (**18C**) (873 mg, yield 100%).

LC-MS, M/Z: 467.4 [M+H]⁺.

### Second step: synthesis of lithium (R)-5-((4-(tert-butoxycarbonyl)morpholin-2-yl)methoxy)-2-fluoro-3-(5-methylthiazol-2-yl)be nzonate (18D)

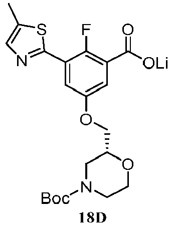

Lithium hydroxide monohydrate (314 mg, 7.49 mmol) and water (5 mL) were sequentially added into a solution of tert-butyl (R)-2-((4-fluoro-3-(methoxycarbonyl)-5-(5-methylthiazol-2-yl)phenoxy)methyl)morpholine-4 -carboxylate (**18C**) (873 mg, 1.871 mmol) (i.e., the crude product in previous step) in methanol (10 mL) and tetrahydrofuran (10 mL). The mixture reacted at room temperature for 24 h. The reaction solution was freeze-dried and the crude product directly used for the next reaction.

LC-MS, M/Z: 453.3 [M+2H-Li]⁺.

### Third step: synthesis of tert-butyl (R)-2-((4-fluoro-3-(5-methylthiazol-2-yl)-5-(((R)-1-(2-(trifluoromethyl)pyrimidin-5-yl)ethyl) carbamoyl)phenoxy)methyl)morpholine-4-carboxylate (18F)

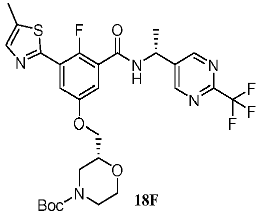

The crude product obtained in the previous step, i.e., lithium (R)-5-((4-(tert-butoxycarbonyl)morpholin-2-yl)methoxy)-2-fluoro-3-(5-methylthiazol-2-yl)be nzonate (**18D**) (847 mg, 1.872 mmol), was dissolved in N, N-dimethylformamide (10 mL). (R)-1-(2-(trifluoromethyl)pyrimidin-5-yl)ethyl-1-amine hydrochloride (469 mg, 2.059 mmol) and N, N-diisopropylethylamine (726 mg, 5.62 mmol) were successively added into the reaction flask, and the reaction was cooled to about 0°C and added with propylphosphonic acid anhydride (3.573 g, 5.62 mmol, 50% N, N-dimethylformamide solution) dropwise. After the dropwise addition, the mixture was heated to room temperature and reacted for 20h. The reaction was quenched by adding water (50 mL), followed by extraction with ethyl acetate (50 mL × 3). The organic phases were combined, dried over anhydrous sodium sulfate, and then concentrated. The residue was separated and purified with silica gel column (petroleum ether: ethyl acetate (V/V) =2: 1) to obtain tert-butyl (R)-2-((4-fluoro-3-(5-methylthiazol-2-yl)-5-(((R)-1-(2-(trifluoromethyl)pyrimidin-5-yl)ethyl) carbamoyl)phenoxy)methyl)morpholine-4-carboxylate (**18F**), a white solid (530 mg, yield 45.3%).

LC-MS, M/Z: 626.2 [M+H]⁺.

### Fourth step: synthesis of 2-fluoro-3-(5-methylthiazol-2-yl)-5-(((R)-morpholin-2-yl)methoxy)-N-((R)-1-(2-(trifluoromet hyl)pyrimidin-5-yl)ethyl)benzamide hydrochloride (I-18)

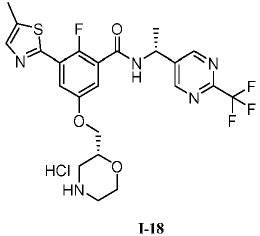

Tert-butyl (R)-2-((4-fluoro-3-(5-methylthiazol-2-yl)-5-(((R)-1-(2-(trifluoromethyl)pyrimidin-5-yl)ethyl) carbamoyl)phenoxy)methyl)morpholine-4-carboxylate (**18F**) (450 mg, 0.719 mmol) was dissolved in methanol (2 mL), and added with a dioxane solution of hydrogen chloride (4 M, 2mL, 8 mmol). The mixture was stirred and reacted at room temperature for 18 h. The reaction solution was concentrated to obtain 2-fluoro-3-(5-methylthiazol-2-yl)-5-(((R)-morpholin-2-yl)methoxy)-N-((R)-1-(2-(trifluoromet hyl)pyrimidin-5-yl)ethyl)benzamide hydrochloride (**I-18**), a yellow solid (400 mg, yield 99%).

¹H NMR (400 MHz, DMSO-d₆): δ 9.44-9.32 (m, 2H), 9.25-9.23 (d, 1H), 9.10 (s, 2H), 7.76-7.71 (m, 2H), 7.24-7.22 (m, 1H), 5.29-5.22 (m, 1H), 4.18-3.96 (m, 5H), 3.81-3.75 (m, 1H), 3.20-3.16 (d, 1H), 2.98-2.92 (m, 2H), 2.51 (s, 3H), 1.55-1.53 (d, 3H).

LC-MS, M/Z: 526.4 [M+H]⁺.

### Example 19: preparation of compound I-19

### 2-fluoro-5-(((R)-4-methylmorpholin-2-yl)methoxy)-3-(5-methylthiazol-2-yl)-N-(( R)-1-(2-(trifluoromethyl)pyrimidin-5-yl)ethyl)benzamide (I-19)

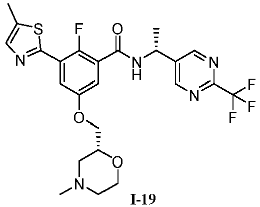

The synthesis scheme for target compound 1-19 is shown below:

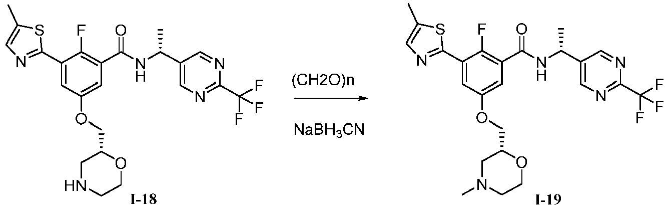

### Synthesis of 2-fluoro-5-(((R)-4-methylmorpholin-2-yl)methoxy)-3-(5-methylthiazol-2-yl)-N-((R)-1-(2-(trif luoromethyl)pyrimidin-5-yl)ethyl)benzamide (I-19)

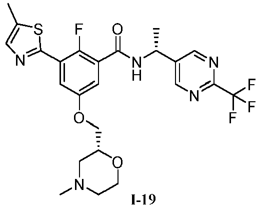

2-fluoro-3-(5-methylthiazol-2-yl)-5-(((R)-morpholin-2-yl)methoxy)-N-((R)-1-(2-(t rifluoromethyl)pyrimidin-5-yl)ethyl)benzamide hydrochloride (for the synthesis thereof, refer to Example 18) (1.26 g, 2.398 mmol) was dissolved into dried methanol (10 mL) in a sealed tube. Polyformaldehyde (1.079 g, 11.99 mmol) and acetic acid (0.144 g, 2.398 mmol) were successively added. The mixture was stirred at room temperature for 10 min and then added with sodium cyanoborohydride (0.753 g, 11.99 mmol), and further reacted at room temperature for 20 h. The reaction was quenched by adding saturated sodium bicarbonate (50 mL) into the reaction solution, followed by extraction with dichloromethane (30 mL×3). The organic phases were combined, dried over anhydrous sodium sulfate, and concentrated. The residue was purified with a silica gel plate (dichloromethane: methanol (V/V) = 50: 1) to obtain 2-fluoro-5-(((R)-4-methylmorpholin-2-yl)methoxy)-3-(5-methylthiazol-2-yl)-N-((R)-1-(2-(trif luoromethyl)pyrimidin-5-yl)ethyl)benzamide (**I-19**), a white solid (598.5 mg, yield 46.3%)

¹H NMR (400 MHz, DMSO-d₆) δ 9.21-9.19 (d, 1H), 9.11 (s, 2H), 7.74-7.72 (m, 2H), 7.23-7.21 (m, 1H), 5.27-5.24 (m, 1H), 4.06-4.05 (d, 2H), 3.82-3.79 (m, 2H), 3.55 (t, 1H), 2.84-2.81 (m, 1H), 2.67-2.64 (m, 1H), 2.51 (s, 3H), 2.23 (s, 3H), 2.08-1.99 (m, 2H), 1.56-1.54 (d, 3H).

LC-MS, M/Z: 540.4 [M+H]⁺.

### Example 20: preparation of compound I-20

### 2-fluoro-5-(((R)-4-(methyl-d3)morpholin-2-yl)methoxy)-3-(5-methylthiazol-2-yl)-N-((R)-1-(2-(trifluoromethyl)pyrimidin-5-yl)ethyl)benzamide (I-20)

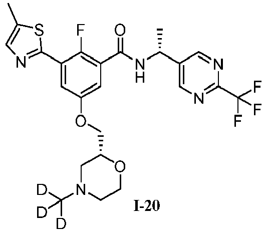

The synthesis scheme for target compound I-20 is shown below:

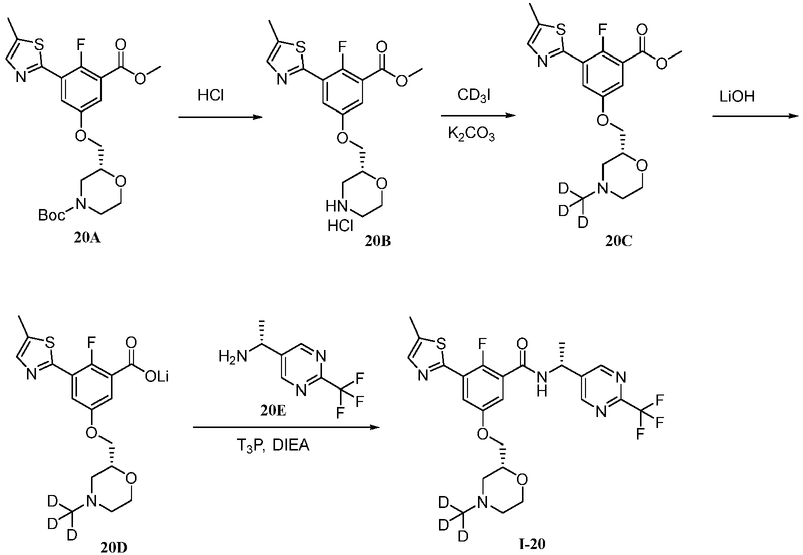

### First step: synthesis of methyl (R)-2-fluoro-3-(5-methylthiazol-2-yl)-5-(morpholin-2-yl-methoxy)benzoate hydrochloride (20B)

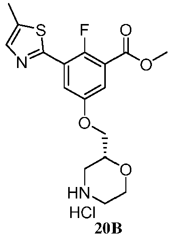

1, 4-dioxane solution of 4M hydrogen chloride (5 mL, 20 mmol) was added into tert-butyl (R)-2-((4-fluoro-3-(methoxycarbonyl)-5-(5-methylthiazol-2-yl)phenoxy)methyl)morpholine-4 -carboxylate (20A) (0.5 g, 1.072 mmol). The mixture was stirred and reacted at room temperature for 30 min. Then, the reaction solution was concentrated, and the crude product was directly fed to the next step of reaction.

### Second step: synthesis of methyl (R)-2-fluoro-5-((4-(methyl-d3)morpholin-2-yl)methoxy)-3-(5-methylthiazol-2-yl)benzoate (20C)

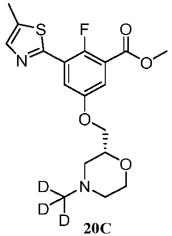

The crude product in the previous step, i.e., methyl (R)-2-fluoro-3-(5-methylthiazol-2-yl)-5-(morpholin-2-yl-methoxy)benzoate hydrochloride (**20B**), was dissolved in dried acetonitrile (10 mL), and then triethylamine (0.325 g, 3.22 mmol), potassium carbonate (0.222 g, 1.608 mmol), and deuterated iodomethane (0.155 g, 1.072 mmol) were successively added. Thereafter, the mixture reacted at room temperature for 20 h. Then, the reaction solution was added with water (50 mL), and extracted with dichloromethane (30 mL×3). The organic phases were combined, dried over anhydrous sodium sulfate, and concentrated. The residue was separated through column chromatography (dichloromethane: methanol (V/V) =100: 1) to obtain a light-yellow oily compound methyl (R)-2-fluoro-5-((4-(methyl-d3)morpholin-2-yl)methoxy)-3-(5-methylthiazol-2-yl)benzoate (**20C**) (300 mg, yield : 73.0%)

LC-MS, M/Z: 384.2 [M+H]⁺.

### Third step: synthesis of lithium (R)-2-fluoro-5-((4-(methyl-d3)morpholin-2-yl)methoxy)-3-(5-methylthiazol-2-yl)benzonate (20D)

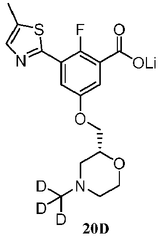

Methyl (R)-2-fluoro-5-((4-(methyl-d3)morpholin-2-yl)methoxy)-3-(5-methylthiazol-2-yl)benzoate (**20C**) (300 mg, 0.782 mmol) was dissolved in methanol (5 mL) and tetrahydrofuran (5 mL). The, lithium hydroxide monohydrate (131 mg, 3.13 mmol) and water (2 mL) were added, and the mixture reacted at room temperature for 4 h. The reaction solution was freeze-dried, and the crude product was directly fed to the next step of reaction.

LC-MS, M/Z: 370.2 [M+2H-Li]⁺.

### Fourth step: synthesis of 2-fluoro-5-(((R)-4-(methyl-d3)morpholin-2-yl)methoxy)-3-(5-methylthiazol-2-yl)-N-((R)-1-( 2-(trifluoromethyl)pyrimidin-5-yl)ethyl)benzamide (I-20)

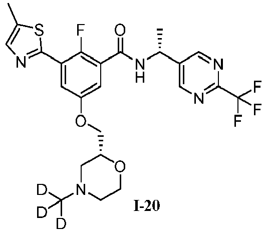

The crude product from the previous step of reaction, i.e., lithium (R)-2-fluoro-5-((4-(methyl-d3)morpholin-2-yl)methoxy)-3-(5-methylthiazol-2-yl)benzonate (20D) (289 mg, 0.782 mmol) was dissolved in dried N, N-dimethylformamide (5 mL). (R)-1-(2-(trifluoromethyl)pyrimidin-5-yl)ethyl-1-amine hydrochloride (196 mg, 0.861 mmol) and N, N-diisopropylethylamine (303 mg, 2.347 mmol) were successively added. The mixture was cooled to 0°C to 5°C, and propylphosphonic acid anhydride (1.493 g, 2.347 mmol, 50% N, N-dimethylformamide solution) was added dropwise. The mixture reacted at room temperature for 4 h. The reaction was quenched by adding water (50 mL) into the reaction solution, followed by extraction with dichloromethane (30 mL×3). The organic phases were combined, dried over anhydrous sodium sulfate and concentrated. The residue was purified with a silica gel plate (dichloromethane: methanol (V/V) =20: 1, +NH3) to obtain 2-fluoro-5-(((R)-4-(methyl-d3)morpholin-2-yl)methoxy)-3-(5-methylthiazol-2-yl)-N-((R)-1-( 2-(trifluoromethyl)pyrimidin-5-yl)ethyl)benzamide (**I-20**), a white solid (25 mg, yield 5.9%)

¹H NMR (400 MHz, DMSO-d₆): δ 9.19-9.17 (d, 1H), 9.09 (s, 2H), 7.72-7.70 (m, 2H), 7.22-7.20 (m, 1H), 5.28-5.21 (m, 1H), 4.04-4.03 (d, 2H), 3.79-3.75 (m, 2H), 3.54-3.48 (m, 1H), 2.76-2.74 (d, 1H), 2.59-2.56 (d, 1H), 2.50 (s, 3H), 2.02-1.89 (m, 2H), 1.54-1.52 (d, 3H).

LC-MS, M/Z: 543.4 [M+H]⁺.

### Example 21: preparation of compound I-21

### (R)-2-fluoro-3-(5-methylthiazol-2-yl)-5-((tetrahydro-2H-pyran-4-yl)methoxy)-N-( 1-(2-(trifluoromethyl)pyrimidin-5-yl)ethyl)benzamide (I-21)

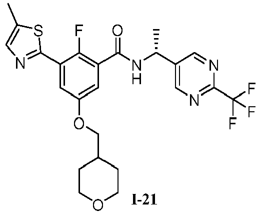

The synthesis scheme for target compound I-21 is shown below:

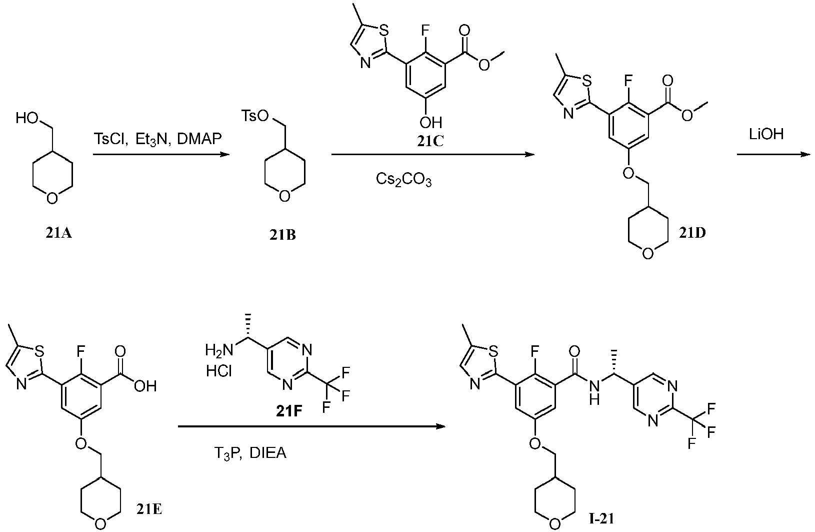

### Methyl 2-fluoro-5-hydroxy-3-(5-methylthiazol-2-yl)benzoate (21C)

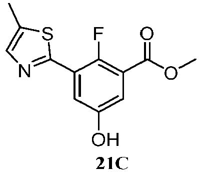

### (R)-1-(2-(trifluoromethyl)pyrimidin-5-yl)ethyl-1-amine hydrochloride (21F)

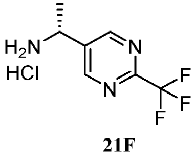

For the preparation thereof, refers to the method disclosed in Patent WO 2016/091776.

### First step: synthesis of (tetrahydro-2H-pyran-4-yl)methyl 4-methylbenzenesulfonate (21B)

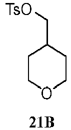

(Tetrahydro-2H-pyran-4-yl)methanol (**21A**) (2 g, 17.22 mmol), triethylamine (3.48 g, 34.4 mmol), and 4-dimethylaminopyridine (0.421 g, 3.44 mmol) were dissolved in dichloromethane (20 mL), and the mixture was stirred at room temperature, p-toluenesulfonyl chloride (3.94 g, 20.66 mmol) was added in batches, and then the mixture further reacted at room temperature for 20 h. The reaction was quenched by adding water (30 mL), followed by liquid separation. The aqueous phase was extracted with dichloromethane (30 mL×2). The organic phases were combined, dried over anhydrous sodium sulfate, and concentrated. The residue was purified with silica gel column (petroleum ether: ethyl acetate (V/V) =4: 1) to obtain (tetrahydro-2H-pyran-4-yl)methyl 4-methylbenzenesulfonate (**21B**), a white solid (4.5 g, yield 97%)

### Second step: synthesis of methyl 2-fluoro-3-(5-methylthiazol-2-yl)-5-((tetrahydro-2H-pyran-4-yl)methoxy)benzoate (21D)

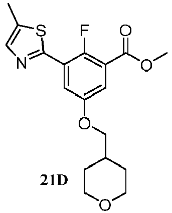

Cesium carbonate (366 mg, 1.122 mmol) was added into a solution of methyl 2-fluoro-5-hydroxy-3-(5-methylthiazol-2-yl)benzoate (**21C**) (200 mg, 0.748 mmol) in N, N-dimethylformamide (5 mL). The mixture was stirred at room temperature for 30 min. Then, (tetrahydro-2H-pyran-4-yl)methyl 4-methylbenzenesulfonate (**21B**) (303 mg, 1.122 mmol) was added, and under nitrogen protection, the mixture was heated to 100 °C and reacted for 3 h. Thereafter, the mixture was cooled to room temperature, and the reaction was quenched by adding water (50 mL), followed by extraction with ethyl acetate (30 mL×3). The organic phases were combined, dried over anhydrous sodium sulfate, and concentrated. The residue was purified with a silica gel column (petroleum ether: ethyl acetate (V/V) =2: 1) to obtain a white solid of methyl 2-fluoro-3-(5-methylthiazol-2-yl)-5-((tetrahydro-2H-pyran-4-yl)methoxy)benzoate (**21D**), a white solid (90 mg, yield 32.9%)

LC-MS, M/Z: 366.2 [M+H]⁺.

### Third step: synthesis of 2-fluoro-3-(5-methylthiazol-2-yl)-5-((tetrahydro-2H-pyran-4-yl)methoxy)benzoic acid (21E)

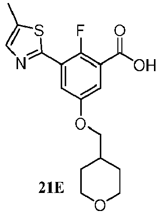

Methyl 2-fluoro-3-(5-methylthiazol-2-yl)-5-((tetrahydro-2H-pyran-4-yl)methoxy)benzoate (**21D**) (90 mg, 0.246 mmol) was dissolved in methanol (2 mL) and tetrahydrofuran (2 mL), and then lithium hydroxide monohydrate (41.3 mg, 0.985 mmol) and water (2 mL) were added. The mixture was stirred and reacted at room temperature for 20 h. Thereafter, methanol and tetrahydrofuran were removed by rotary evaporation under reduced pressure. The aqueous phase was adjusted to pH of about 4 using 2M hydrochloric acid, and then extracted with dichloromethane (10 mL×2). The organic phases were dried over anhydrous sodium sulfate, filtrated, and concentrated to obtain 2-fluoro-3-(5-methylthiazol-2-yl)-5-((tetrahydro-2H-pyran-4-yl)methoxy)benzoic acid (**21E**) (87 mg, crude product ), which was directly fed to the next step of reaction.

LC-MS, M/Z: 352.3 [M+H]⁺.

### Fourth step: synthesis of (R)-2-fluoro-3-(5-methylthiazol-2-yl)-5-((tetrahydro-2H-pyran-4-yl)methoxy)-N-(1-(2-(triflu oromethyl)pyrimidin-5-yl)ethyl)benzamide (I-21)

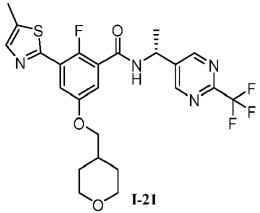

2-fluoro-3-(5-methylthiazol-2-yl)-5-((tetrahydro-2H-pyran-4-yl)methoxy)benzoic acid (**21E**) (87 mg, 0.248 mmol), (R)-1-(2-(trifluoromethyl)pyrimidin-5-yl)ethyl-1-amine hydrochloride (**21F**) (67.6 mg, 0.297 mmol), N, N-diisopropylethylamine (96 mg, 0.743 mmol), and N, N-dimethylformamide (5 mL) were successively added into a reaction flask. The mixture was cooled to about 0°C, and added with propylphosphonic acid anhydride (473 mg, 0.743 mmol, 50% N, N-dimethylformamide solution) dropwise. Thereafter, the mixture was heated to room temperature and reacted for 2 h. The reaction was quenched by adding water (50 mL), followed by extraction with ethyl acetate (30 mL × 3). The organic phases were combined, dried over anhydrous sodium sulfate, and then concentrated. The residue was separated and purified with a silica gel plate (petroleum ether: ethyl acetate (V/V) =1: 1) to obtain (R)-2-fluoro-3-(5-methylthiazol-2-yl)-5-((tetrahydro-2H-pyran-4-yl)methoxy)-N-(1-(2-(triflu oromethyl)pyrimidin-5-yl)ethyl)benzamide (**I-21**), a white solid (59.2 mg, yield 45.6%).

¹H NMR (400 MHz, DMSO-d₆) δ 9.20-9.18 (d, 1H), 9.10 (s, 2H), 7.74-7.71 (m, 2H), 7.21-7.19 (dd, 1H), 5.29-5.22 (m, 1H), 3.91-3.84 (m, 4H), 3.35-3.31 (m, 2H), 2.52-2.51 (d, 3H), 2.05-1.94 (m, 1H), 1.69-1.65 (dd, 2H), 1.56-1.54 (d, 3H), 1.39-1.28 (m, 2H).

LC-MS, M/Z: 525.2 [M+H]⁺.

### Example 22: preparation of compound I-22

### 3-(5-ethylthiazol-2-yl)-2-fluoro-5-(((R)-4-methylmorpholin-2-yl)methoxy)-N-((R) -1-(2-(trifluoromethyl)pyrimidin-5-yl)ethyl)benzamide (target compound I-22)

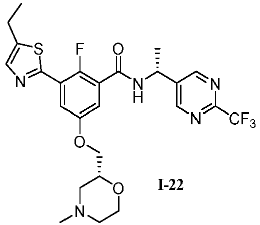

The synthesis scheme for target compound I-22 is shown below:

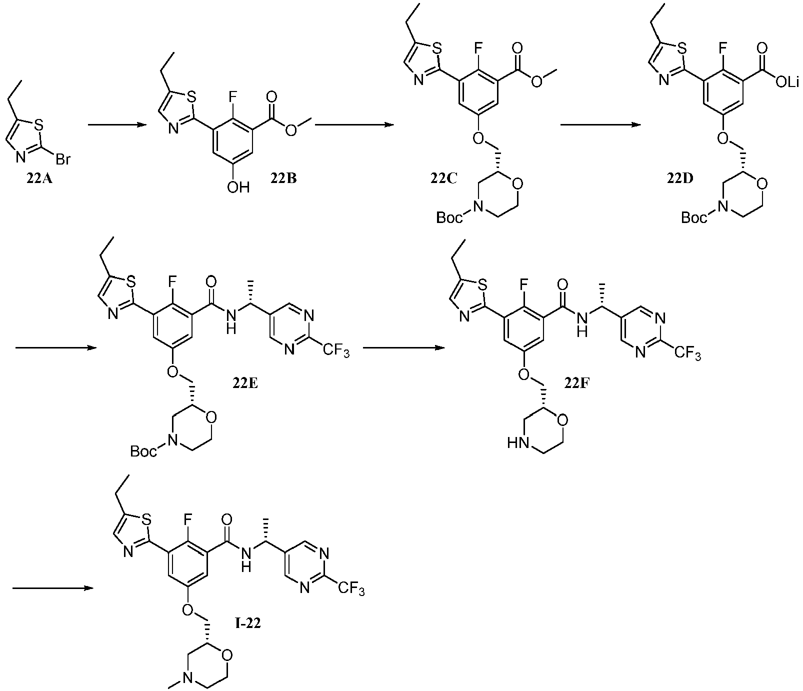

### First step: synthesis of methyl 3-(5-ethylthiazol-2-yl)-2-fluoro-5-hydroxybenzoate (22B)

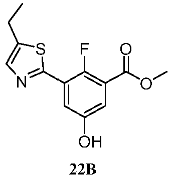

2-bromo-5-ethylthiazol (**22A**) (1.04 g, 5.40 mmol) and methyl 2-fluoro-5-hydroxy-3-(4, 4, 5, 5-tetramethyl-1, 3, 2-dioxaborolan-2-yl)benzoate (1.60 g, 5.40 mmol) (for synthesis thereof, refer to Example 1) were added to 1, 4-dioxane (10 mL) and water (2.5 mL), and then sodium carbonate (2.01 g, 19.87 mmol) was added. After 3 times of nitrogen replacement, [1, 1-bis(diphenylphosphino)ferrocene]dichloropalladium (II) (395 mg, 0.54 mmol) was added. Then, nitrogen replacement was performed again for 3 times, and the mixture was stirred overnight at 80 °C for reaction. The reaction solution was diluted by adding water (20 mL), and extracted with ethyl acetate (20 mL×2). The organic phases were concentrated. The residue was separated and purified by silica gel column (petroleum ether: ethyl acetate (V/V) =5: 1) to obtain methyl 3-(5-ethylthiazol-2-yl)-2-fluoro-5-hydroxybenzoate (**22B**), a white solid (0.8 g, yield 52.6%).

### Second step: synthesis of tert-butyl (R)-2-((3-(5-ethylthiazol-2-yl)-4-fluoro-5-(methoxycarbonyl)phenoxy)methyl)morpholine-4-c arboxylate (22C)

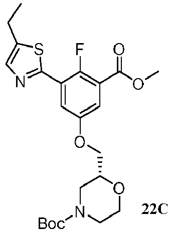

Compound methyl 3-(5-ethylthiazol-2-yl)-2-fluoro-5-hydroxybenzoate (**22B**) (0.80 g, 2.84 mmol) and tert-butyl (R)-2-((toluenesulfonyloxy)methyl)morpholine-4-carboxylate (1.06 g, 2.84 mmol) (for the synthesis thereof, refer to Patent WO2016/091776) were added to DMF (10 mL), and cesium carbonate (2.78 g, 8.53 mmol) was added. The mixture was heated to 80 °C, and stirred overnight for reaction. Thereafter, the mixture was cooled to room temperature, diluted by adding water (20 mL), and then extracted with ethyl acetate (20 mL×3). The organic phases were combined and washed with saturated salt water (10 mL×2). After liquid separation, the organic phase was dried over anhydrous sodium sulfate, filtrated, and concentrated. The residue was separated and purified by silica gel column (petroleum ether: ethyl acetate (V/V) =3: 1) to obtain tert-butyl (R)-2-((3-(5-ethylthiazol-2-yl)-4-fluoro-5-(methoxycarbonyl)phenoxy)methyl)morpholine-4-c arboxylate (**22C**), a white solid (0.75 g, yield 54.9 %).

LC-MS, M/Z (ESI): 481.5 [M+H]⁺.

### Third step: synthesis of lithium (R)-5-((4-(tert-butoxycarbonyl)morpholin-2-yl)methoxy)-3-(5-ethylthiazol-2-yl)-2-fluorobenz onate (22D)

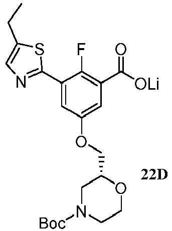

At room temperature, tert-butyl (R)-2-((3-(5-ethylthiazol-2-yl)-4-fluoro-5-(methoxycarbonyl)phenoxy)methyl)morpholine-4-c arboxylate (**22C**) (0.75 g, 1.56 mmol) was added to 8 mL of tetrahydrofuran solution, and then 2 mL of water and lithium hydroxide (0.15 g, 6.24 mmol) were added. The mixture reacted overnight at room temperature, and then concentrated to dryness. The residue was added with water (10 mL) for free-drying to obtain lithium (R)-5-((4-(tert-butoxycarbonyl)morpholin-2-yl)methoxy)-3-(5-ethylthiazol-2-yl)-2-fluorobenz onate (**22D**) (0.72 g, yield 99%).

LC-MS, M/Z (ESI): 467.5 [M+2H-Li]⁺.

### Fourth step: synthesis of tert-butyl (R)-2-((3-(5-ethylthiazol-2-yl)-4-fluoro-5-(((R)-1-(2-(trifluoromethyl)pyrimidin-5-yl)ethyl)ca rbamoyl)phenoxy)methyl)morpholine-4-carboxylate (22E)

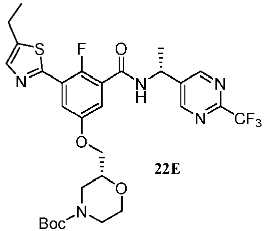

At room temperature, lithium (R)-5-((4-(tert-butoxycarbonyl)morpholin-2-yl)methoxy)-3-(5-ethylthiazol-2-yl)-2-fluorobenz onate (**22D**) (0.60 g, 1.286 mmol) was added to 10 mL of N, N-dimethylformamide, and then (R)-1-(2-(trifluoromethyl)pyrimidin-5-yl)ethyl-1-amine hydrochloride (0.35 g, 1.543 mmol) (for the synthesis thereof, refer to Patent WO2016/091776) and N, N-diisopropylethylamine (0.499 g, 3.86 mmol) were added, followed by dropwise adding a 50% N, N-dimethylformamide solution of propylphosphonic acid anhydride (2.455 g, 3.86 mmol). After the dropwise addition, the mixture was stirred and reacted overnight at room temperature under nitrogen protection. The reaction solution was diluted by adding 10 mL of water, and extracted with ethyl acetate (10 mL×2). The organic phase was concentrated to dryness. The residue was purified with a silica gel column (dichloromethane: methanol (V/V) =10: 1) to obtain tert-butyl (R)-2-((3-(5-ethylthiazol-2-yl)-4-fluoro-5-(((R)-1-(2-(trifluoromethyl)pyrimidin-5-yl)ethyl)ca rbamoyl)phenoxy)methyl)morpholine-4-carboxylate (**22E**), a white solid (0.50 g, yield 60.8%).

LC-MS, M/Z (ESI): 640.7 [M+H]⁺.

### Fifth step: synthesis of 3-(5-ethylthiazol-2-yl)-2-fluoro-5-(((R)-morpholin-2-yl)methoxy)-N-((R)-1-(2-(trifluorometh yl)pyrimidin-5-yl)ethyl)benzamide (22F)

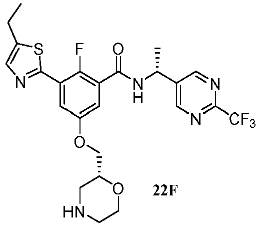

At room temperature, tert-butyl (R)-2-((3-(5-ethylthiazol-2-yl)-4-fluoro-5-(((R)-1-(2-(trifluoromethyl)pyrimidin-5-yl)ethyl)ca rbamoyl)phenoxy)methyl)morpholine-4-carboxylate (**22E**) (0.5 g, 0.782 mmol) was added to 5 mL of methanol, and then 1, 4-dioxane solution of 4M hydrogen chloride (2.0 mL, 8.0 mmol) was added. Thereafter, the mixture was stirred and reacted overnight at room temperature under nitrogen protection. The reaction solution was concentrated and then freeze-dried to obtain 3-(5-ethylthiazol-2-yl)-2-fluoro-5-(((R)-morpholin-2-yl)methoxy)-N-((R)-1-(2-(trifluorometh yl)pyrimidin-5-yl)ethyl)benzamide (**22F**), as white solid (0.2 g, yield 47.4%).

LC-MS, M/Z (ESI): 540.6 [M+H]⁺.

### Sixth step: synthesis of 3-(5-ethylthiazol-2-yl)-2-fluoro-5-(((R)-4-methylmorpholin-2-yl)methoxy)-N-((R)-1-(2-(triflu oromethyl)pyrimidin-5-yl)ethyl)benzamide (target compound I-22)

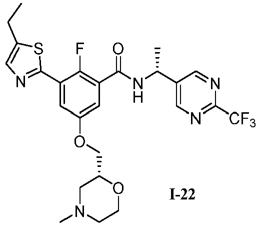

At room temperature, 3-(5-ethylthiazol-2-yl)-2-fluoro-5-(((R)-morpholin-2-yl)methoxy)-N-((R)-1-(2-(trifluorometh yl)pyrimidin-5-yl)ethyl)benzamide (**22F**) (0.2 g, 0.371 mmol) was added to 10 mL of methanol. Then, polyformaldehyde (56 mg, 1.853 mmol), sodium cyanoborohydride (116 mg, 1.853 mmol), and glacial acetic acid (2.23 mg, 0.037 mmol) were added. Thereafter, the mixture was stirred and reacted overnight at room temperature under nitrogen protection. The reaction solution was concentrated, then diluted with a saturated solution of sodium bicarbonate (10 mL), and then extracted with dichloromethane (5 mL×3). The organic phases were concentrated to dryness. The residue was separated and purified with a large silica gel plate (dichloromethane: methanol V/V) =10: 1) to obtain 3-(5-ethylthiazol-2-yl)-2-fluoro-5-(((R)-4-methylmorpholin-2-yl)methoxy)-N-((R)-1-(2-(triflu oromethyl)pyrimidin-5-yl)ethyl)benzamide (**I-22**), a white solid (26 mg, yield 12.6%).

¹H NMR (400 MHz, DMSO-d₆) δ 9.23 (d, 1H), 9.11 (s, 2H), 7.79-7.75 (m, 2H), 7.24-7.22 (m, 1H), 5.30-5.23 (m, 1H), 4.20-4.13 (m, 3H), 4.07-4.03 (m, 1H), 3.87-3.80 (m, 1H), 3.51 (d, 1H), 3.07-3.02 (m, 2H), 2.98-2.88 (m, 2H), 2.80 (s, 3H), 2.01-1.93 (m, 1H), 1.56 (d, 3H), 1.29 (t, 3H).

LC-MS, M/Z: 554.6 [M+H]⁺.

### Example 23: preparation of compound I-23

### 2-chloro-5-(((R)-4-methylmorpholin-2-yl)methoxy)-3-(5-methylthiazol-2-yl)-N-(( R)-1-(2-(trifluoromethyl)pyrimidin-5-yl)ethyl)benzamide (target compound I-23)

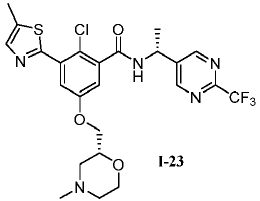

The synthesis scheme for target compound I-23 is shown below:

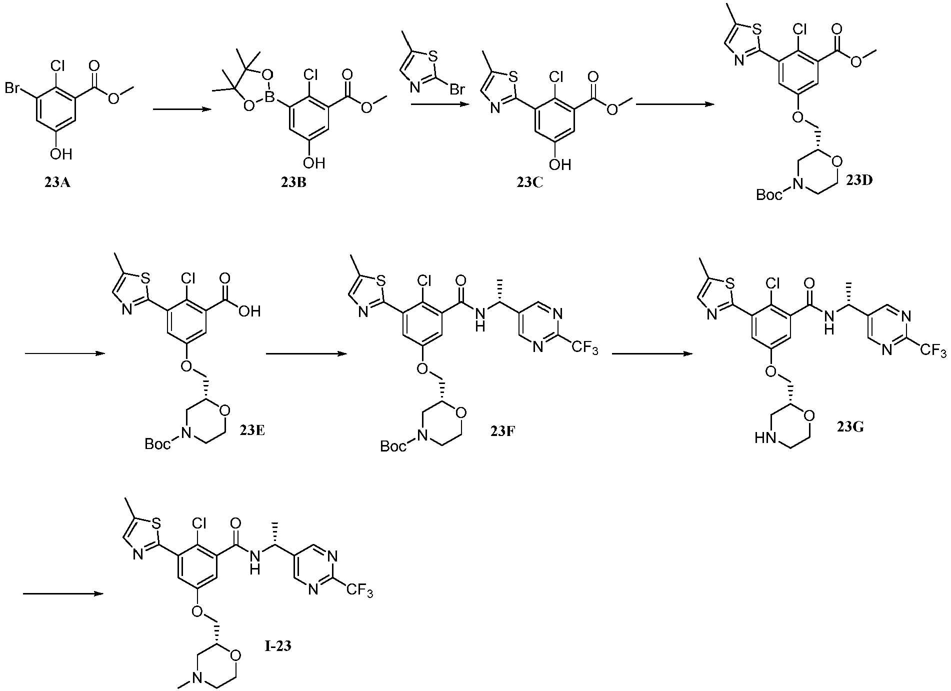

### First step: synthesis of methyl 2-chloro-5-hydroxy-3-(4, 4, 5, 5-tetramethyl-1, 3, 2-dioxaborolan-2-yl)benzoate (23B)

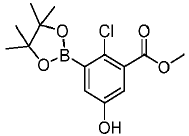

Methyl 3-bromo-2-chloro-5-hydroxybenzoate (**23A**) (1.0 g, 3.77 mmol), bis(pinacolato)diboron (1.052 g, 4.14 mmol), and potassium acetate (1.019 g, 11.30 mmol) were dissolved in 1, 4-dioxane (10 mL). The solution was degassed with a stream of nitrogen for 2 min. [1, 1-bis(diphenylphosphino)ferrocene]dichloropalladium (II) (0.138 g, 0.188 mmol) was added, and the obtained solution was degassed with a stream of nitrogen for 2 min again. Then, the reaction mixture was stirred at 100 °C for 16 hours. The reaction mixture was filtrated and concentrated in vacuum, and the residue was separated and purified by silica gel column to obtain methyl 2-chloro-5-hydroxy-3-(4, 4, 5, 5-tetramethyl-1, 3, 2-dioxaborolan-2-yl)benzoate, a white solid (0.69 g, yield 60.0%).

### Second step: synthesis of methyl 2-chloro-5-hydroxy-3-(5-methylthiazol-2-yl)benzoate (23C)

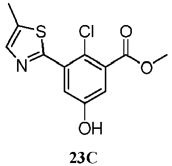

2-bromo-5-methylthiazole (0.43 g, 2.43 mmol) and methyl 2-chloro-5-hydroxy-3-(4, 4, 5, 5-tetramethyl-1, 3, 2-dioxaborolan-2-yl)benzoate (**23B**) (0.69 g, 2.21 mmol) was added to 1, 4-dioxane (5 mL) and water (1.5 mL), and then, sodium carbonate (0.468 g, 4.42 mmol) was added. After 3 times of nitrogen replacement, [1, 1-bis(diphenylphosphino)ferrocene]dichloropalladium (II) (162 mg, 0.22 mmol) was added, followed by 3 times of nitrogen replacement again. The mixture was stirred overnight at 80 °C for reaction. Thereafter, the reaction solution was diluted by adding water (20 mL), and then extracted with ethyl acetate (20 mL×2). The organic phases were concentrated, and the residue was separated and purified by silica gel column (petroleum ether: ethyl acetate (V/V) =5: 1) to obtain methyl 2-chloro-5-hydroxy-3-(5-methylthiazol-2-yl)benzoate (**23C**), a white solid (0.38 g, yield 60.7 %).

### Third step: synthesis of tert-butyl (R)-2-((4-chloro-3-(methoxycarbonyl)-5-(5-methylthiazol-2-yl)phenoxy)methyl)morpholine-4-carboxylate (23D)

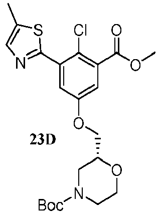

Compound methyl 2-chloro-5-hydroxy-3-(5-methylthiazol-2-yl)benzoate (0.38 g, 1.34 mmol) and tert-butyl (R)-2-((toluenesulfonyloxy)methyl)morpholine-4-carboxylate (**23C**) (0.55 g, 1.47 mmol) (for the synthesis thereof, refer to Patent WO2016/091776) were added to DMF (5 mL), and then, cesium carbonate (1.31 g, 4.02 mmol) was added. The mixture was heated to 80 °C and stirred overnight for reaction. Then, the mixture was cooled to room temperature, diluted by adding water (20 mL), and extracted with ethyl acetate (20 mL×3). The organic phases were combined and washed with saturated salt water (10 mL×2). After liquid separation, the organic phase was dried over anhydrous sodium sulfate, filtrated, and concentrated, and the residue was separated and purified by silica gel column (petroleum ether: ethyl acetate (V/V) =5: 1) to obtain (R)-2-((4-chloro-3-(methoxycarbonyl)-5-(5-methylthiazol-2-yl)phenoxy)methyl)morpholine-4-carboxylate (**23D**), a colorless oily liquid (0.42 g, yield 64.9 %).

LC-MS, M/Z (ESI): 483.1 [M+H]⁺.

### Fourth step: synthesis of (R)-5-((4-(tert-butoxycarbonyl)morpholin-2-yl)methoxy)-2-chloro-3-(5-methylthiazol-2-yl)be nzoic acid (23E)

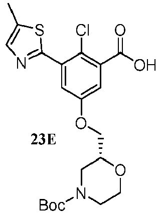

At room temperature, tert-butyl (R)-2-(((4-chloro-3-(methoxycarbonyl)-5-(5-methylthiazol-2-yl)phenoxy)methyl)morpholine-4-carboxylate (**23D**) (0.42 g, 0.87 mmol) was added to 5 mL of tetrahydrofuran solution. Then, 1 mL of water and sodium hydroxide (0.07 g, 1.74 mmol) were added. The mixture was heated to 60 °C, and stirred for reaction for 2 hours. Thereafter, the reaction solution was concentrated to dryness. The residue was added with water (10 mL), and pH was adjusted to 6 by dropwise adding 1M hydrochloric acid. The obtained olution was extracted with dichloromethane (20 mL×3) and concentrated to dryness for free-drying to obtain (R)-5-((4-(tert-butoxycarbonyl)morpholin-2-yl)methoxy)-2-chloro-3-(5-methylthiazol-2-yl)be nzoic acid (**23E**) (0.4 g, yield 98%).

LC-MS, M/Z (ESI): 469.1 [M+H]⁺.

### Fifth step: synthesis of tert-butyl (R)-2-((4-chloro-3-(5-methylthiazol-2-yl)-5-(((R)-1-(2-(trifluoromethyl)pyrimidin-5-yl)ethyl) carbamoyl)phenoxy)methyl)morpholine-4-carboxylate (23F)

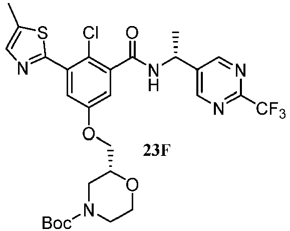

At room temperature, (R)-5-((4-(tert-butoxycarbonyl)morpholin-2-yl)methoxy)-2-chloro-3-(5-methylthiazol-2-yl)be nzoic acid (**23E**) (0.3 g, 0.64 mmol) was added to 5 mL of N, N-dimethylformamide, and then (R)-1-(2-(trifluoromethyl)pyrimidin-5-yl)ethyl-1-amine hydrochloride (0.18 g, 0.77 mmol) (for the synthesis thereof, refer to Patent WO2016/091776) and N, N-diisopropylethylamine (0.25 g, 1.91 mmol) were added. 50% N, N-dimethylformamide solution of propylphosphonic acid anhydride (1.22 g, 1.91 mmol) was added dropwise. After the dropwise addition, the mixture was stirred and reacted overnight at room temperature under nitrogen protection. Thereafter, the reaction solution was diluted by adding 10 mL of water and extracted with ethyl acetate (10 mL×2). The organic phase was concentrated to dryness. The residue was purified with silica gel column (dichloromethane: methanol (V/V) =10: 1) to obtain tert-butyl (R)-2-((4-chloro-3-(5-methylthiazol-2-yl)-5-(((R)-1-(2-(trifluoromethyl)pyrimidin-5-yl)ethyl) carbamoyl)phenoxy)methyl)morpholine-4-carboxylate (**23F**), as white solid (0.25g, yield 62.0%).

LC-MS, M/Z (ESI): 642.2 [M+H]⁺.

### Sixth step: synthesis of 2-chloro-3-(5-methylthiazol-2-yl)-5-(((R)-morpholin-2-yl)methoxy)-N-((R)-1-(2-(trifluorome thyl)pyrimidin-5-yl)ethyl)benzamide (23G)

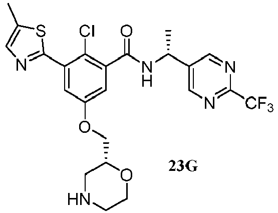

At room temperature, tert-butyl (R)-2-((4-chloro-3-(5-methylthiazol-2-yl)-5-(((R)-1-(2-(trifluoromethyl)pyrimidin-5-yl)ethyl) carbamoyl)phenoxy)methyl)morpholine-4-carboxylate (**23F**) (0.25 g, 0.39 mmol) was added to 5 mL of methanol. A 1, 4-dioxane solution of 4M hydrogen chloride (1.0 mL, 4.0 mmol) was then added. Thereafter, the mixture was stirred and reacted overnight at room temperature under nitrogen protection. The reaction solution was concentrated and freeze-dried to obtain 2-chloro-3-(5-methylthiazol-2-yl)-5-(((R)-morpholin-2-yl)methoxy)-N-((R)-1-(2-(trifluorome thyl)pyrimidin-5-yl)ethyl)benzamide (**23G**), a white solid (0.07 g, yield 33.1%).

LC-MS, M/Z (ESI): 542.1 [M+H]⁺.

### Seventh step: synthesis of 2-chloro-5-(((R)-4-methylmorpholin-2-yl)methoxy)-3-(5-methylthiazol-2-yl)-N-((R)-1-(2-(tri fluoromethyl)pyrimidin-5-yl)ethyl)benzamide (target compound I-23)

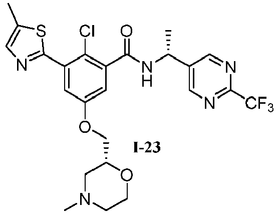

At room temperature, 2-chloro-3-(5-methylthiazol-2-yl)-5-(((R)-morpholin-2-yl)methoxy)-N-((R)-1-(2-(trifluorome thyl)pyrimidin-5-yl)ethyl)benzamide (**23G**) (0.07 g, 0.13 mmol) was added to 5 mL of methanol. Then, polyformaldehyde (19 mg, 0.65 mmol), sodium cyanoborohydride (41 mg, 0.65mmol), and glacial acetic acid (0.77 mg, 0.01 mmol) were added. Thereafter, the mixture was stirred and reacted overnight at room temperature under nitrogen protection. The reaction solution was concentrated, then diluted with a saturated solution of sodium bicarbonate (10 mL), and extracted with dichloromethane (5 mL×3). The organic phase was concentrated to dryness. The residue was separated with a large silica gel plate (dichloromethane: methanol V/V) =10: 1) to prepare 2-chloro-5-(((R)-4-methylmorpholin-2-yl)methoxy)-3-(5-methylthiazol-2-yl)-N-((R)-1-(2-(tri fluoromethyl)pyrimidin-5-yl)ethyl)benzamide (**I-23**), a white solid (12 mg, yield 16.6%).

¹H NMR (400 MHz, DMSO-d₆) δ 9.20 (d, 1H), 9.10 (s, 2H), 7.70 (d, 1H), 7.66 (d, 1H), 7.20 (d, 1H), 5.26-5.15 (m, 1H), 4.05 (d, 1H), 3.88-3.64 (m, 2H), 3.62-3.43 (m, 1H), 2.74 (d, 1H), 2.57 (d, 1H), 2.50 (s, 3H), 2.17 (s, 3H), 2.02-1.92 (m, 2H), 1.91-1.83 (m, 1H), 1.52 (d, 3H).

LC-MS, M/Z: 556.1 [M+H]⁺.

### Example 24: preparation of compound I-24

### (R)-2-fluoro-5-((4-methylmorpholin-2-yl)methoxy)-3-(5-methylthiazol-2-yl)-N-((2-(trifluoro methyl)pyrimidin-5-yl)methyl)benzamide (target compound I-24)

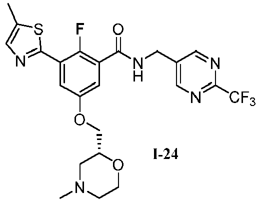

The synthesis scheme for target compound I-24 is shown below:

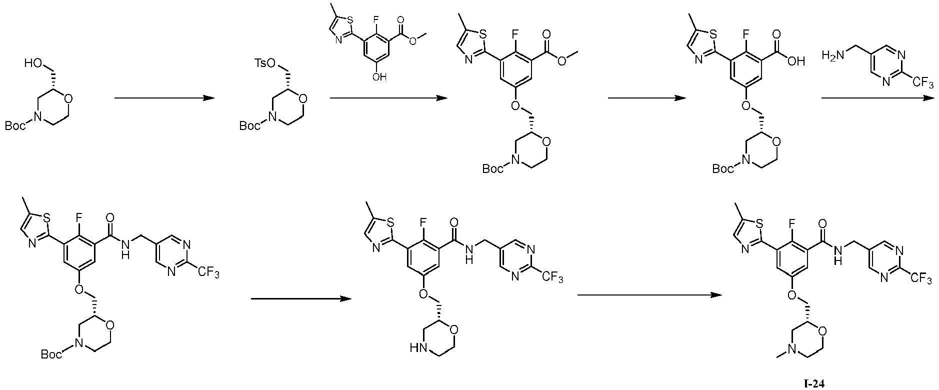

### First step: synthesis of tert-butyl (R)-2-((toluenesulfonyloxy)methyl)morpholine-4-carboxylate

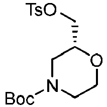

Tert-butyl (R)-2-(hydroxymethyl)morpholine-4-carboxylate (10.0 g, 46.0 mmol), 4-dimethylaminopyridine (1.125 g, 9.21 mmol), and triethylamine (9.32 g, 92.0 mmol) were added to anhydrous dichloromethane (100 mL). At room temperature, p-toluenesulfonyl chloride (9.65 g, 50.6 mmol) was added, and the mixture reacted at room temperature for 15 h. TLC (petroleum ether: ethyl acetate (V/V) = 3: 1) monitored that the reaction of the raw materials was complete. Water (100 mL) and DCM (300 mL) were added to the reaction solution. The aqueous phase was extracted with DCM (100 mL×3). The extracts were combined, washed with saturated salt water (100 mL), and dried over sodium sulfate, followed by filtration and rotary evaporation, to obtain a crude product. The crude product was separated and purified with silica gel column (petroleum ether: ethyl acetate (V/V) =100: 1 to 1: 1) to obtain a compound tert-butyl (R)-2-((toluenesulfonyloxy)methyl)morpholine-4-carboxylate (12.0 g, yield 70.2 %).

### Second step: synthesis of tert-butyl (R)-2-((4-fluoro-3-(methoxycarbonyl)-5-(5-methylthiazol-2-yl)phenoxy)methyl)morpholine-4 -carboxylate

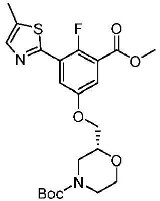

Methyl 2-fluoro-5-hydroxy-3-(5-methylthiazol-2-yl)benzoate (0.60 g, 2.245 mmol) and cesium carbonate (1.46 g, 4.49 mmol) were added to DMF (10 mL). Then, tert-butyl (R)-2-((toluenesulfonyloxy)methyl)morpholine-4-carboxylate (1.00 g, 2.69 mmol) was added. The reaction solution reacted at 100 °C for 15 h. TLC (petroleum ether: ethyl acetate (V/V) = 1: 1) monitored that most of the raw materials was completely reacted. Water (100 mL) and EA (100 mL) were added to the reaction solution. The aqueous phase was extracted with EA (100 mL×3). The extracts were combined, washed with saturated salt water (100 mL×3), and dried over sodium sulfate, followed by filtration and rotary evaporation, to obtain a crude product. The crude product was separated and purified with silica gel column (petroleum ether: ethyl acetate (V/V) =30: 1 to 1: 100) to obtain tert-butyl (R)-2-((4-fluoro-3-(methoxycarbonyl)-5-(5-methylthiazol-2-yl)phenoxy)methyl)morpholine-4 -carboxylate (0.60 g, yield 57.3 %).

### Third step: synthesis of (R)-5-((4-(tert-butoxycarbonyl)morpholin-2-yl)methoxy)-2-fluoro-3-(5-methylthiazol-2-yl)be nzoic acid (4)

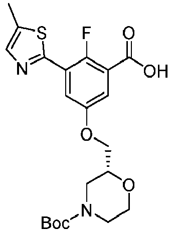

Tert-butyl (R)-2-((4-fluoro-3-(methoxycarbonyl)-5-(5-methylthiazol-2-yl)phenoxy)methyl)morpholine-4 -carboxylate (0.60 g, 1.286 mmol) was added to methanol (10 mL), THF (2 mL), and water (2 mL). At room temperature, LiOH·H₂O (0.123 g, 5.14 mmol) was added. The reaction solution reacted at room temperature for 15 hours. TLC (petroleum ether: ethyl acetate (V/V) = 1: 1) monitored that the reaction of the raw materials was complete. The reaction solution was dried through rotatory evaporation. The crude product was added with water (50 mL) and EA (100 mL), and pH was adjusted to 5 with 1N hydrochloric acid, followed by extraction with ethyl acetate (100 mL×2). The organic phases were combined, washed with saturated salt water (50 mL), dried over sodium sulfate, and concentrated, to obtain (R)-5-((4-(tert-butoxycarbonyl)morpholin-2-yl)methoxy)-2-fluoro-3-(5-methylthiazol-2-yl)be nzoic acid (0.50 g, yield 86.0 %).

### Fourth step: synthesis of tert-butyl (R)-2-((4-fluoro-3-(5-methylthiazol-2-yl)-5-(((2-(trifluoromethyl)pyrimidin-5-yl)metyl)carba moyl)phenoxy)methyl)morpholine-4-carboxylate

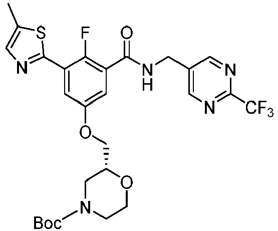

(R)-5-((4-(tert-butoxycarbonyl)morpholin-2-yl)methoxy)-2-fluoro-3-(5-methylthia zol-2-yl)benzoic acid (0.15 g, 0.331 mmol), T₃P (0.316 g, 0.497 mmol 50% in DMF), and DIPEA (0.171 g, 1.326 mmol) were added to DMF (5 mL). The mixture was stirred and reacted at room temperature for 0.5 h, and then added with (2-(trifluoromethyl)pyrimidin-5-yl) methylamine (0.088 g, 0.497 mmol). The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified with silica gel column (petroleum ether: ethyl acetate (V/V) =30: 1 to 1: 100) to obtain a compound tert-butyl (R)-2-((4-fluoro-3-(5-methylthiazol-2-yl)-5-(((2-(trifluoromethyl)pyrimidin-5-yl)methyl)carb amoyl)phenoxy)methyl)morpholine-4-carboxylate (0.10 g, yield 49.3 %).

### Fifth step: synthesis of (R)-2-fluoro-3-(5-methylthiazol-2-yl)-5-(morpholin-2-yl-methoxy)-N-((2-(trifluoromethyl)py rimidin-5-yl)methyl)benzamide (7)

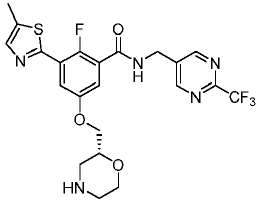

Tert-butyl (R)-2-((4-fluoro-3-(5-methylthiazol-2-yl)-5-(((2-(trifluoromethyl)pyrimidin-5-yl)methyl)carb amoyl)phenoxy)methyl)morpholine-4-carboxylate (0.10 g, 0.164 mmol) was added to DCM (2 mL), and then HCl/Dioxane (5 mL, 3 mol/L) was added. The mixture reacted at room temperature for 15 h. TLC (petroleum ether: ethyl acetate (V/V) = 1: 1) monitored that the reaction of the raw materials was complete. The reaction solution was dried through rotatory evaporation to obtain a compound (R)-2-fluoro-3-(5-methylthiazol-2-yl)-5-(morpholin-2-yl-methoxy)-N-((2-(trifluoromethyl)py rimidin-5-yl)methyl)benzamide (0.05 g, yield 59.8 %).

### Sixth step: synthesis of (R)-2-fluoro-5-((4-methylmorpholin-2-yl)methoxy)-3-(5-methylthiazol-2-yl)-N-((2-(trifluoro methyl)pyrimidin-5-yl)methyl)benzamide (I-24)

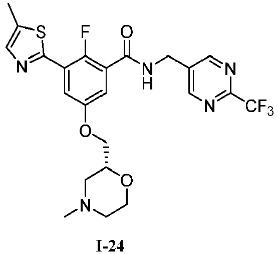

(R)-2-fluoro-3-(5-methylthiazol-2-yl)-5-(morpholin-2-yl-methoxy)-N-((2-(trifluor omethyl)pyrimidin-5-yl)methyl)benzamide (**7**) (0.05 g, 0.098 mmol), polyformaldehyde (0.015 g, 0.489 mmol), and NaBH3CN (0.031 g, 0.489 mmol) were added to MeOH (5 mL), and then acetic acid (0.2 mL) was added. The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified through Pre-TLC (petroleum ether: ethyl acetate (V/V) = 1: 1), to obtain a white solid of (R)-2-fluoro-5-((4-methylmorpholin-2-yl)methoxy)-3-(5-methylthiazol-2-yl)-N-((2-(trifluoro methyl)pyrimidin-5-yl)methyl)benzamide (target compound **I-24**) (10 mg, yield 19.47 %).

¹H NMR (400 MHz, DMSO) δ 9.19 (s, 1H), 9.04 (s, 2H), 7.72 (s, 2H), 7.27 (s, 1H), 4.61 (d, *J* = 4.4 Hz, 2H), 4.04 (d, *J* = 4.0 Hz, 2H), 3.78 (d, *J* = 9.0 Hz, 2H), 3.53 (d, *J* = 10.8 Hz, 1H), 2.75 (d, *J* = 10.8 Hz, 1H), 2.57 (d, *J* = 10.8 Hz, 1H), 2.51 (s, 3H), 2.17 (s, 3H), 1.93 (dd, *J* = 29.8, 10.0 Hz, 2H).

LC-MS, M/Z: 526.2 [M+H]⁺.

### Example 25: preparation of compound I-25

### 2-fluoro-5-(((R)-4-methylmorpholin-2-yl)methoxy)-3-(5-methylthiazol-2-yl)-N-(( R)-1-(6-(trifluoromethyl)pyridazin-3-yl)ethyl)benzamide (target compound 1-25)

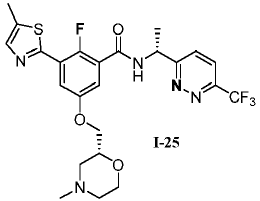

For the synthesis thereof, refer to Example 19.

LC-MS, M/Z: 540.1 [M+H]⁺.

### Example 26: preparation of compound I-26

### 2-fluoro-5-(((R)-4-methylmorpholin-2-yl)methoxy)-N-((R)-1-(2-methylpyrimidin-5-yl)ethyl)-3-(5-methylthiazol-2-yl)benzamide (target compound I-26)

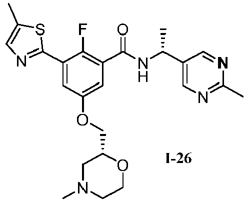

The synthesis scheme for target compound I-26 is shown below:

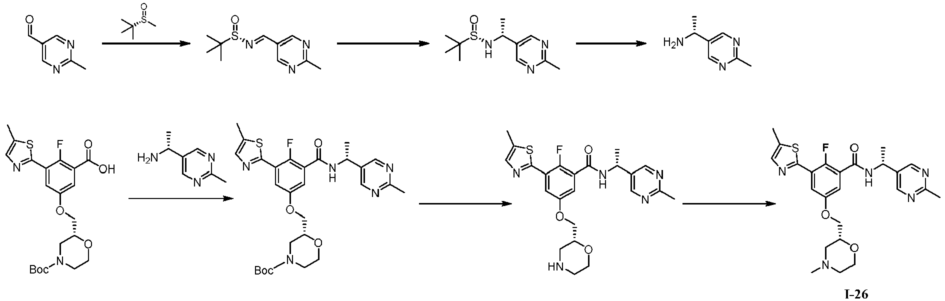

### First step: synthesis of (S)-2-methyl-N-((2-methylpyrimidin-5-yl)methylene)propane-2-sulfinamide

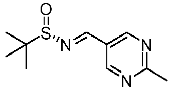

2-methylpyrimidine-5-carbaldehyde (1.0 g, 8.19 mmol) and cesium carbonate (3.20 g, 9.83 mmol) were added to anhydrous dichloromethane (20 mL). At room temperature, (S)-2-methylpropane-2-sulfinamide (1.19 g, 9.83 mmol) was added. The mixture reacted at room temperature for 15 h. TLC (petroleum ether: ethyl acetate (V/V) = 2: 1) monitored that the reaction of the raw materials was complete. Water (100 mL) and DCM (100 mL) were added to the reaction solution. The aqueous phase was extracted with DCM (100 mL×3). The extracts were combined, washed with saturated salt water (100 mL), and dried over sodium sulfate, followed by filtration and rotatory evaporation, to obtain a crude product. The crude product was separated and purified with silica gel column (petroleum ether: ethyl acetate (V/V) =100: 1 to 1: 1) to obtain a compound (S)-2-methyl-N-((2-methylpyrimidin-5-yl)methylene)propane-2-sulfinamide (1.2 g, yield 65.0 %).

### Second step: synthesis of (S)-2-methyl-N-((R)-1-(2-methylpyrimidin-5-yl)ethyl)propane-2-sulfinamide

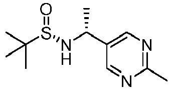

(S)-2-methyl-N-((2-methylpyrimidin-5-yl)methylene)propane-2-sulfinamide (1.20 g, 5.33 mmol) was added to anhydrous THF (20 mL), and methylmagnesium chloride (10.6 mL, 32 mmol, 3 mol/L) was added dropwise at -10 °C. The reaction solution reacted at 0°C for 3 h. TLC (petroleum ether: ethyl acetate (V/V) = 2: 1) monitored that most of the raw materials was completely reacted. Water (100 mL) and EA (100 mL) were added to the reaction solution. The aqueous phase was extracted with EA (100 mL×3). The extracts were combined, washed with saturated salt water (100 mL×3), and dried over sodium sulfate, followed by filtration and rotatory evaporation, to obtain a crude product. The crude product was separated and purified with silica gel column (petroleum ether: ethyl acetate (V/V) =30: 1 to 1: 100) to obtain a compound (S)-2-methyl-N-((R)-1-(2-methylpyrimidin-5-yl)ethyl)propane-2-sulfinamide (0.60 g, yield 46.7 %).

### Third step: synthesis of (R)-1-(2-methylpyrimidin-5-yl)ethyl-1-amine

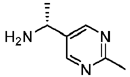

(S)-2-methyl-N-((R)-1-(2-methylpyrimidin-5-yl)ethyl)propane-2-sulfinamide (0.60 g, 2.486 mmol) was added to EA (5 mL), and then HCl/Dioxane (15 mL 3 mol/L) was added. The mixture reacted at room temperature for 15 h. TLC (petroleum ether: ethyl acetate (V/V) = 1: 1) monitored that the reaction of the raw materials was complete. The reaction solution was dried through rotatory evaporation to obtain a compound (R)-1-(2-methylpyrimidin-5-yl)ethyl-1-amine (0.7 g, yield 61.6 %).

### Fourth step: synthesis of tert-butyl (R)-2-((4-fluoro-3-(((R)-1-(2-methylpyrimidin-5-yl)ethyl)carbamoyl)-5-(5-methylthiazol-2-yl )phenoxy)methyl)morpholine-4-carboxylate

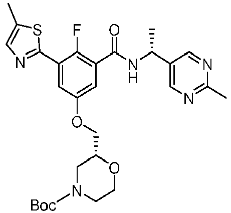

(R)-5-((4-(tert-butoxycarbonyl)morpholin-2-yl)methoxy)-2-fluoro-3-(5-methylthia zol-2-yl)benzoic acid (HW091050-IN-04) (0.20 g, 0.442 mmol), T3P (0.42 g, 0.663 mmol 50% in DMF) and DIPEA (0.23 g, 1.768 mmol) were added to DMF (5 mL). The mixture was stirred and reacted at room temperature for 0.5 h, and then added with (R)-1-(2-methylpyrimidin-5-yl)ethyl-1-amine (HW091050-IN-03) (0.091 g, 0.663 mmol). The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified with silica gel column (petroleum ether: ethyl acetate (V/V) =30: 1 to 1: 100) to obtain a compound tert-butyl (R)-2-((4-fluoro-3-(((R)-1-(2-methylpyrimidin-5-yl)ethyl)carbamoyl)-5-(5-methylthiazol-2-yl )phenoxy)methyl)morpholine-4-carboxylate (0.20 g, yield 79.0 %).

### Fifth step: synthesis of 2-fluoro-N-((R)-1-(2-methylpyrimidin-5-yl)ethyl)-3-(5-methylthiazol-2-yl)-5-(((R)-morpholin -2-yl)methoxy)benzamide

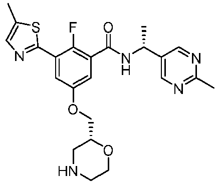

Tert-butyl (R)-2-((4-fluoro-3-(((R)-1-(2-methylpyrimidin-5-yl)ethyl)carbamoyl)-5-(5-methylthiazol-2-yl )phenoxy)methyl)morpholine-4-carboxylate (0.20 g, 0.350 mmol) was added to DCM (2 mL), and then HCl/Dioxane (5 mL, 3 mol/L) was added. The mixture reacted at room temperature for 15 h. TLC (petroleum ether: ethyl acetate (V/V) = 1: 1) monitored that the reaction of the raw materials was complete. The reaction solution was dried through rotatory to obtain a compound 2-fluoro-N-((R)-1-(2-methylpyrimidin-5-yl)ethyl)-3-(5-methylthiazol-2-yl)-5-(((R)-morpholin -2-yl)methoxy)benzamide (0.10 g, yield 60.6 %).

### Sixth step: synthesis of 2-fluoro-5-(((R)-4-methylmorpholin-2-yl)methoxy)-N-((R)-1-(2-methylpyrimidin-5-yl)ethyl)-3-(5-methylthiazol-2-yl)benzamide (I-26)

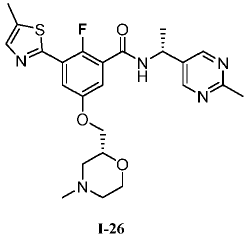

2-fluoro-N-((R)-1-(2-methylpyrimidin-5-yl)ethyl)-3-(5-methylthiazol-2-yl)-5-(((R) -morpholin-2-yl)methoxy)benzamide (0.10 g, 0.212 mmol), polyformaldehyde (0.032 g, 1.060 mmol), and NaBH₃CN (0.067 g, 1.060 mmol) were added to MeOH (5 mL), and then acetic acid (0.2 mL) was added. The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified through Pre-TLC (petroleum ether: ethyl acetate (V/V) = 1: 1), to obtain a white solid of 2-fluoro-5-(((R)-4-methylmorpholin-2-yl)methoxy)-N-((R)-1-(2-methylpyrimidin-5-yl)ethyl)-3-(5-methylthiazol-2-yl)benzamide (**I-26**) (16 mg, yield 15.54 %).

¹H NMR (400 MHz, DMSO) δ 9.07 (d, *J* = 7.6 Hz, 1H), 8.70 (s, 2H), 7.71 (t, *J* = 4.0 Hz, 2H), 7.15 (dd, *J* = 5.0, 3.2 Hz, 1H), 5.13-5.08 (m, 1H), 4.04 (d, *J* = 5.0 Hz, 2H), 3.81 - 3.75 (m, 2H), 3.58-3.46 (m, 2H), 2.75 (d, *J* = 11.0 Hz, 1H), 2.59 (s, 3H), 2.51 (d, *J* = 0.6 Hz, 3H), 2.17 (s, 3H), 1.99 (dd, *J* = 11.0, 8.0 Hz, 1H), 1.88 (t, *J* = 10.6 Hz, 1H), 1.48 (d, *J* = 7.0 Hz, 3H).

LC-MS, M/Z: 486.1 [M+H]⁺.

### Example 27: preparation of compound I-27

### 2-fluoro-5-(((2S, 3R)-3-hydroxybutan-2-yl)oxy)-3-(5-methylthiazol-2-yl)-N-((R)-1-(2-(trifluoromethyl)pyrimid in-5-yl)ethyl)benzamide (I-27)

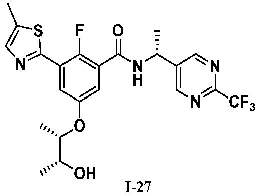

The synthesis scheme for target compound I-27 is shown below:

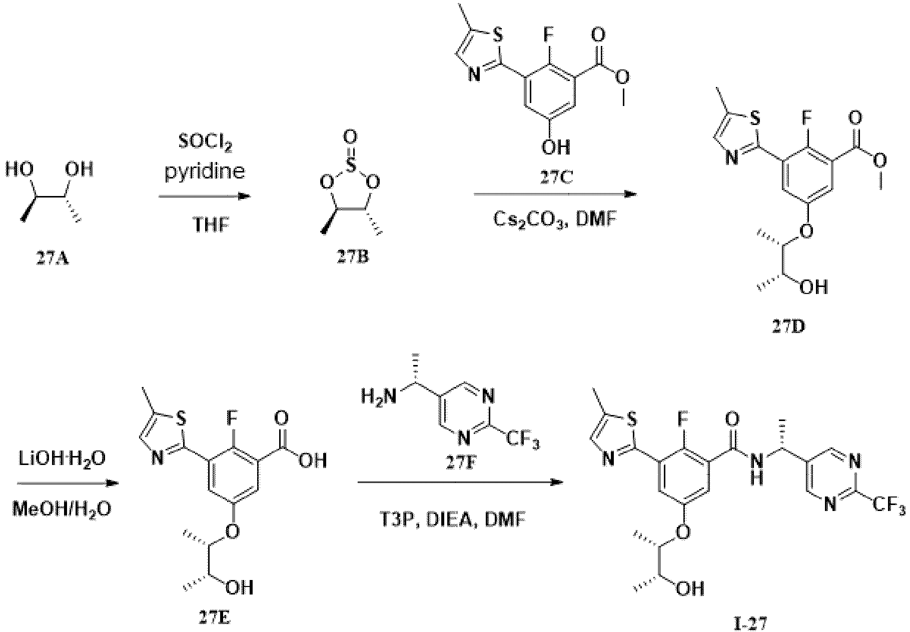

### First step: synthesis of (4R, 5R)-4, 5-dimethyl-1, 3, 2-dioxathiolane 2-oxide (27B)

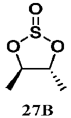

(2R, 3R)-(-)-2, 3-butanediol (2 g, 22.19 mmol) and pyridine (3.86 g, 48.8 mmol) were dissolved in dried tetrahydrofuran (20 mL). After the reaction temperature was adjusted to 0°C to 5 °C, thionyl chloride (2.9 g, 24.41mmol) was slowly added. Then, the reaction temperature was raised to room temperature, and the mixture was stirred for 16 h. The reaction was quenched by adding water (30 mL). The reaction solution was added with 20 mL of ethyl acetate, followed by liquid separation. The organic phase was washed with a saturated solution of ammonium chloride (20 mL) and saturated aqueous sodium chloride (20 mL), dried over anhydrous sodium sulfate, and concentrated under reduced pressure, to obtain colorless liquid of (4R, 5R)-4, 5-dimethyl-1, 3, 2-dioxathiolane 2-oxide (27B) (2.4 g, yield 79%).

¹H NMR (400 MHz, Chloroform-d) δ 4.63 (dq, J=9.0, 6.1 Hz, 1H), 4.07 (dq, J=9.0, 6.1 Hz, 1H), 1.52 (d, J=6.2 Hz, 3H), 1.43 (d, J=6.1 Hz, 3H).

### Second step: synthesis of methyl 2-fluoro-5-(((2S, 3R)-3-hydroxybutan-2-yl)oxy)-3-(5-methylthiazol-2-yl)benzoate (27D)

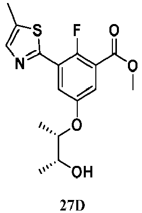

Under nitrogen protection, cesium carbonate (1.22g, 3.74 mmol) was added to a solution of methyl 2-fluoro-5-hydroxy-3-(5-methylthiazol-2-yl)benzoate (500 mg, 1.871 mmol) (for synthesis thereof, refer to Example 1) in N, N-dimethylformamide (5 mL). The mixture was stirred at room temperature for 30 min. Then, (4R, 5R)-4, 5-dimethyl-1, 3, 2-dioxathiolane 2-oxide (**27B**) (382 mg, 2.81 mmol) was added. The mixture was heated to 80 °C and reacted for 16 h, and was then cooled to room temperature. The reaction solution was concentrated to dryness under reduced pressure. Chloroform (20 mL) and a 4M sulfuric acid solution (20 mL) were added, and the mixture solution was stirred at 70 °C for 5 h, followed by liquid separation. The aqueous phase was adjusted to pH 7 to 8 with sodium bicarbonate and extracted with dichloromethane (20 mL×3). The organic phases were combined, dried over anhydrous sodium sulfate, and concentrated. The residue was purified with a silica gel column (petroleum ether: ethyl acetate (V/V) =2: 1) to obtain a white solid of methyl 2-fluoro-5-(((2S, 3R)-3-hydroxybutan-2-yl)oxy)-3-(5-methylthiazol-2-yl)benzoate (**27D**) (380 mg, yield 60%).

LC-MS, M/Z: 340.1 [M+H]⁺.

### Third step: synthesis of 2-fluoro-5-(((2S, 3R)-3-hydroxybutan-2-yl)oxy)-3-(5-methylthiazol-2-yl)benzoic acid (27E)

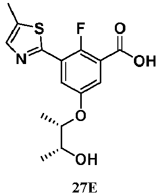

Methyl 2-fluoro-5-(((2S, 3R)-3-hydroxybutan-2-yl)oxy)-3-(5-methylthiazol-2-yl)benzoate (27D) (200 mg, 0.589 mmol) was dissolved in methanol (4 mL), and then lithium hydroxide monohydrate (70.7 mg, 1.765 mmol) and water (0.4 mL) were added. The mixture was stirred and reacted at room temperature for 16h. Thereafter, the reaction solution was directly concentrated to dryness. Then, the residue was added with water (5 mL), and pH was adjusted to 2 to 3 with a 1M aqueous hydrochloric acid solution. The aqueous phase was extracted with dichloromethane (5 mL×3). The organic phases were combined, dried over anhydrous sodium sulfate, and concentrated to obtain a white solid of 2-fluoro-5-(((2S, 3R)-3-hydroxybutan-2-yl)oxy)-3-(5-methylthiazol-2-yl)benzoic acid (**27E**) (190 mg, yield 99%).

LC-MS, M/Z: 326.1 [M+H]⁺.

### Fourth step: 2-fluoro-5-(((2S, 3R)-3-hydroxybutan-2-yl)oxy)-3-(5-methylthiazol-2-yl)-N-((R)-1-(2-(trifluoromethyl)pyrimid in-5-yl)ethyl)benzamide (I-27)

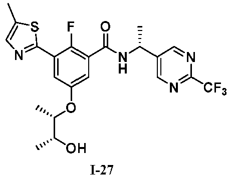

Under nitrogen protection, 2-fluoro-5-(((2S, 3R)-3-hydroxybutan-2-yl)oxy)-3-(5-methylthiazol-2-yl)benzoic acid (**27E**) (190 mg, 0.584 mmol), (R)-1-(2-(trifluoromethyl)pyrimidin-5-yl)ethyl-1-amine hydrochloride (160 mg, 0.701 mmol), N, N-diisopropylethylamine (226 mg, 1.752 mmol), and N, N-dimethylformamide (5 mL) were successively added to a reaction flask. The mixture was cooled to about 0°C, and added with a solution of propylphosphonic acid anhydride in N, N-dimethylformamide (50%, 557 mg, 0.876 mmol) dropwise. Thereafter, the mixture was heated to room temperature and reacted for 16 h. The reaction was quenched by adding a saturated solution of sodium bicarbonate (5 mL), and the reaction mixture was extracted with ethyl acetate (10 mL×3). The organic phases were combined, washed with saturated salt water (30 mL×2), dried over anhydrous sodium sulfate, and concentrated. The residue was separated and purified with a silica gel plate (petroleum ether: ethyl acetate (V/V) =1: 1) to obtain a white solid of 2-fluoro-5-(((2S, 3R)-3-hydroxybutan-2-yl)oxy)-3-(5-methylthiazol-2-yl)-N-((R)-1-(2-(trifluoromethyl)pyrimid in-5-yl)ethyl)benzamide (**I-27**) (110 mg, yield 37.8%).

¹H NMR (400 MHz, Chloroform-d) δ 8.94 (s, 2H), 7.86 (dd, J=5.9, 3.3 Hz, 1H), 7.63-7.58 (m, 1H), 7.50 (dd, J=5.8, 3.4 Hz, 1H), 7.10 (dd, J=12.3, 6.5 Hz, 1H), 5.38 (ddd, J=7.7, 4.4, 1.5 Hz, 1H), 4.40 (qd, J=6.3, 3.3 Hz, 1H), 4.01 (ddd, J=6.5, 4.8, 3.3 Hz, 1H), 2.56 (d, J=1.2 Hz, 3H), 2.07 (d, J=4.9 Hz, 1H), 1.72 (d, J=7.1 Hz, 3H), 1.26 (d, J=6.3 Hz, 3H), 1.23 (d, J=6.5 Hz, 3H).

LC-MS, M/Z: 499.1 [M+H]⁺.

### Example 28: preparation of compound I-28

### 2-fluoro-5-(((2S, 3S)-3-hydroxybutan-2-yl)oxy)-3-(5-methylthiazol-2-yl)-N-((R)-1-(2-(trifluoromethyl)pyrimid in-5-yl)ethyl)benzamide (I-28)

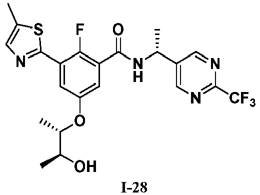

The synthesis scheme for target compound I-28 is shown below:

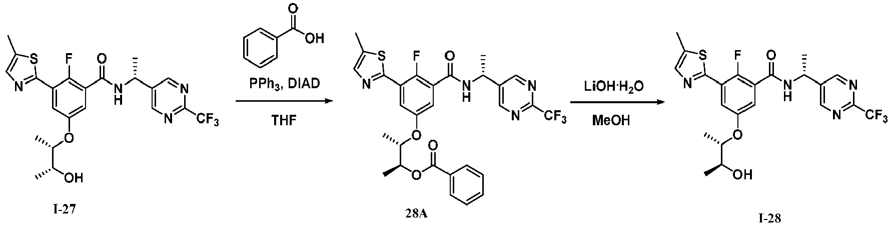

### First step: (2S, 3S)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (28A)

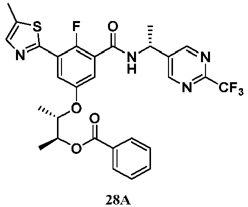

Under nitrogen protection, 2-fluoro-5-(((2S, 3R)-3-hydroxybutan-2-yl)oxy)-3-(5-methylthiazol-2-yl)-N-((R)-1-(2-(trifluoromethyl)pyrimid in-5-yl)ethyl)benzamide (**I-27**) (60 mg, 0.12 mmol), triphenylphosphine (95 mg, 0.361 mmol), benzoic acid (22.1 mg, 0.181mmol), and dried tetrahydrofuran (2 mL) were successively added to a reaction flask. After the temperature of the reaction solution was adjusted to 0 °C to 5 °C, diisopropyl azodicarboxylate (73.0 mg, 0.361 mmol) was slowly added. Then, the reaction solution was heated to room temperature and reacted for 16h, and was then directly concentrated to dryness. The residue was purified with a silica gel column (petroleum ether: ethyl acetate (V/V) =2: 1) to obtain a white solid of (2S, 3S)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (28A) (60 mg, yield 83%).

LC-MS, M/Z: 603.2 [M+H]⁺.

### Second step: synthesis of 2-fluoro-5-(((2S, 3S)-3-hydroxybutan-2-yl)oxy)-3-(5-methylthiazol-2-yl)-N-((R)-1-(2-(trifluoromethyl)pyrimid in-5-yl)ethyl)benzamide (I-28)

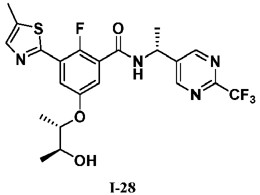

(2S, 3S)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (**28A**) (60 mg, 0.1 mmol) was dissolved in methanol (4 mL), and then lithium hydroxide monohydrate (20 mg, 0.5 mmol) and water (0.4 mL) were added. The mixture was stirred and reacted at room temperature for 16 h. The reaction solution was directly concentrated to dryness. The residue was added with water (5 mL). The aqueous phase was extracted with dichloromethane (5 mL×3). The organic phases were combined, dried over anhydrous sodium sulfate, and concentrated. The residue was purified with a silica gel plate to obtain a white solid of 2-fluoro-5-(((2S, 3S)-3-hydroxybutan-2-yl)oxy)-3-(5-methylthiazol-2-yl)-N-((R)-1-(2-(trifluoromethyl)pyrimid in-5-yl)ethyl)benzamide (**I-28**) (190 mg, yield 71%).

¹H NMR (400 MHz, Chloroform-d) δ 8.95 (s, 2H), 7.88 (dd, J=5.9, 3.3 Hz, 1H), 7.61 (dd, J=2.5, 1.3 Hz, 1H), 7.52 (dd, J=5.8, 3.3 Hz, 1H), 7.09 (dd, J=12.3, 6.4 Hz, 1H), 5.38 (td, J=9.1, 8.5, 2.9 Hz, 1H), 4.24 (p, J=6.2 Hz, 1H), 3.85 (td, J=6.4, 3.2 Hz, 1H), 2.57 (d, J=1.1 Hz, 3H), 2.42 (d, J=3.8 Hz, 1H), 1.73 (d, J=7.1 Hz, 3H), 1.26 (d, J=6.3 Hz, 3H), 1.24 (d, J=6.6 Hz, 3H).

LC-MS, M/Z: 499.1 [M+H]⁺.

### Example 29: preparation of compound I-29

### 2-fluoro-5-(((2R, 3S)-3-hydroxybutan-2-yl)oxy)-3-(5-methylthiazol-2-yl)-N-((R)-1-(2-(trifluoromethyl)pyrimid in-5-yl)ethyl)benzamide (I-29)

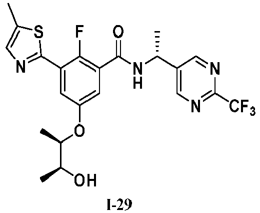

The synthesis scheme for target compound I-29 is shown below:

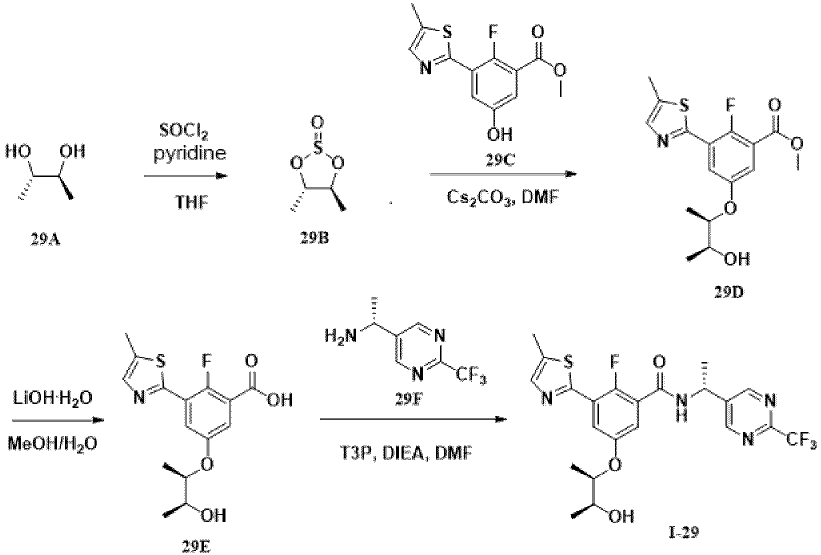

### First step: synthesis of (4S, 5S)-4, 5-dimethyl-1, 3, 2-dioxathiolane 2-oxide (29B)

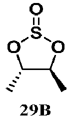

(2S, 3S)-(-)-2, 3-butanediol (1 g, 11.10 mmol) and pyridine (1.93 g, 24.41 mmol) were dissolved in dried tetrahydrofuran (10 mL). After the reaction temperature was adjusted to 0°C to 5 °C, thionyl chloride (1.45 g, 12.21 mmol) was slowly added. Then, the reaction temperature was raised to room temperature, and the mixture was stirred for 16 h. The reaction was quenched by adding water (15 mL), and then 10 mL of ethyl acetate was added, followed by liquid separation. The organic phase was washed with saturated ammonium chloride (10 mL) and brine (10 mL), dried over anhydrous sodium sulfate, and concentrated under reduced pressure, to obtain a colorless liquid of (4S, 5S)-4, 5-dimethyl-1, 3, 2-dioxathiolane 2-oxide (**29B**) (0.8 g, yield 53%).

### Second step: synthesis of methyl 2-fluoro-5-(((2R, 3S)-3-hydroxybutan-2-yl)oxy)-3-(5-methylthiazol-2-yl)benzoate (29D)

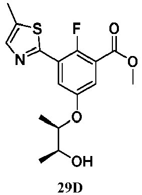

Under nitrogen protection, cesium carbonate (1.22 g, 3.74 mmol) was added to a solution of methyl 2-fluoro-5-hydroxy-3-(5-methylthiazol-2-yl)benzoate (500 mg, 1.871 mmol) (for synthesis thereof, refer to Example 1) in N, N-dimethylformamide (5 mL), and the mixture was stirred at room temperature for 30 min. Then, (4S, 5S)-4, 5-dimethyl-1, 3, 2-dioxathiolane 2-oxide (**29B**) (382 mg, 2.81 mmol) was added, and the mixture was heated to 80 °C and reacted for 16 h. Thereafter, the mixture was cooled to room temperature. The reaction solution was concentrated to dryness under reduced pressure. The residue was added with chloroform (20 mL) and a 4M sulfuric acid solution (20 mL). The mixture solution was stirred at 70 °C for 5h, followed by liquid separation. The aqueous phase was added with sodium bicarbonate to adjust pH at 7 to 8, and extracted with dichloromethane (20 mL×3). The organic phases were combined, dried over anhydrous sodium sulfate, and concentrated. The residue was purified with a silica gel column (petroleum ether: ethyl acetate (V/V) =2: 1) to obtain a white solid of methyl 2-fluoro-5-(((2R, 3S)-3-hydroxybutan-2-yl)oxy)-3-(5-methylthiazol-2-yl)benzoate (29D) (400 mg, yield 63%).

LC-MS, M/Z: 340.1 [M+H]⁺.

### Third step: synthesis of 2-fluoro-5-(((2R, 3S)-3-hydroxybutan-2-yl)oxy)-3-(5-methylthiazol-2-yl)benzoic acid (29E)

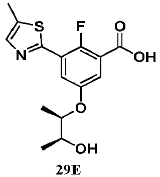

Methyl 2-fluoro-5-(((2R, 3S)-3-hydroxybutan-2-yl)oxy)-3-(5-methylthiazol-2-yl)benzoate (**29D**) (400 mg, 1.18 mmol) was dissolved in methanol (8 mL), and then lithium hydroxide monohydrate (141 mg, 3.54 mmol) and water (0.8 mL) were added. The mixture was stirred and reacted at room temperature for 16 h. The reaction solution was directly concentrated to dryness. The residue was added with water (10 mL), and pH was adjusted to 2 to 3 with a 1M aqueous hydrochloric acid solution. The aqueous phase was extracted with dichloromethane (10 mL×3). The organic phases were combined, dried over anhydrous sodium sulfate, and concentrated to obtain a white solid of 2-fluoro-5-(((2R, 3S)-3-hydroxybutan-2-yl)oxy)-3-(5-methylthiazol-2-yl)benzoic acid (**29E**) (356 mg, yield 93%).

LC-MS, M/Z: 326.1 [M+H]⁺.

### Fourth step: 2-fluoro-5-(((2R, 3S)-3-hydroxybutan-2-yl)oxy)-3-(5-methylthiazol-2-yl)-N-((R)-1-(2-(trifluoromethyl)pyrimid in-5-yl)ethyl)benzamide (I-29)

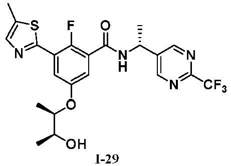

Under nitrogen protection, 2-fluoro-5-(((2R, 3S)-3-hydroxybutan-2-yl)oxy)-3-(5-methylthiazol-2-yl)benzoic acid (**29E**) (356 mg, 1.09 mmol), (R)-1-(2-(trifluoromethyl)pyrimidin-5-yl)ethyl-1-amine hydrochloride (272 mg, 1.42 mmol), N, N-diisopropylethylamine (424 mg, 3.82 mmol), and N, N-dimethylformamide (5 mL) were successively added to a reaction flask. The mixture was cooled to about 0 °C, and added with a solution of propylphosphonic acid anhydride in N, N-dimethylformamide (50%, 1.04 g, 1.64 mmol) dropwise. Thereafter, the mixture was heated to room temperature and reacted for 16 h. The reaction was quenched by adding a saturated solution of sodium bicarbonate (5 mL), and the reaction mixture was extracted with ethyl acetate (10 mL×3). The organic phases were combined, washed with saturated salt water (30 mL×2), dried over anhydrous sodium sulfate, and concentrated. The residue was separated and purified with silica gel plate (petroleum ether: ethyl acetate (V/V) =1: 1) to obtain a white solid of 2-fluoro-5-(((2R, 3S)-3-hydroxybutan-2-yl)oxy)-3-(5-methylthiazol-2-yl)-N-((R)-1-(2-(trifluoromethyl)pyrimid in-5-yl)ethyl)benzamide (**I-29**) (190 mg, yield 34.8%).

¹H NMR (400 MHz, Chloroform-d) δ 8.94 (s, 2H), 7.86 (dd, J=5.9, 3.3 Hz, 1H), 7.63-7.58 (m, 1H), 7.50 (dd, J=5.8, 3.4 Hz, 1H), 7.10 (dd, J=12.3, 6.5 Hz, 1H), 5.38 (ddd, J=7.7, 4.4, 1.5 Hz, 1H), 4.40 (qd, J=6.3, 3.3 Hz, 1H), 4.01 (ddd, J=6.5, 4.8, 3.3 Hz, 1H), 2.56 (d, J=1.2 Hz, 3H), 2.07 (d, J=4.9 Hz, 1H), 1.72 (d, J=7.1 Hz, 3H), 1.26 (d, J=6.3 Hz, 3H), 1.23 (d, J=6.5 Hz, 3H).

LC-MS, M/Z: 499.1 [M+H]⁺.

### Example 30: preparation of compound I-30

### 2-fluoro-5-(((2R, 3R)-3-hydroxybutan-2-yl)oxy)-3-(5-methylthiazol-2-yl)-N-((R)-1-(2-(trifluoromethyl)pyrimid in-5-yl)ethyl)benzamide (I-30)

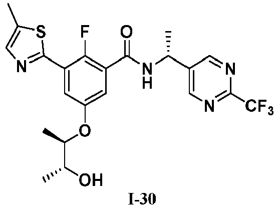

The synthesis scheme for target compound **I-30** is shown below:

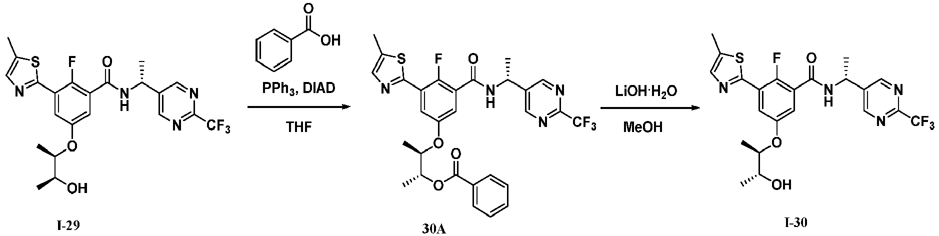

### First step: synthesis of (2R, 3R)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (30A)

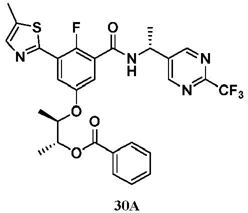

Under nitrogen protection, 2-fluoro-5-(((2R, 3S)-3-hydroxybutan-2-yl)oxy)-3-(5-methylthiazol-2-yl)-N-((R)-1-(2-(trifluoromethyl)pyrimid in-5-yl)ethyl)benzamide (**I-29**) (80 mg, 0.16 mmol), triphenylphosphine(126 mg, 0.481 mmol), benzoic acid (29.4 mg, 0.241 mmol), and dried tetrahydrofuran (2 mL) were successively added to a reaction flask. After the temperature of the reaction solution was adjusted to 0 °C to 5 °C, diisopropyl azodicarboxylate (97.0 mg, 0.481 mmol) was slowly added. Then, the reaction solution was heated to room temperature and reacted for 16 h, and was then directly concentrated to dryness. The residue was purified with a silica gel plate (petroleum ether: ethyl acetate (V/V) =2: 1) to obtain a white solid of (2R, 3R)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (**30A**) (90 mg, yield 93%).

LC-MS, M/Z: 603.2 [M+H]⁺.

### Second step: synthesis of 2-fluoro-5-(((2R, 3R)-3-hydroxybutan-2-yl)oxy)-3-(5-methylthiazol-2-yl)-N-((R)-1-(2-(trifluoromethyl)pyrimid in-5-yl)ethyl)benzamide (I-30)

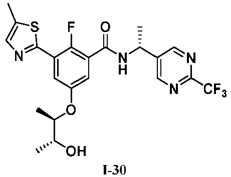

(2R, 3R)-3-(4-fluoro-3-(5-methylthiazol-2-yl)-5-(((R)-1-(2-(trifluoromethyl)pyrimidin-5-yl)ethyl)c arbamoyl)phenoxy)butane-2-benzoate (**30A**) (60 mg, 0.1 mmol) was dissolved in methanol (4 mL), and then lithium hydroxide monohydrate (20 mg, 0.5 mmol) and water (0.4 mL) were added. The mixture was stirred and reacted at room temperature for 16 h. The reaction solution was directly concentrated to dryness. The residue was added with water (5 mL). The aqueous phase was extracted with dichloromethane (5 mL×3). The organic phases were combined, dried over anhydrous sodium sulfate, and concentrated. The residue was purified with a silica gel plate to obtain a white solid of 2-fluoro-5-(((2R, 3R)-3-hydroxybutan-2-yl)oxy)-3-(5-methylthiazol-2-yl)-N-((R)-1-(2-(trifluoromethyl)pyrimid in-5-yl)ethyl)benzamide (**I-30**) (39 mg, yield 79%).

¹H NMR (400 MHz, Chloroform-d) δ 8.95 (s, 2H), 7.88 (dd, J=5.9, 3.3 Hz, 1H), 7.61 (dd, J=2.5, 1.3 Hz, 1H), 7.52 (dd, J=5.8, 3.3 Hz, 1H), 7.09 (dd, J=12.3, 6.4 Hz, 1H), 5.38 (td, J=9.1, 8.5, 2.9 Hz, 1H), 4.24 (p, J=6.2 Hz, 1H), 3.85 (td, J=6.4, 3.2 Hz, 1H), 2.57 (d, J=1.1 Hz, 3H), 2.42 (d, J=3.8 Hz, 1H), 1.73 (d, J=7.1 Hz, 3H), 1.26 (d, J=6.3 Hz, 3H), 1.24 (d, J=6.6 Hz, 3H).

LC-MS, M/Z: 499.1 [M+H]⁺.

### Example 31: preparation of compound I-31

### (R)-2-fluoro-5-(2-hydroxy-2-methylpropoxy)-3-(5-methylthiazol-2-yl)-N-(1-(2-(tri fluoromethyl)pyrimidin-5-yl)ethyl)benzamide (target compound I-31)

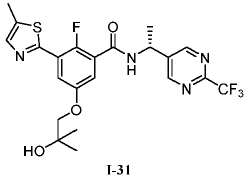

The synthesis scheme for target compound **I-3**1 is shown below:

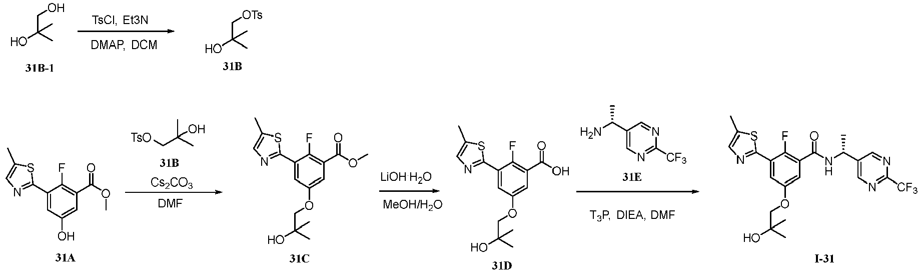

### First step: synthesis of 2-hydroxy-2-methylpropyl 4-methylbenzenesulfonate (31B)

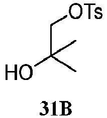

2-methylpropane-1, 2-diol (**31B-1**) (2.0 g, 22.19 mmol), 4-dimethylaminopyridine (0.271 g, 2.22 mmol) and triethylamine (4.49 g, 44.40 mmol) were added to anhydrous dichloromethane (40 mL). At room temperature, p-toluensulfonyl chloride (4.65 g, 24.21 mmol) was added. The mixture reacted at room temperature for 15 h. TLC (petroleum ether: ethyl acetate (V/V) =3: 1) monitored that the reaction of the raw materials was complete. Water (100 mL) and DCM (100 mL) were added to the reaction solution. The aqueous phase was extracted with DCM (100 mL×3). The extracts were combined, washed with saturated salt water (100 mL), dried over sodium sulfate, filtrated, and concentrated under reduced pressure to obtain a crude product. The crude product was separated and purified with silica gel column (petroleum ether: ethyl acetate (V/V) =100: 1 to 1: 1) to obtain a compound 2-hydroxy-2-methylpropyl 4-methylbenzenesulfonate (**31B**) (4.5 g, yield 83 %).

### Second step: synthesis of methyl 2-fluoro-5-(2-hydroxy-2-methylpropoxy)-3-(5-methylthiazol-2-yl)benzoate (31C)

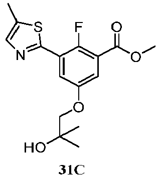

Methyl 2-fluoro-5-hydroxy-3-(5-methylthiazol-2-yl)benzoate (**31A**) (for synthesis thereof, refer to Example 1) (0.2 g, 0.748 mmol) and cesium carbonate (0.366 g, 1.122 mmol) were added to DMF (4 mL), and then 2-hydroxy-2-methylpropyl 4-methylbenzenesulfonate (31B) (0.219 g, 0.898 mmol) was added. The reaction solution reacted at 110 °C for 36 h. TLC (petroleum ether: ethyl acetate (V/V) = 1: 1) monitored that most of the starting materials completely reacted. Water (100 mL) and EA (100 mL) were added to the reaction solution. The aqueous phase was extracted with EA (100 mL×3). The extracts were combined, washed with saturated salt water (100 mL×3), dried over sodium sulfate, filtrated, and concentrated under reduced pressure to obtain a crude product. The crude product was separated and purified with silica gel column (petroleum ether: ethyl acetate (V/V) =30: 1 to 1: 100) to obtain a compound methyl 2-fluoro-5-(2-hydroxy-2-methylpropoxy)-3-(5-methylthiazol-2-yl)benzoate (**31C**) (0.1 g, yield 39 %).

### Third step: synthesis of 2-fluoro-5-(2-hydroxy-2-methylpropoxy)-3-(5-methylthiazol-2-yl)benzoic acid (31D)

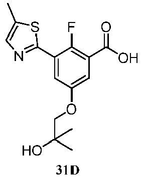

Methyl 2-fluoro-5-(2-hydroxy-2-methylpropoxy)-3-(5-methylthiazol-2-yl)benzoate (**31C**) (0.1 g, 0.295 mmol) was added to methanol (3 mL) and water (0.5 mL). At room temperature, lithium hydroxide monohydrate (0.049 g, 1.179 mmol) was added. The reaction solution reacted at room temperature for 15 hours. TLC (petroleum ether: ethyl acetate (V/V) = 1: 1) monitored that the reaction of the raw materials was complete. The reaction solution was concentrated to dryness under reduced pressure. The crude product was added with water (50 mL) and EA (100 mL), and pH was adjusted to 7 with 1N hydrochloric acid. The solution was extracted with ethyl acetate (100 mL×2). The organic phases were combined, washed with saturated salt water (50 mL), dried over sodium sulfate, and concentrated, to obtain 2-fluoro-5-(2-hydroxy-2-methylpropoxy)-3-(5-methylthiazol-2-yl)benzoic acid (31D) (50 mg, yield 52.2 %).

### Fourth step: synthesis of (R)-2-fluoro-5-(2-hydroxy-2-methylpropoxy)-3-(5-methylthiazol-2-yl)-N-(1-(2-(trifluorometh yl)pyrimidin-5-yl)ethyl)benzamide (target compound I-31)

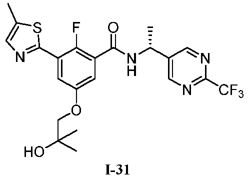

2-fluoro-5-(2-hydroxy-2-methylpropoxy)-3-(5-methylthiazol-2-yl)benzoic acid (31D) (0.05 g, 0.154 mmol), a solution of propylphosphonic acid anhydride in N, N-dimethylformamide (50%, 0.147 g, 0.231 mmol), and DIPEA (0.079 g, 0.615 mmol) were added to DMF (2 mL). The mixture was stirred and reacted at room temperature for 0.5 h, and then added with (R)-1-(2-(trifluoromethyl)pyrimidin-5-yl)ethyl-1-amine hydrochloride (31E) (0.035 g, 0.184 mmol). The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), dried over sodium sulfate, filtrated, and concentrated under reduced pressure to obtain a crude product. The crude product was separated and purified by a silica gel plate (petroleum ether: ethyl acetate (V/V) = 1: 1), to obtain a white solid of (R)-2-fluoro-5-(2-hydroxy-2-methylpropoxy)-3-(5-methylthiazol-2-yl)-N-(1-(2-(trifluorometh yl)pyrimidin-5-yl)ethyl)benzamide (target compound **I-31**) (7 mg, yield 9 %).

¹H NMR (400 MHz, DMSO) δ 9.20 (d, J=7.0 Hz, 1H), 9.11 (s, 2H), 7.78-7.69 (m, 2H), 7.22-7.19 (m, 1H), 5.27 (m, 1H), 4.64 (s, 1H), 3.78 (s, 2H), 2.51 (s, 3H), 1.55 (d, 7=7.0 Hz, 3H), 1.19 (s, 6H).

LC-MS, M/Z: 499.10 [M+H]⁺.

### Example 32: preparation of compound I-32

### (R)-2-fluoro-5-((1-hydroxycyclopropyl)methoxy)-3-(5-methylthiazol-2-yl)-N-(1-(2-(trifluoro methyl)pyrimidin-5-yl)ethyl)benzamide (compound I-32)

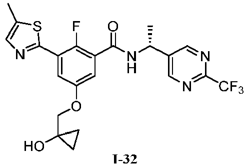

For the synthesis of compound **I-32**, refer to Example 31.

LC-MS, M/Z: 497.1 [M+H]⁺.

### Example 33: preparation of compound I-33

### (R)-2-fluoro-5-(1-(hydroxymethyl)cyclopropoxy)-3-(5-methylthiazol-2-yl)-N-(1-(2-(trifluoro methyl)pyrimidin-5-yl)ethyl)benzamide (I-33)

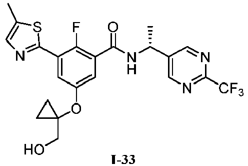

For the synthesis of compound **I-33**, refer to Example 31.

LC-MS, M/Z: 497.1 [M+H]⁺.

### Example 34: preparation of target compound I-34

### (R)-2-fluoro-5-((1-hydroxy-2-methylpropan-2-yl)oxy)-3-(5-methylthiazol-2-yl)-N-(1-(2-(trifl uoromethyl) pyrimidin-5-yl)ethyl)benzamide (I-34)

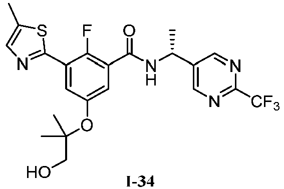

The synthesis scheme for compound **I-34** is shown below:

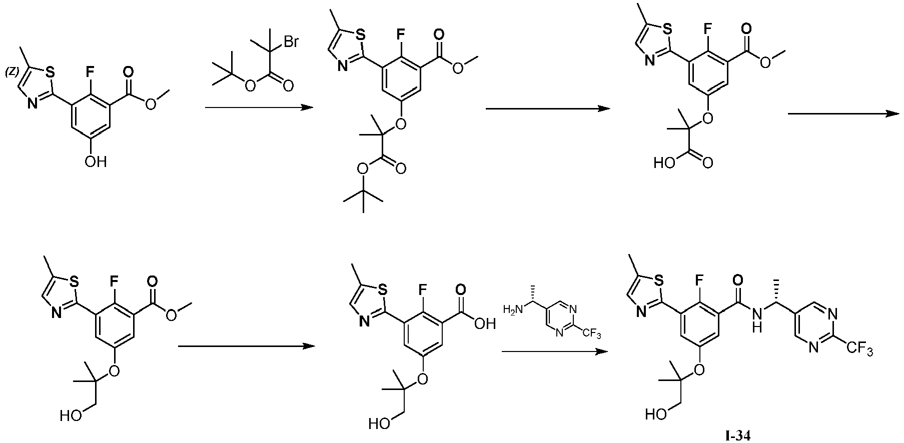

### First step: synthesis of methyl 5-((1-(tert-butoxy)-2-methyl-1-oxopropan-2-yl)oxy)-2-fluoro-3-(5-methylthiazol-2-yl)benzoat e

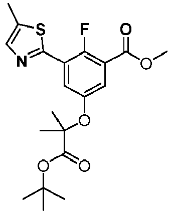

Methyl 2-fluoro-5-hydroxy-3-(5-methylthiazol-2-yl)benzoate (0.30 g, 1.122 mmol) and cesium carbonate (0.73 g, 2.245 mmol) was added to anhydrous DMF (5 mL). At room temperature, tert-butyl 2-bromo-2-methylpropionate (0.38 g, 1.684 mmol) was added. The mixture reacted at room temperature for 15 h. TLC (petroleum ether: ethyl acetate (V/V) = 2: 1) monitored that the reaction of the raw materials was complete. Water (100 mL) and EA (100 mL) were added to the reaction solution. The aqueous phase was extracted with EA (100 mL×3). The extracts were combined, washed with saturated salt water (100 mL), and dried over sodium sulfate, followed by filtration and rotatory evaporation, to obtain a crude product. The crude product was separated and purified with silica gel column (petroleum ether: ethyl acetate (V/V) =100: 1 to 1: 1) to obtain a compound of methyl 5-((1-(tert-butoxy)-2-methyl-1-oxopropan-2-yl)oxy)-2-fluoro-3-(5-methylthiazol-2-yl)benzoat e (0.3 g, yield 65.3 %).

### Second step: synthesis of 2-(4-fluoro-3-(methoxycarbonyl)-5-(5-methylthiazol-2-yl)phenoxy)-2-methylpropionic acid

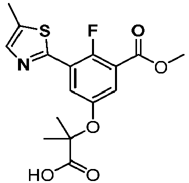

Methyl 5-((1-(tert-butoxy)-2-methyl-1-oxopropan-2-yl)oxy)-2-fluoro-3-(5-methylthiazol-2-yl)benzoat e (0.3 g, 0.733 mmol) was added to anhydrous DCM (5 mL), and CF₃COOH (1.0 mL) was added dropwise at 0°C. The reaction solution reacted at 0°C for 15h. TLC (petroleum ether: ethyl acetate (V/V) = 2: 1) monitored that most of the raw materials completely reacted. Water (100 mL) and DCM (100 mL) were added to the reaction solution. The aqueous phase was extracted with DCM (100 mL×3). The extracts were combined, washed with saturated salt water (100 mL ×3), and dried over sodium sulfate, followed by filtration and rotatory evaporation, to obtain a crude product. The crude product was separated and purified with silica gel column (petroleum ether: ethyl acetate (V/V) =30: 1 to 1: 100) to obtain a compound 2-(4-fluoro-3-(methoxycarbonyl)-5-(5-methylthiazol-2-yl)phenoxy)-2-methylpropionic acid (3) (0.25 g, yield 97.0 %).

### Third step: synthesis of methyl 2-fluoro-5-((1-hydroxy-2-methylpropan-2-yl)oxy)-3-(5-methylthiazol-2-yl)benzoate

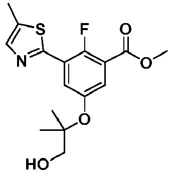

2-(4-fluoro-3-(methoxycarbonyl)-5-(5-methylthiazol-2-yl)phenoxy)-2-methylpropi onic acid (0.25 g, 0.707 mmol) was added to anhydrous THF (5 mL), and then BH3/THF (2.1 mL, 2.212mol, 1 mol/L) was added. The mixture reacted at room temperature for 15 h. TLC (petroleum ether: ethyl acetate (V/V) = 1: 1) monitored that the reaction of the raw materials was complete. The reaction solution was dried through rotatory evaporation to obtain a compound methyl 2-fluoro-5-((1-hydroxy-2-methylpropan-2-yl)oxy)-3-(5-methylthiazol-2-yl)benzoate (0.08 g, yield 33.3 %).

### Fourth step: synthesis of 2-fluoro-5-((1-hydroxy-2-methylpropan-2-yl)oxy)-3-(5-methylthiazol-2-yl)benzoic acid

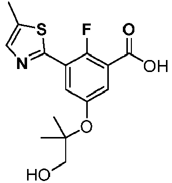

Methyl 2-fluoro-5-((1-hydroxy-2-methylpropan-2-yl)oxy)-3-(5-methylthiazol-2-yl)benzoate (0.08 g, 0.236 mmol) was added to MeOH (4 mL), H₂O (0.5 mL) and THF (0.5 mL). At room temperature, LiOH·H₂O (0.023 g, 0.943 mmol) was added. The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), dried over sodium sulfate, filtrated, and dried through rotatory evaporation, to obtain a compound 2-fluoro-5-((1-hydroxy-2-methylpropan-2-yl)oxy)-3-(5-methylthiazol-2-yl)benzoic acid (5) (0.06 g, yield 78.0 %).

### Fifth step: synthesis of (R)-2-fluoro-5-((1-hydroxy-2-methylpropan-2-yl)oxy)-3-(5-methylthiazol-2-yl)-N-(1-(2-(trifl uoromethyl)) pyrimidin-5-yl)ethyl)benzamide (I-34)

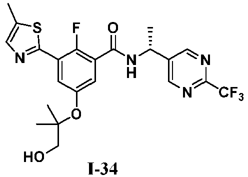

2-fluoro-5-((1-hydroxy-2-methylpropan-2-yl)oxy)-3-(5-methylthiazol-2-yl)benzoi c acid (5) (0.06 g, 0.184 mmol), T3P (0.21 g, 0.277 mmol 50% in DMF), and DIPEA (0.095 g, 0.738 mmol) were added to DMF (6 mL). The mixture was stirred and reacted at room temperature for 0.5 h, and then added with (R)-1-(2-(trifluoromethyl)pyrimidin-5-yl)ethyl-1-amine (0.042 g, 0.221 mmol). The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified through Pre-TLC (petroleum ether: ethyl acetate (V/V) = 1: 1), to obtain a white solid of (R)-2-fluoro-5-((1-hydroxy-2-methylpropan-2-yl)oxy)-3-(5-methylthiazol-2-yl)-N-(1-(2-(trifl uoromethyl)) pyrimidin-5-yl)ethyl)benzamide (**I-34**) (2.5 mg, yield 2.72 %).

¹H NMR (400 MHz, DMSO) δ 9.19 (d, *J* = 6.8 Hz, 1H), 9.09 (s, 2H), 7.89 - 7.83 (m, 1H), 7.71 (s, 1H), 7.25 (s, 1H), 5.51 (d, *J* = 12.4 Hz, 1H), 5.34 - 5.18 (m, 2H), 4.99 (s, 1H), 2.51 (s, 3H), 1.55 (d, *J* = 7.0 Hz, 3H), 1.20 (s, 6H).

LC-MS, M/Z: 499.1 [M+H]⁺.

### Example 35: preparation of target compound I-35

### 2-fluoro-5-((1-hydroxypropan-2-yl)oxy)-3-(5-methylthiazol-2-yl)-N-((R)-1-(2-(trif luoromethyl)pyrimidin-5-yl)ethyl)benzamide (I-35)

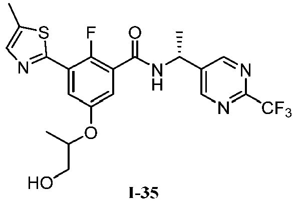

The synthesis scheme for compound **I-35** is shown below:

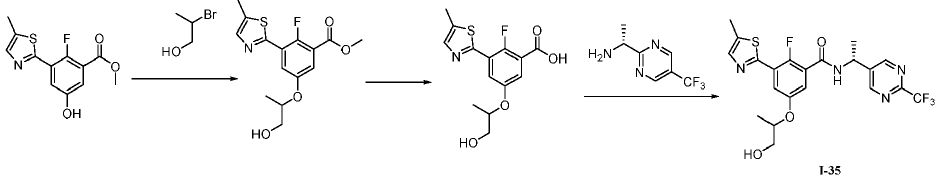

### First step: synthesis of methyl 2-fluoro-5-((1-hydroxypropan-2-yl)oxy)-3-(5-methylthiazol-2-yl)benzoate

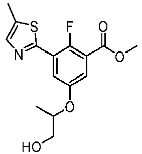

Methyl 2-fluoro-5-hydroxy-3-(5-methylthiazol-2-yl)benzoate (0.15 g, 0.561 mmol) and cesium carbonate (0.366 g, 1.122 mmol) were added to anhydrous DMF (2 mL). At room temperature, 2-bromopropan-1-ol (0.12 g, 0.842 mmol) was added and the mixture reacted at 110°C for 15h. TLC (petroleum ether: ethyl acetate (V/V) = 2: 1) monitored that the reaction of the raw materials was complete. Water (100 mL) and EA (100 mL) were added to the reaction solution. The aqueous phase was extracted with EA (100 mL×3). The extracts were combined, washed with saturated salt water (100 mL), and dried over sodium sulfate, followed by filtration and rotatory evaporation, to obtain a crude product. The crude product was separated and purified with silica gel column (petroleum ether: ethyl acetate (V/V) =100: 1 to 1: 1), to obtain a compound methyl 2-fluoro-5-((1-hydroxypropan-2-yl)oxy)-3-(5-methylthiazol-2-yl)benzoate (0.15 g, yield 82 %).

### Second step: synthesis of 2-fluoro-5-((1-hydroxypropan-2-yl)oxy)-3-(5-methylthiazol-2-yl)benzoic acid

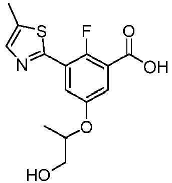

Methyl 2-fluoro-5-((1-hydroxypropan-2-yl)oxy)-3-(5-methylthiazol-2-yl)benzoate (0.15 g, 0.461 mmol) was added to MeOH (4 mL), H₂O (0.5 mL), and THF (0.5 mL). At room temperature, LiOH·H₂O (0.044 g, 1.844 mmol) was added. The reaction solution reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL), dried over sodium sulfate, filtrated, and dried through rotatory evaporation, to obtain a compound 2-fluoro-5-((1-hydroxypropan-2-yl)oxy)-3-(5-methylthiazol-2-yl)benzoic acid (0.10 g, yield 69.7 %).

### Third step: synthesis of 2-fluoro-5-((1-hydroxypropan-2-yl)oxy)-3-(5-methylthiazol-2-yl)-N-((R)-1-(2-(trifluorometh yl)pyrimidin-5-yl)ethyl)benzamide (I-35)

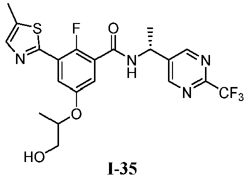

2-fluoro-5-((1-hydroxypropan-2-yl)oxy)-3-(5-methylthiazol-2-yl)benzoic acid (0.10 g, 0.321 mmol), T3P (0.366 g, 0.482 mmol 50% in DMF), and DIPEA (0.166 g, 1.285 mmol) were added to DMF (5 mL). The mixture was stirred and reacted at room temperature for 0.5h, and then added with (R)-1-(5-(trifluoromethyl)pyrimidin-2-yl)ethyl-1-amine (0.061 g, 0.321 mmol). The mixture reacted at room temperature for 15 h. TLC (petroleum ether: ethyl acetate (V/V) = 1: 1) monitored that the reaction of the raw materials was complete. Water (50 mL) and ethyl acetate (100 mL) were added to the reaction solution. The aqueous phase was extracted with ethyl acetate (100 mL×3). The extracts were combined, washed with saturated salt water (50 mL×3), and dried over sodium sulfate, followed by filtration and rotatory evaporation, to obtain a crude product. The crude product was separated and purified through Pre-TLC (petroleum ether: ethyl acetate (V/V) = 1: 1), to obtain a white solid of 2-fluoro-5-((1-hydroxypropan-2-yl)oxy)-3-(5-methylthiazol-2-yl)-N-((R)-1-(2-(trifluorometh yl)pyrimidin-5-yl)ethyl)benzamide (**I-35**) (6.0 mg, yield 3.86 %).

¹H NMR (400 MHz, DMSO) δ 9.20 (d, *J* = 7.0 Hz, 1H), 9.09 (d, *J* = 6.8 Hz, 2H), 7.74 (d, *J* = 5.4 Hz, 1H), 7.71 (s, 1H), 7.21 - 7.17 (m, 1H), 5.26 (d, *J* = 7.0 Hz, 1H), 4.90 (s, 1H), 3.94 (s, 1H), 3.87 (d, *J* = 5.2 Hz, 2H), 2.51 (s, 3H), 1.55 (d, *J* = 7.0 Hz, 3H), 1.14 (d, J = 6.2 Hz, 3H).

LC-MS, M/Z: 485.1 [M+H]⁺.

### Example 36: preparation of target compound I-36

### 2-fluoro-5-(2-hydroxypropoxy)-3-(5-methylthiazol-2-yl)-N-((R)-1-(2-(trifluoromet hyl)pyrimidin-5-yl)ethyl)benzamide (I-36)

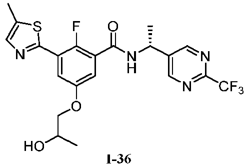

For the synthesis of compound **I-36**, refer to Example 35.

LC-MS, M/Z: 485.1 [M+H]⁺.

### Example 37: preparation of target compound I-37

### 2-fluoro-5-(((3R, 5R)-5-hydroxytetrahydrofuran-3-yl)oxy)-3-(5-methylthiazol-2-yl)-N-((R)-1-(2-(trifluorometh yl)pyrimidin-5-yl)ethyl)benzamide (I-37) and 2-fluoro-5-(((3R, SS)-5-hydroxytetrahydrofuran-3-yl)oxy)-3-(5-methylthiazol-2-yl)-N-((R)-1-(2-(trifluorometh yl)pyrimidin-5-yl)ethyl)benzamide (I-38)

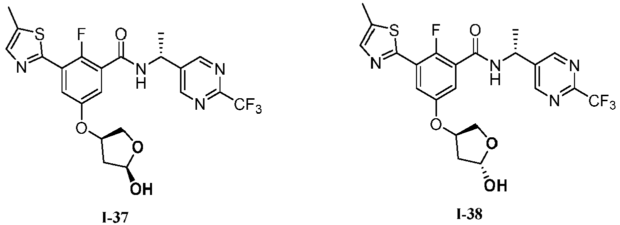

The synthesis scheme for compound **I-37** and **I-38** is shown below:

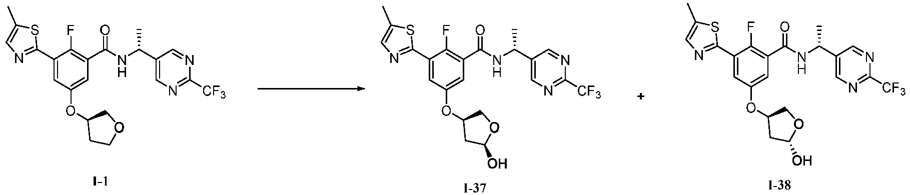

2-fluoro-3-(5-methylthiazol-2-yl)-5-(((R)-tetrahydrofuran-3-yl)oxy)-N-((R)-1-(2-(t rifluoromethyl)pyrimidin-5-yl)ethyl)benzamide (**I-1**) (1.5g, 3 mmol) and ferric trichloride (0.245g, 1.5 mmol) were placed in a round-bottom flask. Under nitrogen protection, pyridine (15 mL) was added, and then tert-butyl hydroperoxide (1.56 g, 12.1 mmol, 70% aqueous solution) was slowly added. The mixture was stirred at room temperature for 48 hours, and a saturated solution of EDTA monosodium salt (100 ml) was added and stirred for 10 min, followed by adding 100 mL of brine. The aqueous phase was extracted with DCM. The organic phase was collected, dried over anhydrous sodium sulfate, and concentrated. The residue was purified through preparative chromatography to obtain a light-yellow solid of 2-fluoro-5-(((3R, 5R)-5-hydroxytetrahydrofuran-3-yl)oxy)-3-(5-methylthiazol-2-yl)-N-((R)-1-(2-(trifluorometh yl)pyrimidin-5-yl)ethyl)benzamide (**I-37**) and 2-fluoro-5-(((3R, SS)-5-hydroxytetrahydrofuran-3-yl)oxy)-3-(5-methylthiazol-2-yl)-N-((R)-1-(2-(trifluorometh yl)pyrimidin-5-yl)ethyl)benzamide (**I-38**) (2 mg, yield 0.13%).

¹H NMR (400 MHz, DMSO-d6) δ 9.20 (d, J = 7.0 Hz, 1H), 9.13 - 9.07 (m, 2H), 7.90 - 7.66 (m, 2H), 7.21 (dd, J = 5.0, 3.4 Hz, 1H), 6.47 (d, J = 4.5 Hz, 1H), 5.40 - 5.10 (m, 2H), 4.73 (d, J = 4.7 Hz, 1H), 4.09 - 3.65 (m, 2H), 2.51 (s, 3H), 2.41 - 2.29 (m, 1H), 2.07-1.87 (m, 1H), 1.54 (t, J = 6.3 Hz, 3H).

LC-MS, M/Z: 513.1 [M+H]⁺.

### Example 38: preparation of target compound I-39

### 2-fluoro-3-(5-methylthiazol-2-yl)-5-(((S)-3-oxobutan-2-yl)oxy)-N-((R)-1-(2-(triflu oromethyl))pyrimidin-5-yl)ethyl)benzamide (target compound I-39)

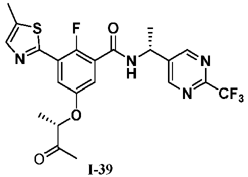

The synthesis scheme for compound **I-39** is shown below:

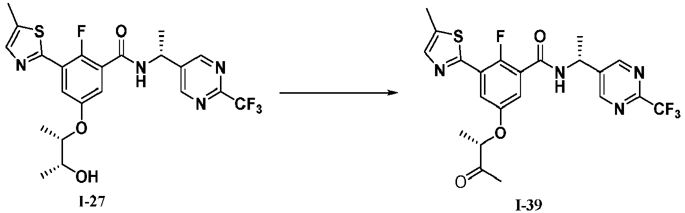

2-fluoro-5-(((2S, 3R)-3-hydroxybutan-2-yl)oxy)-3-(5-methylthiazol-2-yl)-N-((R)-1-(2-(trifluoromethyl)pyrimid in-5-yl)ethyl)benzamide (**I-27**) (200 mg, 0.4 mmol) was placed in a round-bottom flask. Under nitrogen protection, Dess-Martin reagent (340 mg, 0.8 mmol) and DCM (5 ml) were added. The mixture was stirred at room temperature for 3 hours. The reaction was monitored by TLC. After the raw materials had completely reacted, the reaction solution was diluted by adding ethyl acetate (5 ml) and water (5 ml), to perform extraction and collect the organic phase. The organic phase was dried over anhydrous sodium sulfate. The residue was separated and purified with silica gel column (petroleum ether: ethyl acetate (V/V) =20: 1 to 5: 1), to obtain a white solid of 2-fluoro-3-(5-methylthiazol-2-yl)-5-(((S)-3-oxobutan-2-yl)oxy)-N-((R)-1-(2-(trifluoromethyl) )pyrimidin-5-yl)ethyl)benzamide (**I-39**) (120 mg, yield 60%).
1H NMR (400 MHz, CDCl3) δ 8.87 (s, 2H), 7.84 - 7.71 (m, 1H), 7.53 (s, 1H), 7.45 - 7.36 (m, 1H), 7.03 (dd, J = 11.9, 6.5 Hz, 1H), 5.75 - 5.13 (m, 1H), 4.67 (q, J = 6.8 Hz, 1H), 2.49 (s, 3H), 2.15 (s, 3H), 1.81 - 1.57 (m, 3H), 1.44 (d, J = 6.8 Hz, 3H).
LC-MS, M/Z (ESI): 497.5 [M+H]+

### Example 39: preparation of target compound I-40

### (R)-5-((2-oxabicyclo[2.1.1]hexan-1-yl)methoxy)-2-fluoro-3-(5-methylthiazol-2-yl) -N-(1-(2-(trifluoromethyl)pyrimidin-5-yl)ethyl)benzamide (target compound I-40)

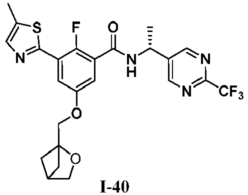

The synthesis scheme for compound **I-40** is shown below:

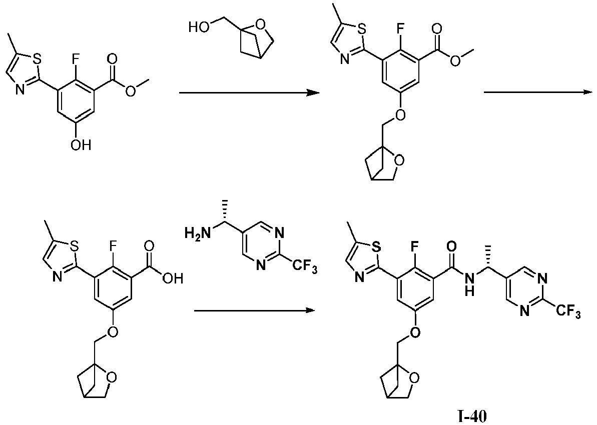

### First step: synthesis of methyl 5-((2-oxabicyclo[2.1.1]hexan-1-yl)methoxy)-2-fluoro-3-(5-methylthiazol-2-yl)benzoate (3)

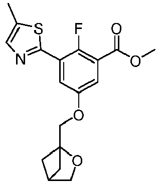

Methyl 2-fluoro-5-hydroxy-3-(5-methylthiazol-2-yl)benzoate (1) (100 mg, 0.374 mmol) was added in a round-bottom flask, and then triphenylphosphine(294 mg, 1.12 mmol) and a compound (64.1 mg, 0.56 mmol) were added. Under nitrogen protection, DIAD (227 mg, 1.12 mmol) and THF (2 ml) were added. The mixture reacted at room temperature for 16 hours. The reaction was monitored by TLC. When the raw materials completely reacted, the reaction was quenched by adding water. Then, the reaction solution was diluted with ethyl acetate (5 ml). Extraction was performed, and the organic phase was collected and dried over anhydrous sodium sulfate. The residue was separated and purified with silica gel column (petroleum ether: ethyl acetate (V/V) =20: 1 to 5: 1), to obtain a colorless oily liquid of methyl 5-((2-oxabicyclo[2.1.1]hexan-1-yl)methoxy)-2-fluoro-3-(5-methylthiazol-2-yl)benzoate (120 mg, yield 88%).

### Second step: synthesis of 5-((2-oxazolylcyclo[2.1.1]hexan-1-yl)methoxy)-2-fluoro-3-(5-methylthiazol-2-yl)benzoic acid

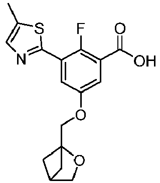

Methyl 5-((2-oxabicyclo[2.1.1]hexan-1-yl)methoxy)-2-fluoro-3-(5-methylthiazol-2-yl)benzoate (3) (140 mg, 0.385 mmol) and lithium hydroxide monohydrate (64.6 mg, 1.54 mmol) were placed in a round-bottom flask. Then, methanol (3 ml) and water (0.3 ml) were added. The mixture was stirred at room temperature for 3 hours. The reaction was monitored by TLC. After the raw materials had completely reacted, methanol was removed, and the reaction solution was added with 4M hydrochloric acid at 0°C to adjust pH to 3. A large amount of white solid was precipitated, and stirred for 30 min. The solid was collected through suction filtration, and dried in a vacuum drying oven, to obtain white solid of 5-((2-oxazolylcyclo[2. 1. 1]hexan-1-yl)methoxy)-2-fluoro-3-(5-methylthiazol-2-yl)benzoic acid (**4**) (92 mg, yield 68.1%).

### Third step: synthesis of (R)-5-((2-oxabicyclo[2.1.1]hexan-1-yl)methoxy)-2-fluoro-3-(5-methylthiazol-2-yl)-N-(1-(2-(t rifluoromethyl)pyrimidin-5-yl)ethyl)benzamide

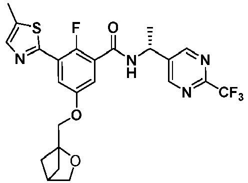

5-((2-oxazolylcyclo[2.1.1]hexan-1-yl)methoxy)-2-fluoro-3-(5-methylthiazol-2-yl) benzoic acid (4) (92 mg, 0.263mmol) was placed in a round-bottom flask, and HOBT (42.7 mg, 0.316 mmol), EDCI (60.6 mg, 0.316 mmol) and a compound (76 mg, 118 mmol) were added. Then, DMF (3 ml) was added, and DIPEA (102 mg, 0.79 mmol) was slowly added dropwise at 0 °C. After the dropwise addition, the flask was transferred to room temperature environment and reacted overnight. The reaction was monitored by TLC. After the raw materials had been completely consumed, the reaction solution was diluted by adding ethyl acetate (5 ml) and added with saturated sodium bicarbonate (5 ml), to perform extraction and collect the organic phase. The organic phase was washed once with saturated sodium bicarbonate, twice with diluted hydrochloric acid aqueous solution, and once with saturated sodium chloride aqueous solution. The organic phase was collected, and dried over anhydrous sodium sulfate. The residue was separated and purified with silica gel column (100% ethyl acetate) to obtain a light-yellow solid of (R)-5-((2-oxabicyclo[2.1.1]hexan-1-yl)methoxy)-2-fluoro-3-(5-methylthiazol-2-yl)-N-(1-(2-(t rifluoromethyl)pyrimidin-5-yl)ethyl)benzamide (70 mg, yield 50.7%).

1H NMR (400 MHz, DMSO-d6) δ 9.19 (d, J = 7.1 Hz, 1H), 9.10 (s, 2H), 7.77-7.73 (m, 1H), 7.72- 7.69 (m, 1H), 7.25- 7.21 (m, 1H), 5.29 - 5.22 (m, 1H), 4.30 (s, 2H), 3.69 (s, 2H), 2.92 (t, J = 3.1 Hz, 1H), 2.51 (s, 3H), 1.85- 1.81 (m, 2H), 1.54 (d, J = 7.1 Hz, 3H), 1.43 (dd, J = 4.4, 1.6 Hz, 2H).

LC-MS, M/Z (ESI): 523.5 [M+H]⁺.

### Test Example 1: Determination of antagonistic activity of human P2X3 (hP2X3) antagonists against hP2X3 receptor by means of FLIPR

The antagonistic activity of hP2X3 antagonists against hP2X3 receptor was evaluated by means of FLIPR Calcium 4 Assay Kit (Molecular Devices, R8141) and FLIPR TETRA instrument (Molecular Devices, 0296) by detecting calcium flow signals. 24h before the experiment, human cells, which had been stably transfected with hP2X3 receptors, were plated on a 384-well plate at 2×10⁵ cells/mL, with 50 µL of cell suspension per well, and the plate was cultured in an incubator (37°C, 5% CO₂) for 16 h to 24 h. The test compound solution (20 to 50 mM DMSO stock solution) with a concentration of 180 times the required concentration was prepared using DMSO. The stock solution was added to the 384-well plate at 500nL per well, supplemented with 30 µL of FLIPR Assay buffer (1 ×HBSS+2mM CaCl2 20mM HEPES containing 1.26mM Ca²⁺). The 384-well plate was shaken for 20 to 40 min to mix evenly. An agonist (α, β-meATP) with a concentration of 3 times the required concentration (required final concentration was 400nM) was prepared with FLIPR Assay buffer, and was added to another 384-well plate at 45 µL per well. The culture plate, on which the cells were plated one day ago, was taken, the cell supernatant was sucked and discarded, each well was added with 30 µL of Dye (FLIPR^{®} Calcium 4 Assay Kit, diluted with FLIPR buffer), and then the plate was incubated for 1h. 15 µL of compound was added to each well of cells (using FLIPR sample injector). After 15 minutes, 22.5 µL of the agonist was added to each well, and the fluorescence signal (exciting light wavelength ranging from 470 nm to 495 nm, and emission wavelength ranging from 515 nm to 575 nm) was detect. The difference between signal peak value and valley value was taken as the basic data; the highest concentration data of positive drug was taken as the 100% inhibition rate; and DMSO data was regarded as the 0% inhibition rate. An inhibition effect curve of the compound was fitted on the software GraphpadPrism6, and the IC₅₀ value was calculated.

**[Table 1] Antagonistic activity of test compound against hP2X3 receptor**

| Test compounds | hP2X3 IC₅₀ (nM) |
|---|---|
| Reference compound 1 | 241 |
| I-1 | 83 |
| I-2 | 298 |
| I-3 | 168 |
| I-4 | 63 |
| I-5 | 50 |
| I-7 | 271 |
| I-9 | 305 |
| I-14 | 99 |
| I-15 | 1357 |
| I-16 | 433 |
| I-17 | 388 |
| I-18 | 448 |
| I-19 | 310 |
| I-20 | 373 |
| I-27 | 67 |
| I-28 | 63 |
| I-29 | 57 |
| I-30 | 48 |

The test results indicate that the compounds I-1, I-4, I-5, I-14, I-27, I-28, I-29 and I-30 according to the present disclosure have better antagonistic activity against hP2X3 receptor than Reference compound 1.

### Test Example 2: Pharmacokinetic assay in rat and mouse

For a rat pharmacokinetic assay, male SD rats (180 g to 240 g) were selected and fasted overnight. Three rats were intragastrically administered with 10 mg/kg of the corresponding compound. The blood of the rats was collected before the administration and at 15 min, 30 min, 1 h, 2 h, 4 h, 8 h, and 24 h after the administration. The blood samples were centrifuged at 4 °C for 6 minutes at 8,000 r/min, and plasma was collected and stored at -20 °C. Plasma at each time point was taken, and added with 3 to 5 times the amount of an internal standard-containing acetonitrile solution to mix, and the mixture was vortex mixed for 1 minute, and centrifuged at 4 °C for 10 minutes at 13,000 r/min. The supernatant was taken and mixed with 3 times the amount of water, and an appropriate amount of the mixed solution was taken for LC-MS/MS analysis. The primary pharmacokinetic parameters were analyzed by WinNonlin 7.0 software non-atrioventricular model.

For a mouse pharmacokinetic assay, male ICR mice (20 g to 25 g) were selected and fasted overnight. Three mice were intragastrically administered with 10 mg/kg of the corresponding compound. The blood of the mice was collected before the administration and at 15 min, 30 min, 1 h, 2 h, 4 h, 8 h, and 24 h after the administration. The blood samples were centrifuged at 4 °C for 6 minutes at 6,800 g, plasma was collected and stored at -80 °C. Plasma at each time point was taken, and added with 3-5 times the amount of internal standard-containing acetonitrile to mix, and the mixture was vortex mixed for 1 minute, and centrifuged at 4 °C for 10 minutes at 13,000 r/min. The supernatant was mixed with 3 times the amount of water, and an appropriate amount of the mixed solution was taken for LC-MS/MS analysis. The primary pharmacokinetic parameters were analyzed by WinNonlin 7.0 software non-atrioventricular model.

**[Table 2] Pharmacokinetics of test compounds in rat**

| Test compounds | Pharmacokinetic parameters in rat (intragastric administration) | | | |
|---|---|---|---|---|
| | Cmax (ng/mL) | Tmax (hr) | AUC0-t h*ng/mL) | T1/2 (hr) |
| Reference compound 1 | 417.4 | 2.00 | 3325.3 | 4.01 |
| I-1 | 569.4 | 4.00 | 5937.4 | 3.61 |

The test results indicate that in the rat model, the pharmacokinetic properties of the compound I-1 according to the present disclosure are improved to certain extent over the reference compound 1.

**[Table 3] Pharmacokinetics of test compounds in mouse**

| Test compounds | Pharmacokinetic parameters in mouse (intragastric administration) | | | |
|---|---|---|---|---|
| | Cmax (ng/mL) | Tmax (hr) | AUC0-t (h*ng/mL) | T1/2 (hr) |
| Reference compound 1 | 1246.6 | 0.50 | 5201.3 | 2.41 |
| Reference compound 2 | 845.3 | 0.50 | 3186.1 | 2.66 |
| I-1 | 1546.7 | 2.00 | 12084.6 | 2.30 |
| I-27 | 3377.5 | 0.50 | 14974.5 | 2.89 |
| I-28 | 2400.2 | 0.50 | 8761.3 | 2.65 |
| I-29 | 4098.9 | 1.00 | 14889.1 | 2.23 |
| I-30 | 2945.5 | 1.00 | 13784.5 | 2.35 |

The results of the mouse assay indicate that the compounds I-1, I-27, I-28, I-29 and I-30 according to the present disclosure have significantly improved pharmacokinetic properties over Reference compound 1 and Reference compound 2

### Test Example 3: Taste Test in rat

SD rats were administered with the corresponding compound after 3 days of overnight water prohibition training. Each rat was supplied with 1 bottle of water and 1 bottle of 0.3 mM aqueous quinine solution half an hour after the administration. After 15 min of water supply, the bottles were removed. The intake amount of water and the intake amount of the 0.3 mM aqueous quinine solution were measured, respectively. The taste influence of the compound on SD rats was evaluated based on the difference between the intake amount of water and the intake amount of the aqueous quinine solution.

**[Table 4] Ratio of water intake to aqueous quinine solution intake of rats administrated with the corresponding compounds**

| Compounds | Water/aqueous quinine solution |
|---|---|
| Solvent | 34.7 |
| Reference compound 1 (30mg/kg i.p) | 11.4 |
| I-1 (30mg/kg i.p) | 18.2 |
| I-27 (30mg/kg i.p) | 29.4 |
| I-28 (30mg/kg i.p) | 15.4 |
| I-29 (30mg/kg i.p) | 12.0 |
| I-30 (30mg/kg i.p) | 22.9 |

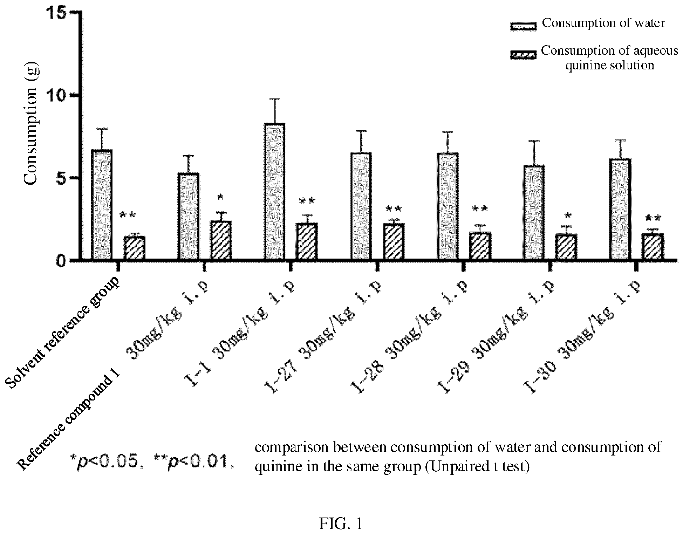

The test results indicate that compared with the positive reference compound, the compounds of the present disclosure have less interference on rat taste. Particularly, compound I-1, I-27 and I-30 have significantly less interference on rat taste than Reference compound 1 (FIG. 1).

### Test Example 4: Efficacy test for histamine/citric acid-stimulated cough in guinea pig

Guinea pigs were adaptively fed for 3 days to 7 days before being grouped. When the body weight of the guinea pigs reached 300g to 400g, they were numbered and randomly grouped.

Compounds or an excipient were administered to guinea pigs by nasal drops 0.25 hour to 24 hours before the start of the cough assessment. The administration dose of the test sample ranged from 0.17 mg/kg to 1.5 mg/kg. For the cough assessment, the guinea pigs were placed in a whole-body volume scan box for adaptation, and then, they were provided with histamine nebulization and then citric acid nebulization. The cough times within 22 min and the cough latency were recorded from the beginning of histamine nebulization until the end of the observation period.

The experimental data was statistically analyzed by one-way ANOVA to analyze and compare data of the various groups. The statistical analysis result p < 0.05 was regarded as a significant difference. The t-test was employed for difference comparison between any two.

**[Table 5] Number of histamine/citric acid-stimulated coughs in guinea pigs after administration of corresponding compounds**

| **Compound** | **Average cough times within 15 min** | **Cough inhibition rate vs. solvent (%)** |
|---|---|---|
| Solvent | 26.6 | |
| Reference compound 1 | 11.4 | 57.1 |
| I-1 | 10.8 | 59.4 |
| I-27 | 8.9 | 66.5 |
| I-28 | 9.1 | 65.8 |
| I-29 | 10.8 | 59.4 |
| I-30 | 9.8 | 63.2 |

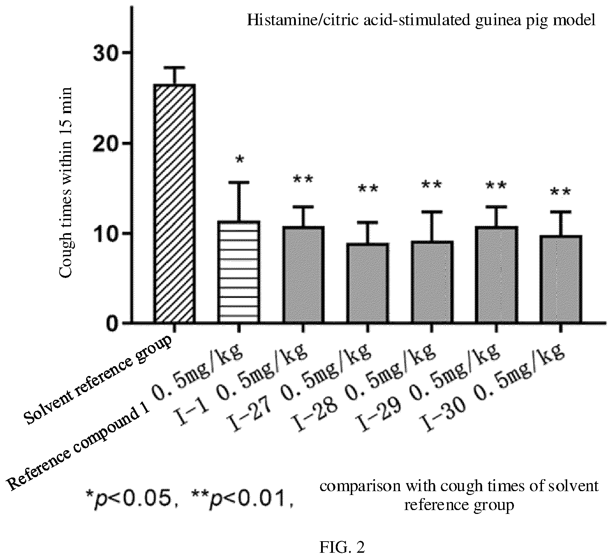

The data indicates that compared with the positive reference compond, the compounds of the present disclosure significantly reduce the cough times and prolong the cough latency in the citric acid/histamine-stimulated guinea pig cough model, having a good antitussive effect (FIG. 2).

### Test Example 5: Efficacy test for ATP/citric acid-stimulated cough in guinea pig

Guinea pigs were adaptively fed for 3 days to 7 days before being grouped. When the body weight of the guinea pigs reached 300g to 400g, they were numbered and randomly grouped. Compounds or an excipient were administered to guinea pigs by nasal drops 0.25 hour to 24 hours before the start of the cough assessment. The administration dose of the test sample ranged from 0.17 mg/kg to 1.5 mg/kg. For the cough assessment, the guinea pigs were placed in a whole-body volume scan box for adaptation, and then provided with ATP nebulization, and a few minutes later, they were provided with citric acid nebulization. The cough times within 15 minutes and the cough latency were recorded from the beginning of citric acid nebulization.

The experimental data was statistically analyzed by one-way ANOVA to analyze and compare data of the various groups. The statistical analysis result p < 0.05 was regarded as a significant difference. The t-test was employed for difference comparison between any two.

**[Table 6] Number of ATP/citric acid-stimulated coughs in guinea pigs after administration of corresponding compounds**

| **Compound** | **Average cough times within 15 min** | **Cough inhibition rate vs. solvent (%)** |
|---|---|---|
| Solvent | 19.8 | |
| Reference compound 1 | 8.1 | 59.1 |
| I-1 | 6.5 | 67.2 |
| I-27 | 6.3 | 68.2 |
| I-30 | 7.1 | 64.1 |

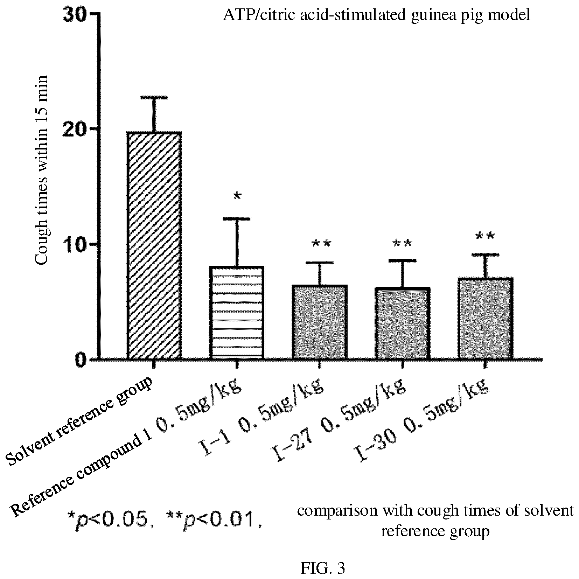

The data indicates that compared with the positive reference compound, the compounds of the present disclosure significantly reduce the cough times and prolong the cough latency in the citric acid/ATP-stimulated guinea pig cough model, having a good antitussive effect (FIG. 3).

### Test Example 4: Pharmacokinetic assay in canine

For a canine pharmacokinetic assay, male Beagle dogs (8 kg to 10 kg) were selected and fasted overnight. Three Beagle dogs were intragastrically administered with 5 mg/kg of the corresponding compound. Another 3 Beagle dogs were intravenously administered with 1 mg/kg of the corresponding compound. The blood of the dogs was collected before the administration and at 15 min, 30 min, 1 h, 2 h, 4 h, 8 h, and 24 h after the administration. The blood samples were centrifuged at 4 °C for 6 minutes at 8,000 r/min, plasma was collected and stored at -20 °C. Plasma at each time point was taken, added with 3 to 5 times the amount of internal standard-containing acetonitrile to mix, vortex mixed for 1 minute, and centrifuged at 4 °C for 10 minutes at 13,000 r/min. The supernatant was taken and mixed with 3 times the amount of water, and an appropriate amount of the mixed solution was taken for LC-MS/MS analysis. The primary pharmacokinetic parameters were analyzed by WinNonlin 7.0 software non-atrioventricular model.

**[Table 4] Pharmacokinetics of test compounds in canine**

| Test compound | Pharmacokinetic parameters in canine (intravenous administration) | | | | Pharmacokinetic parameters in canine (intragastric administration) | | | |
|---|---|---|---|---|---|---|---|---|
| | CL (L/h/kg) | Vz (L/kg) | AUC (0-t) h*ng/mL | T1/2 (h) | Tmax (hr) | Cmax (ng/mL) | AUC (0-t) (h*ng/mL) | T1/2 (hr) |
| Reference compound 2 | 0.7 | 6.59 | 1408.0 | 7.01 | 2.0 | 499.7 | 5690.5 | 7.95 |
| I-29 | 0.45 | 5.00 | 2013.9 | 7.64 | 1.00 | 1156.5 | 8730.3 | 7.26 |
| I-30 | 0.26 | 4.88 | 2821.7 | 13.01 | 1.00 | 1025.1 | 12600.9 | 13.37 |

The test results indicate that the compounds I-29 and I-30 of the present disclosure have a low clearance rate in intravenous administration, high exposure in oral and intravenous administrations, and long half-life. Compounds I-29 and I-30 exhibit better pharmacokinetic characteristics than the reference compound, and also have good druggability.

## Claims

1. A compound represented by Formula I, or a tautomer, a stereoisomer, a hydrate, a solvate, a pharmaceutically acceptable salt, or a prodrug thereof: 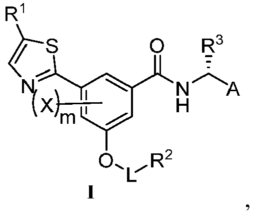 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted five-to ten-membered heteroaryl, wherein the R^{e}-substituted five-to ten-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents comprise a plurality of R^{e} substituents, the plurality of R^{e} substituents is identical or different.

2. A compound represented by Formula I, or a tautomer, a stereoisomer, a hydrate, a solvate, a pharmaceutically acceptable salt, or a prodrug thereof: 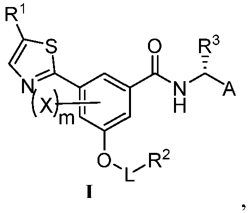 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
X is halogen;
m is an integer selected from 1, 2, or 3;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents each independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R³ is independently selected from hydrogen, unsubstituted C₁-C₄ alkyl, or C₁-C₄ alkyl substituted with 1 to 5 identical or different halogen atoms;
R⁴ is independently selected from hydrogen or C₁-C₄ alkyl;
R^{b} and R^{c} are independently selected from hydrogen or C₁-C₄ alkyl; and
A is independently unsubstituted or R^{e}-substituted five-to ten-membered heteroaryl, wherein the R^{e}-substituted five-to ten-membered heteroaryl has one or more R^{e} substituents, each R^{e} substituent being independently selected from halogen, unsubstituted C₁-C₃ alkyl, C₁-C₃ alkyl substituted with 1 to 5 identical or different halogen atoms, unsubstituted -O-(C₁-C₃ alkyl), or -O-(C₁-C₃ alkyl) substituted with 1 to 5 identical or different halogen atoms; when the one or more R^{e} substituents comprise a plurality of R^{e} substituents, the plurality of R^{e} substituents is identical or different.

3. The compound represented by Formula I, or the tautomer, the stereoisomer, the hydrate, the solvate, the pharmaceutically acceptable salt, or the prodrug thereof according to claim 1 or 2, wherein:
when R¹ is halogen, the halogen is F, Cl, Br, or I, and preferably Cl; and/or
when R¹ is the unsubstituted C₁-C₄ alkyl, the C₁-C₄ alkyl is methyl, ethyl, n-propyl, or isopropyl, and preferably methyl or ethyl; and/or
when R¹ is the C₁-C₄ alkyl substituted with 1 to 5 identical or different halogen atoms, the C₁-C₄ alkyl is methyl, ethyl, n-propyl, or isopropyl, and preferably methyl or ethyl; and/or
when R¹ is the C₁-C₄ alkyl substituted with 1 to 5 identical or different halogen atoms, the halogen atoms are F, Cl, Br, or I, and preferably Cl or F.

4. The compound represented by Formula I, or the tautomer, the stereoisomer, the hydrate, the solvate, the pharmaceutically acceptable salt, or the prodrug thereof according to claim 1 or 2, wherein:
when X is halogen, the halogen is F or Cl; and/or
m is an integer selected from 1, 2 or 3, and preferably 1.

5. The compound represented by Formula I, or the tautomer, the stereoisomer, the hydrate, the solvate, the pharmaceutically acceptable salt, or the prodrug thereof according to claim 1 or 2, wherein:
when L is -(CH₂)ₙ-, n is an integer of 0, 1, or 2, and preferably n is 0 or 1; and/or
when R² is the unsubstituted or R^{a}-substituted C₁-C₆ alkyl, the C₁-C₆ alkyl is C₁-C₄ alkyl, and preferably sec-butyl; and/or
when R² is the unsubstituted or R^{a}-substituted C₃-C₇ cycloalkyl, the C₃-C₇ alkyl is cyclopropyl, cyclobutyl, cyclopentyl, or cyclohexyl, and preferably cyclopropyl; and/or
when R² is the unsubstituted or R^{a}-substituted four-to seven-membered heterocycloalkyl, the four-to seven-membered heterocycloalkyl is four-membered, five-membered, or six-membered heterocycloalkyl; and/or
when R² is the unsubstituted or R^{a}-substituted four-to seven-membered heterocycloalkyl, the four-to seven-membered heterocycloalkyl has one or more heteroatoms selected from N, S, O, or P, and preferably selected from N or O; and/or
when R² is the unsubstituted or R^{a}-substituted four-to seven-membered heterocycloalkyl, the four-to seven-membered heterocycloalkyl has one to three heteroatoms, and preferably one or two heteroatoms; and/or
when R² is the unsubstituted or R^{a}-substituted four-to seven-membered heterocycloalkyl, R² has one to three R^{a} substituents, and preferably one R^{a} substituent; and/or
R^{a} is hydroxyl; and/or
when R^{a} is halogen, the halogen is F, Cl, Br, or I, and preferably For Cl; and/or
when R^{a} is C₁-C₆ alkyl, the C₁-C₆ alkyl is C₁-C₃ alkyl, and preferably methyl; and/or
when R^{a} is the deuterated C₁-C₆ alkyl, the C₁-C₆ alkyl is deuterated C₁-C₃ alkyl, and preferably

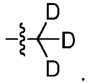

6. The compound represented by Formula I, or the tautomer, the stereoisomer, the hydrate, the solvate, the pharmaceutically acceptable salt, or the prodrug thereof according to claim 1 or 2, wherein:
when R² is the unsubstituted or R^{a}-substituted C₁-C₆ alkyl, the C₁-C₆ alkyl is preferably methyl, ethyl, n-propyl, isopropyl, n-butyl, isobutyl, sec-butyl, or tert-butyl.

7. The compound represented by Formula I, or the tautomer, the stereoisomer, the hydrate, the solvate, the pharmaceutically acceptable salt, or the prodrug thereof according to claim 1, wherein:
when R^{a} is -(C₁-C₆ alkylene)-OH, the C₁-C₆ alkylene is C₁-C₄ alkylene, preferably methylene, ethylene, n-propylene, or isopropylidene, and more preferably methylene.

8. The compound represented by Formula I, or the tautomer, the stereoisomer, the hydrate, the solvate, the pharmaceutically acceptable salt, or the prodrug thereof according to claim 1 or 2, wherein:
R³ is hydrogen; and/or
when R³ is the unsubstituted C₁-C₄ alkyl, the C₁-C₄ alkyl is methyl, ethyl, n-propyl, or isopropyl, and preferably methyl.

9. The compound represented by Formula I, or the tautomer, the stereoisomer, the hydrate, the solvate, the pharmaceutically acceptable salt, or the prodrug thereof according to claim 1 or 2, wherein:
when A is the R^{e}-substituted five-to ten-membered heteroaryl, the five-to ten-membered heteroaryl is six-membered heteroaryl, and preferably pyrimidinyl or pyridazinyl; and/or
when A is the R^{e}-substituted five-to ten-membered heteroaryl, A has one R^{e} substituent; and/or
when A is the R^{e}-substituted five-to ten-membered heteroaryl, R^{e} is C₁-C₃ alkyl substituted with 1 to 5 identical or difference halogen atoms, and preferably trifluoromethyl; and/or
when A is the R^{e}-substituted five-to ten-membered heteroaryl, R^{e} is unsubstituted C₁-C₃ alkyl, and preferably methyl.

10. The compound represented by Formula I, or the tautomer, the stereoisomer, the hydrate, the solvate, the pharmaceutically acceptable salt, or the prodrug thereof according to claim 1, wherein -L-R² is selected from: 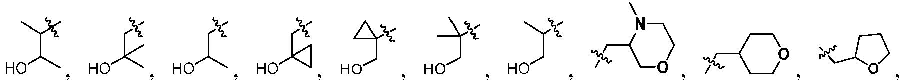 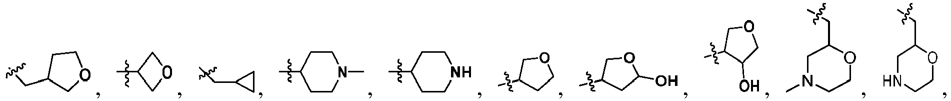 or

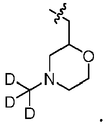

11. The compound represented by Formula I, or the tautomer, the stereoisomer, the hydrate, the solvate, the pharmaceutically acceptable salt, or the prodrug thereof according to claim 1 or 2, wherein -L-R² is selected from:

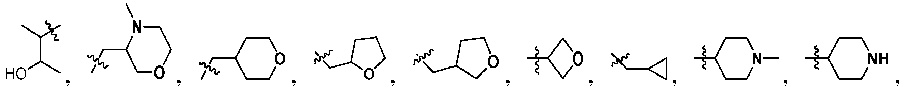

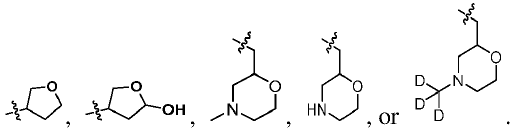

12. The compound represented by Formula I, or the tautomer, the stereoisomer, the hydrate, the solvate, the pharmaceutically acceptable salt, or the prodrug thereof according to claim 1 or 2, wherein -L-R² is

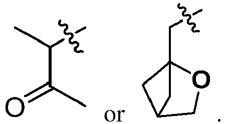

13. The compound represented by Formula I, or the tautomer, the stereoisomer, the hydrate, the solvate, the pharmaceutically acceptable salt, or the prodrug thereof according to claim 1, wherein:
R¹ is methyl, ethyl, or Cl; and/or
-L-R² is selected from: 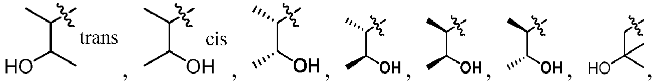 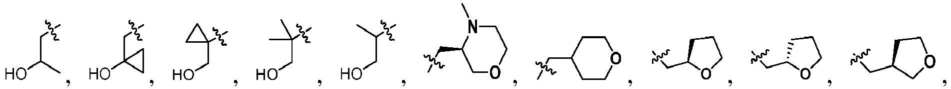 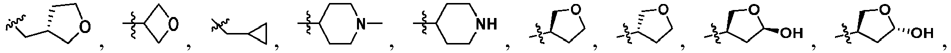 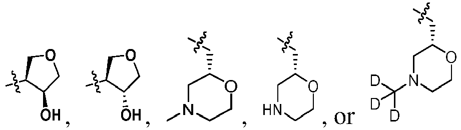 ; and/or R³ is methyl or hydrogen; and/or
A is

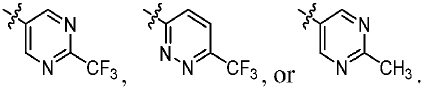

14. The compound represented by Formula I, or the tautomer, the stereoisomer, the hydrate, the solvate, the pharmaceutically acceptable salt, or the prodrug thereof according to claim 1 or 2, wherein:
R¹ is methyl, ethyl, or Cl; and/or
-L-R² is selected from: 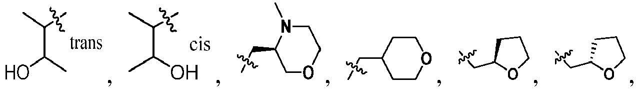 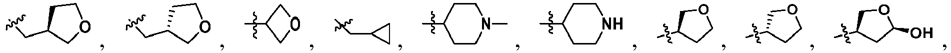 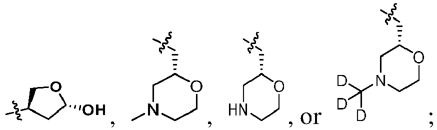 and/or
R³ is methyl or hydrogen; and/or
A is

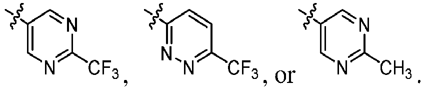

15. The compound represented by Formula I, or the tautomer, the stereoisomer, the hydrate, the solvate, the pharmaceutically acceptable salt, or the prodrug thereof according to claim 1, wherein the compound has a structural formula of: 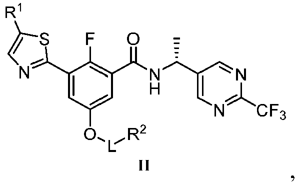 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

16. The compound represented by Formula I, or the tautomer, the stereoisomer, the hydrate, the solvate, the pharmaceutically acceptable salt, or the prodrug thereof according to claim 1, wherein the compound has a structural formula of: 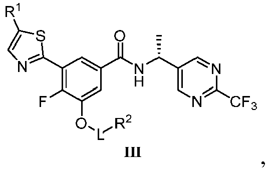 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

17. The compound represented by Formula I, or the tautomer, the stereoisomer, the hydrate, the solvate, the pharmaceutically acceptable salt, or the prodrug thereof according to claim 1, wherein the compound has a structural formula of: 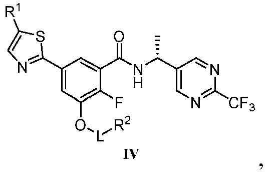 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

18. The compound represented by Formula I, or the tautomer, the stereoisomer, the hydrate, the solvate, the pharmaceutically acceptable salt, or the prodrug thereof according to claim 1, wherein the compound has a structural formula of: 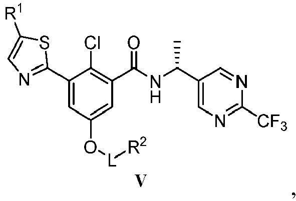 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

19. The compound represented by Formula I, or the tautomer, the stereoisomer, the hydrate, the solvate, the pharmaceutically acceptable salt, or the prodrug thereof according to claim 1, wherein the compound has a structural formula of: 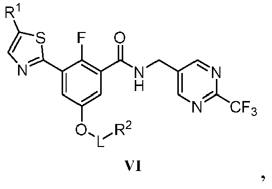 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

20. The compound represented by Formula I, or the tautomer, the stereoisomer, the hydrate, the solvate, the pharmaceutically acceptable salt, or the prodrug thereof according to claim 1, wherein the compound has a structural formula of: 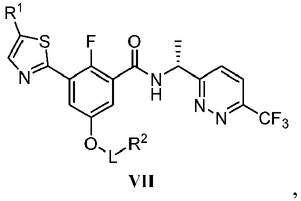 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

21. The compound represented by Formula I, or the tautomer, the stereoisomer, the hydrate, the solvate, the pharmaceutically acceptable salt, or the prodrug thereof according to claim 1, wherein the compound has a structural formula of: 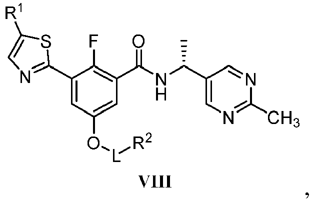 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo ( = O), -C(O)O-(C₁-C₄ alkyl), -C(O)-(C₁-C₄ alkyl), -(C₁-C₆ alkylene)-OH, or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

22. The compound represented by Formula I, or the tautomer, the stereoisomer, the hydrate, the solvate, the pharmaceutically acceptable salt, or the prodrug thereof according to claim 1 or 2, wherein the compound has a structural formula of: 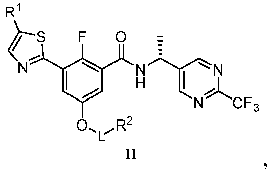 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

23. The compound represented by Formula I, or the tautomer, the stereoisomer, the hydrate, the solvate, the pharmaceutically acceptable salt, or the prodrug thereof according to claim 1 or 2, wherein the compound has a structural formula of: 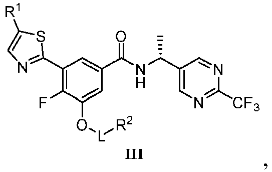 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

24. The compound represented by Formula I, or the tautomer, the stereoisomer, the hydrate, the solvate, the pharmaceutically acceptable salt, or the prodrug thereof according to claim 1 or 2, wherein the compound has a structural formula of: 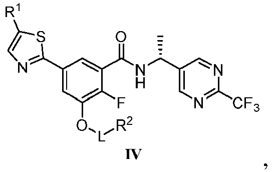 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

25. The compound represented by Formula I, or the tautomer, the stereoisomer, the hydrate, the solvate, the pharmaceutically acceptable salt, or the prodrug thereof according to claim 1 or 2, wherein the compound has a structural formula of: 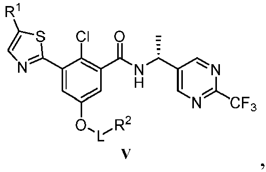 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

26. The compound represented by Formula I, or the tautomer, the stereoisomer, the hydrate, the solvate, the pharmaceutically acceptable salt, or the prodrug thereof according to claim 1 or 2, wherein the compound has a structural formula of: 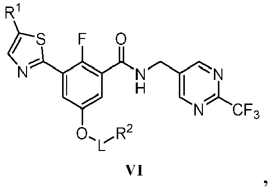 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

27. The compound represented by Formula I, or the tautomer, the stereoisomer, the hydrate, the solvate, the pharmaceutically acceptable salt, or the prodrug thereof according to claim 1 or 2, wherein the compound has a structural formula of: 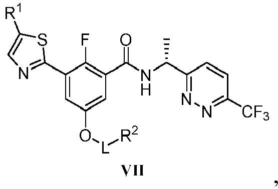 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

28. The compound represented by Formula I, or the tautomer, the stereoisomer, the hydrate, the solvate, the pharmaceutically acceptable salt, or the prodrug thereof according to claim 1 or 2, wherein the compound has a structural formula of: 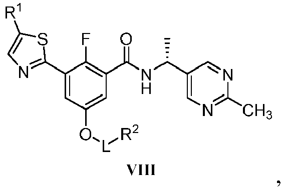 wherein:
R¹ is independently selected from halogen, C₃-C₆ cycloalkyl, unsubstituted C₁-C₄ alkyl, or C₁-C₄ alkyl substituted with 1 to 5 identical or different halogen atoms;
L is -(CH₂)ₙ-, where n is an integer selected from 0, 1, or 2;
R² is independently selected from hydrogen, unsubstituted or R^{a}-substituted C₁-C₆ alkyl, unsubstituted or R^{a}-substituted C₃-C₇ cycloalkyl, unsubstituted or R^{a}-substituted five-to eight-membered aryl, unsubstituted or R^{a}-substituted five-to ten-membered heteroaryl, unsubstituted or R^{a}-substituted four-to seven-membered heterocycloalkyl, or unsubstituted or R^{a}-substituted six-to twelve-membered heterobicycloalkyl, wherein the R^{a}-substituted C₁-C₆ alkyl, the R^{a}-substituted C₃-C₇ cycloalkyl, the R^{a}-substituted five-to eight-membered aryl, the R^{a}-substituted five-to ten-membered heteroaryl, the R^{a}-substituted four-to seven-membered heterocycloalkyl, or the R^{a}-substituted six-to twelve-membered heterobicycloalkyl has one or more R^{a} substituents, each R^{a} substituent being independently selected from unsubstituted C₁-C₄ alkyl, C₁-C₄ alkyl substituted with 1 to 5 identical or different halogen atoms, halogen, -OH, -NR^{b}R^{c}, -COOR⁴, oxo (=O), -C(O)O-(C₁-C₄ alkyl), -C(O)-(C₁-C₄ alkyl), or deuterated C₁-C₆ alkyl; when the one or more R^{a} substituents comprise a plurality of R^{a} substituents, the plurality of R^{a} substituents is identical or different; and wherein:
the unsubstituted or R^{a}-substituted five-to ten-membered heteroaryl has 1 to 3 heteroatoms each selected from N, S, O, or P;
the unsubstituted or R^{a}-substituted four-to seven-membered heterocycloalkyl has 1 to 3 heteroatoms each selected from N, S, O, or P; and
the unsubstituted or R^{a}-substituted six-to twelve-membered heterobicycloalkyl has 1 to 3 heteroatoms each selected from N, S, O, or P;
R⁴ is independently selected from hydrogen or C₁-C₄ alkyl; and
R^{b} and R^{c} are independently selected from hydrogen or C₁-C₄ alkyl.

29. The compound represented by Formula I, or the tautomer, the stereoisomer, the hydrate, the solvate, the pharmaceutically acceptable salt, or the prodrug thereof according to claim 1 or 2, wherein the compound represented by Formula I is any one selected from:

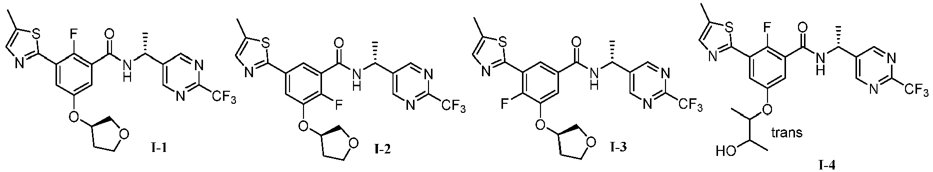

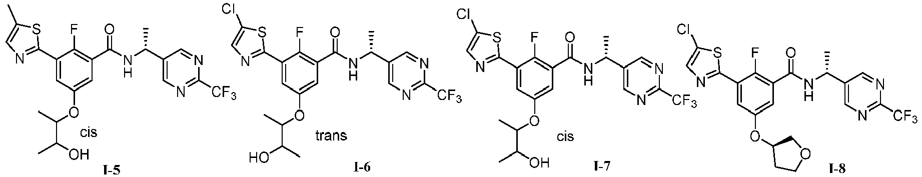

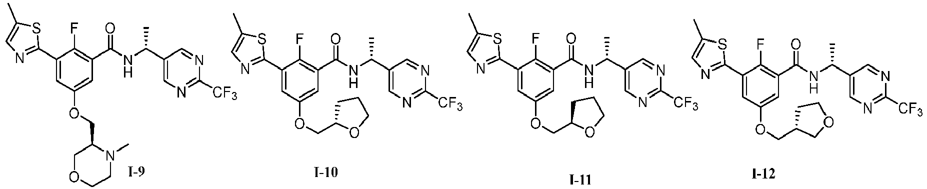

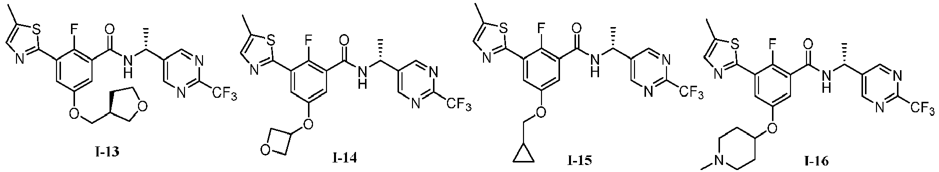

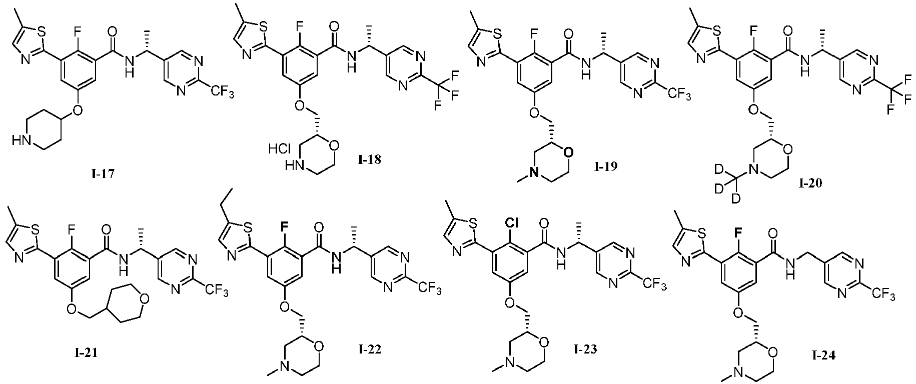

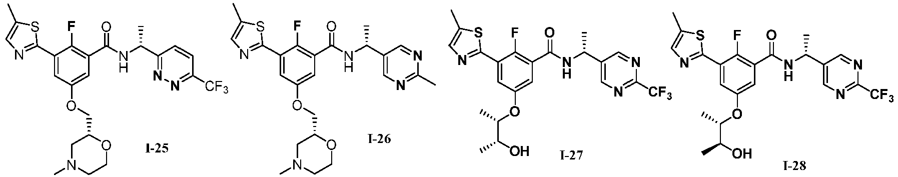

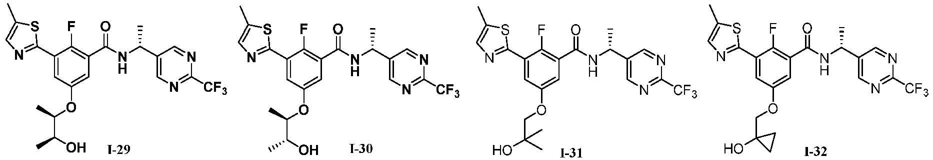

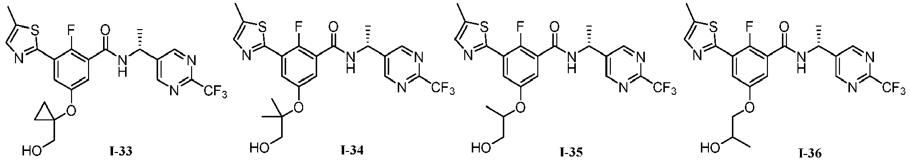

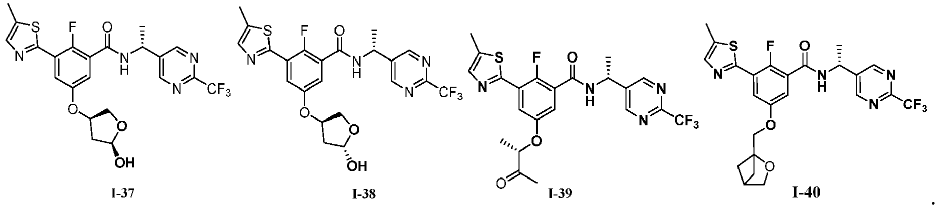

30. A pharmaceutical composition, comprising:
the compound represented by Formula I, or the tautomer, the stereoisomer, the hydrate, the solvate, the pharmaceutically acceptable salt, or the prodrug thereof according to any one of claims 1 to 29; and
a pharmaceutically acceptable excipient.

31. Use of the compound represented by Formula I, or the tautomer, the stereoisomer, the hydrate, the solvate, the pharmaceutically acceptable prodrug, or the salt thereof according to any one of claims 1 to 29, or the pharmaceutical composition according to claim 30 in the preparation of a medicament for treating a P2X3-related disease.

32. The use according to claim 31, wherein the P2X3-related disease is pain, a respiratory disease, or a urogenital disease.

33. The use according to claim 32, wherein the pain is chronic pain, acute pain, endometriosis pain, neuropathic pain, back pain, cancer pain, inflammatory pain, surgical pain, migraine, or visceral pain, and preferably, endometriosis pain or neuropathic pain.

34. The use according to claim 32, wherein the urogenital disease is decreased bladder capacity, frequent urination, urge incontinence, stress incontinence, hyperreactive bladder, benign prostatic hypertrophy, prostatitis, detrusor hyperreflexia, frequent micturition, nocturia, urgent urination, overactive bladder, pelvic hypersensitivity, urethritis, pelvic pain syndrome, prostate pain, cystitis, or idiopathic bladder hypersensitivity, and preferably overactive bladder.

35. The use according to claim 32, wherein the respiratory disease is chronic obstructive pulmonary disease, pulmonary hypertension, pulmonary fibrosis, asthma and obstructive apnea, refractory chronic cough, or acute cough.

36. A method for treating a P2X3-related disease, comprising:
administering, to a subject, the compound represented by Formula I, or the tautomer, the stereoisomer, the hydrate, the solvate, the pharmaceutically acceptable prodrug, or the salt thereof according to any one of claims 1 to 29, and/or the pharmaceutical composition according to claim 30.

37. The method according to claim 36, wherein the P2X3-related disease is pain, a respiratory disease, or a urogenital disease.

38. The method according to claim 37, wherein the pain is chronic pain, acute pain, endometriosis pain, neuropathic pain, back pain, cancer pain, inflammatory pain, surgical pain, migraine, or visceral pain, and preferably, endometriosis pain or neuropathic pain.

39. The method according to claim 37, wherein the urogenital disease is decreased bladder capacity, frequent urination, urge incontinence, stress incontinence, hyperreactive bladder, benign prostatic hypertrophy, prostatitis, detrusor hyperreflexia, frequent micturition, nocturia, urgent urination, overactive bladder, pelvic hypersensitivity, urethritis, pelvic pain syndrome, prostate pain, cystitis, or idiopathic bladder hypersensitivity, and preferably overactive bladder.

40. The method according to claim 37, wherein the respiratory disease is chronic obstructive pulmonary disease, pulmonary hypertension, pulmonary fibrosis, asthma and obstructive apnea, refractory chronic cough, or acute cough.
